# EUROPEAN PATENT SPECIFICATION

(11) **EP 3 366 784 B1**
(45) Date of publication and mention of the grant of the patent: **13.11.2019**
(21) Application number: 18166085.3
(22) Date of filing: 26.02.2015
(51) Int. Cl.: C12Q 1/68, G01N 33/558, G01N 33/68

(54) **BRAIN SPECIFIC EXOSOME BASED DIAGNOSTICS AND EXTRACORPOREAL THERAPIES**
HIRNSPEZIFISCHE EXOSOM-BASIERTE DIAGNOSTIKA UND EXTRAKORPORALE THERAPIEN
DIAGNOSTICS BASÉS SUR DES EXOSOMES SPÉCIFIQUES DU CERVEAU ET THÉRAPIES EXTRACORPORELLES

(30) Priority: 28.02.2014 US 201461946606 P; 03.03.2014 US 201461947276 P
(43) Date of publication of application: 29.08.2018
(62) Divisional of application: 15755141.7
(73) Proprietor: Exosome Sciences Inc., San Diego, CA 92122 (US)
(72) Inventor: Joyce, James, San Diego, CA 92123 (US); Taylor, Douglas, San Diego, CA 92123 (US); Taylor, Cicek, San Diego, CA 92123 (US)
(74) Representative: Aera A/S

(56) References cited:
- WO-A1-2011/066589
- WO-A2-2008/030546
- WO-A2-2010/065765
- US-A1- 2006 040 408
- SASCHA KELLER ET AL: "Body fluid derived exosomes as a novel template for clinical diagnostics", JOURNAL OF TRANSLATIONAL MEDICINE, BIOMED CENTRAL, LONDON, GB, vol. 9, no. 1, 8 June 2011 (2011-06-08), page 86, XP021100813, ISSN: 1479-5876, DOI: 10.1186/1479-5876-9-86
- MYRIAM OLIVEIRA-RODR?GUEZ ET AL: "Development of a rapid lateral flow immunoassay test for detection of exosomes previously enriched from cell culture medium and body fluids", JOURNAL OF EXTRACELLULAR VESICLES, vol. 5, no. 1, 1 January 2016 (2016-01-01) , page 31803, XP055388237, DOI: 10.3402/jev.v5.31803

## Description

### Field of the Invention

Embodiments of the present invention relate to, diagnostic devices for isolating, identifying, measuring, detecting, and analyzing extracellular vesicles, such as exosomes, which contain biomarkers including peptides, proteins, and/or nucleic acids that indicate the presence of a brain injury, including the presence or proclivity for traumatic brain injury (TBI) such as chronic traumatic encephalopathy (CTE) or other brain diseases, such as Alzheimer's disease. In particular, devices that measure and analyze exosomes derived specifically from brain cells, identified by the presence of certain antigens, including tau, β-amyloid, S100 β, neuron-specific enolase, glycoprotein A2B5, CD133, NQ01, synaptophysin, neuronal nuclei, MAB1569, polysialic acid-neural cell adhesion molecule, or neurogenic differentiation 1 molecules or glycosylated or phosphorylated forms of these molecules, are provided herein.

### Description of the Related Art

In the United States, at least 1.7 million people sustain a traumatic brain injury (TBI) each year. Injuries to the brain result in a variety of outcomes including 52,000 deaths, 275,000 hospitalizations, and 1,365,000 individuals treated and released from an emergency department. In children from 0 to 14 years of age, brain injuries each year result in approximately 2,685 deaths, 37,000 hospitalizations, and 435,000 emergency department visits. All brain injuries are unique and the type of injury the brain receives may be limited to one functional area of the brain, or various areas, or all areas of the brain. Brain injuries can be grouped into two broad types: traumatic brain injury and acquired brain injury. There are multiple causes of brain injuries, including: strokes, anoxic brain injury (prolonged lack of oxygen), infections, encephalopathy, such as severe dehydration or over hydration, disruption of the heart, or exposure to toxic agents, hypoxic injury (decreased flow of oxygen), tumors of the brain, or trauma caused by the head being struck or whipped, such as traumatic brain injury (TBI).

Chronic traumatic encephalopathy (CTE) is a brain disorder caused by multiple TBIs. CTE is a progressive neurodegenerative disease that is most prevalent among professional athletes and soldiers exposed to blast injury, and results from multiple concussions or repeated head trauma. The long term effects of repetitive head trauma result in progressive and debilitating neurological symptoms, such as memory disturbances, violent behavior, confusion, cognitive defects, depression, dementia, and even death (Stern, R.A. et al., Clinical presentation of chronic traumatic encephalopathy. Neurology, 2013. 81: 1121-29). These debilitating symptoms are generally manifest in the patient long after the head trauma has occurred, indicating that the brain experiences continual and progressive degeneration from some time point immediately after an initial trauma until the symptoms are manifest.

Unfortunately, the field of clinical neurology is unable to adequately address the problems associated with TBI, including CTE, because early stage diagnosis is poor. In addition, self-diagnosis is poor because the patient is entirely unaware of the progressive and continual degeneration in the brain until years after the trauma. Often, the patient continues to unknowingly aggravate the condition, and only years later, following irreversible damage, do the symptoms manifest. Additionally, the current state of the art only provides postmortem diagnosis. Postmortem studies reveal that patients with CTE exhibit accumulation of elevated levels of tau protein tangles in the cortex (McKee, A. C. et al., The spectrum of disease in chronic traumatic encephalopathy. Brain, 2013. 136(10): 43-64). Although this provides insight into the disease, such postmortem studies cannot provide treatment. Accordingly, a simple and non-invasive detection of brain injury and disease states stemming from brain injury, such as CTE and Alzheimer's disease, especially during the early stages of manifestation, is a long felt need in the field. Such early detection could provide both preventative measures, as well as early treatment opportunities.

Despite extensive neurological and radiographic examination, clinical evaluations are often suboptimal. For clinicians, it is critical to definitively identify those who have sustained even mild TBI and those who are at risk for adverse outcomes. Current diagnostic tools are often time consuming and expensive. For example, magnetic resonance imaging (MRI) is capable of scanning a patient's brain for changes that might be related to brain injury or brain disease. But MRIs are inconvenient and expensive. These tests require dedicated facilities that are not always readily available to particular persons in need. Thus, many individuals who are in a high risk population may be unwilling or unable to submit to diagnostic testing. Although MRI scans can potentially yield valuable information, they can be unreliable since they are based on the skill of the reading physician and variations from subject to subject may make diagnosis difficult. In addition, such diagnosis would likely require multiple scanning sessions spread out over time in order to detect specific changes in a given individual. Thus, the current state of the art for MRI is insufficient for assessing TBI.

Another promising imaging technique used for TBI diagnosis is positron emission tomography (PET). This imaging technique uses radiolabeled compounds that specifically label tau protein, thereby identifying precise locations of protein accumulation. However, PET scans are limited by the high cost, both of the process itself and of the specially required equipment, which requires dedicated facilities. In addition, the labeling molecules have a short half-life, and require specially adapted chemical synthesis. Thus, although effective for providing diagnosis of tau accumulation in patients suspected of CTE, PET scans are inconvenient and insufficient for the number of persons requiring testing (Wolf, L. K., Racing to detect brain trauma. Chem Eng News, 2014. 92(29): 9-14). Accordingly, the need for additional methods for detecting TBI, such as CTE, is manifest.

WO 2010/065765 disclose a system for facilitating diagnosis of CTE and/or Alzheimer. The device is an affinity capture device for selectively concentrating and harvesting exosomes which carry biomarkers like beta-amyloid, and the capture agent which binds the exosomes comprise a lectin, however WO 2010/065765 do not disclose nor suggest a lateral flow device for detection of exosomes, let alone exosomes for diagnosing CTE or Alzheimers.

### SUMMARY OF THE INVENTION

Point-of-care (POC) diagnostics provide reliable, inexpensive, portable, rapid, and simple approaches capable of diagnostic testing. However, POC devices for brain injuries, such as CTE, have not been realized. The lateral flow device, also known as a lateral flow assay (LFA), is a POC diagnostic tool that is capable of identifying biomarkers in a biological sample. Like most POC devices, LFA devices are minimally invasive, inexpensive, portable, and reliable. Other POC devices capable of detecting biomarkers include the flow through device (FTD).

One common example of a LFA is the common household pregnancy test. LFAs generally involve the use of a labeled antibody deposited at a first position on a solid substrate. Sample is applied to the first position, causing the antibody to dissolve in solution, whereupon the antibody recognizes and binds a first epitope on the analyte in the sample. A complex of analyte and antibody forms and this complex flows along the liquid front from the first location through the solid substrate to a second location, a test line, where immobilized antibodies are located. The immobilized antibody recognizes and binds a second epitope on the analyte, resulting in a high concentration of labeled antibody at the test line. The high concentration of labeled antibody provides a detectable visual signal. Gold nanoparticles are typically used to label the antibodies because they are relatively inexpensive and provide easily observable color indications based on the surface plasmon resonance properties of gold nanoparticles. Generally, this signal provides qualitative information, such as whether or not the analyte is present in the sample.

The term "extracellular vesicles" is intended to encompass microvesicles and exosomes. Extracellular vesicles, such as exosomes, are small vesicles released by mammalian cells for a number of purposes. During pregnancy, for example, exosomes inhibit the production of certain T-cells thereby protecting the fetus (Taylor, D. D., et al., Pregnancy-associated exosomes and their modulation of T cell signaling. J Immunol, 2006. 176(3): 1534-42). In the case of certain bacterial infections, exosomes derived from infected cells express antigenic fragments of the bacterium to stimulate the immune system against the pathogen (Bhatnagar, S., et al., Exosomes released from macrophages infected with intracellular pathogens stimulate a proinflammatory response in vitro and in vivo. Blood, 2007. 110(9): 3234-44). It has been postulated that cancers use the immunomodulatory properties of exosomes in order to evade the immune system (Taylor D. D. et al., Tumor-derived exosomes and their role in cancer associated T-cell signaling defects. Brit J Cancer, 2005. 92:305-311).

Antigens, as well as nucleic acids associated with pathogens or cancer, have been identified as being associated with exosomes (*see e.g.*, U.S. Pat. No. 8,288,172). Biomarkers associated with head injury, such as Alzheimer's disease and TBI, including CTE, are also present in exosomes (*see e.g.*, U.S. Pat. App. Pub. No. 2012/0164628). Exosomes can also be isolated or captured from a variety of patient samples (e.g., blood, plasma, urine, saliva, and spinal fluid). Antigens found at the surface of specific exosomes have been useful for the identification of specific conditions or diseases, for example.

Lectins, including Concanavalin A (Con-A) from *Canavalia ensiformis* (Jack-bean), ricin from *Ricinus communis* (RCA), and *Galanthus nivalis* lectin (GNL) have been shown to bind specifically to antigens present on cancer cells and cancer-specific exosomes secreted from these cells can be detected in binding assays utilizing lectin capture. The detection of particular antigens on extracellular vesicles, such as exosomes is unpredictable; however, because particularly useful antigens, which may be associated with the presence of disease, can be displayed on extracellular vesicles, such as exosomes, in a manner that avoids detection, e.g., epitopes of a particular biomarker that are otherwise available in tissues or a patient sample analyzed by other detection techniques, may be hidden from detection on exosomes because of differences in folding, fragmentation of the epitope, or steric hindrance.

Currently, biomarkers (including proteins, peptides, lipids, RNAs, DNA and modifications thereof) for conditions and diseases, such as cancerous tumors, rely almost exclusively on obtaining samples from tissue to identify the condition or disease. Methods to obtain these tissues of interest for analysis are often invasive, costly and pose complication risks for the subject. Furthermore, use of bodily fluids to isolate or detect biomarkers often significantly dilutes a biomarker resulting in readouts that lack requisite sensitivity. Additionally, most biomarkers are produced in low or moderate amounts in normal tissues other than the diseased tissue and thus, lack of specificity can also be problematic. Accordingly, there is a need for more effective and less invasive approaches to detect and/or predict disease and to monitor the efficacy of therapeutic approaches, specifically brain specific pathologies.

Circulating biomarkers have been proposed to be promising for the definitive diagnosis and monitoring treatment of brain diseases and brain injuries. Defining brain injury-linked biomarkers is desirable for diagnosing the disease, identifying processes that are difficult to image (such as diffuse axonal injury), predicting outcome by identifying patients at risk for long-term neurocognitive consequences, defining injury specific molecular and pathological alterations for developing therapeutic targets, and monitoring responses to acute interventions. These circulating biomarkers could also serve to monitor disease progression by assessing tissue injury and predicting risk of neurological deterioration. However, circulating biomarkers are problematic and exhibit several critical issues. Free protein and nucleic acid biomarkers are extremely unstable in the circulation, thus to detect these a high steady-state must be reached for detection, which is generally not observed except in severe brain injury. In addition, minor changes over time (which is essential for monitoring) are difficult to quantify and these biomarkers are sensitive to sample handling. To circumvent these issues, embodiments described herein utilize exosome-associated biomarkers. These are extremely stable in the circulation and are detectable for days (versus minutes for soluble markers). In addition to serving as biomarkers of injury, data demonstrate that these injury-derived exosomes may mediate events associated with brain injury. Thus, their characterization may provide insight into the "driver" mechanisms of neurodegenerative disease leading to treatment options. Furthermore, once a detection of exosomes having specific antigens is made, one can utilize extracorporeal approaches to remove these exosomes from the body (e.g., by employing binding agents such as antibodies and/or lectins to specifically remove the exosomes having the detected antigens or to more non-specifically remove all exosomes).

Proliferating and injured cells have been demonstrated to release exosomes, containing protein and RNA from the originating cell. By correlating circulating exosomal markers with molecular characteristics and real-time clinical parameters, the use of circulating exosomes create a "liquid biopsy." Specific miRNAs have been associated with development of neurological disorders, contributing to the onset and progression of complications linked with brain injuries. Exosomal-RNAs derived from injured brain tissue represent surrogates to brain biopsies and are stable, clinically accessible biomarkers for brain injury detection and sensitive measures for therapeutic outcomes. Since exosomal-RNA cargo contains mRNA fragments allowing molecular profiling of "injured" tissue, identification of driver mechanisms also defines therapeutic targets.

The types of brain injuries to which the analysis of brain-derived exosomes as described herein are useful include diagnostic markers for definitive identification of the brain injury, stratification of patients for therapy and/or monitoring of injury-progression including: diffuse axonal injury, including injury caused by shaking or strong rotation of the head, concussion, CTE, contusion, second impact syndrome, penetrating injury including injuries occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

Compositions and methods for isolating and characterizing brain-specific extracellular vesicles or exosomes are provided herein. Embodiments include compositions comprising an isolated extracellular vesicle or exosome joined to a first binding agent that specifically binds to an antigenic site present on tau, β-amyloid, S100 β, neuron specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2) protein, or glycosylated or phosphorylated forms of these molecules, or an antigenic fragment of these molecules, which e.g., is present on the surface of the exosomes. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the composition comprising an isolated extracellular vesicle or exosome joined to a first binding agent that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules comprises a first binding agent, which is an antibody, such as a monoclonal antibody, or a binding fragment thereof (e.g., a CDR or Fab fragment of an antibody, desirably a monoclonal antibody), a lectin, or an aptamer, such as a DNA aptamer that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the composition comprising an isolated extracellular vesicle or exosome joined to a first binding agent that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules comprises a first binding agent, which is an antibody, such as a monoclonal antibody, or a binding fragment thereof (e.g., a CDR or Fab fragment of an antibody, desirably a monoclonal antibody), a lectin, or an aptamer, such as a DNA aptamer that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, wherein said first binding agent is also joined to a first support. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the composition comprising an isolated extracellular vesicle or exosome joined to a first binding agent that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, wherein the first binding agent is an antibody, such as a monoclonal antibody, or a binding fragment thereof (e.g., a CDR or Fab fragment of an antibody, desirably a monoclonal antibody), a lectin, or an aptamer, such as a DNA aptamer that is specific for tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, wherein said first binding agent is also joined to a first support, comprises a first support that comprises a plastic, such as a polystyrene or polyvinylchloride, a chip, a membrane, such as a nylon or nitrocellulose membrane, a lateral flow device (or LFA), a bead, such as an agarose, latex, acrylamide, magnetic, colored bead, or polymeric bead, quantum dots, or a fiber, such as a hollow fiber, or a filter, such as a hollow filter. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the first binding agent further comprises a detectable moiety. In some embodiments, the detectable moiety is selected from the group consisting of an affinity tag, a colored bead, a photoreactive group, a radionuclide, a hapten, a peptide, an enzyme, a fluorescent species, a luminescent species, a dye, biotin, a triazole, an alkyne, biotin, quantum dots, and a chelating species.

In some embodiments, the first support further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

In some embodiments, the composition further comprises a second binding agent joined to said extracellular vesicle or exosome at a site that is distinct from a site to which said first binding agent binds said extracellular vesicle or exosome, such as a second binding agent that is specific for an antigen present on MHC class I or MHC class II molecules (e.g., non-polymorphic regions thereof).

In some embodiments, the composition further comprises a second binding agent joined to said extracellular vesicle or exosome at a site that is distinct from a site to which said first binding agent binds said extracellular vesicle or exosome, such as a second binding agent that is specific for an antigen present on MHC class I or MHC class II molecules. In some embodiments, the second binding agent is an antibody, such as a monoclonal antibody, or a binding fragment thereof (e.g., a CDR or Fab fragment of an antibody, desirably a monoclonal antibody), a lectin, or an aptamer, such as a DNA aptamer, that specifically binds to the Fas ligand, MHC I, MHC II, CD44, placental alkaline phosphatase, TSG-101, MHC I-peptide complexes, MHC II-peptide complexes, or a protein found to be present on the exterior of brain originating exosomes.

In some embodiments, the second binding agent is also joined to a second support. In some embodiments, the second support is a plastic, such as a polystyrene or polyvinylchloride, a chip, a membrane, such as a nylon or nitrocellulose membrane, a lateral flow device (or LFA), a bead, such as an agarose, latex, acrylamide, magnetic, colored bead, or polymeric bead, quantum dots, a fiber, such as a hollow fiber, or a filter, such as a hollow filter.

In some embodiments, the second binding agent further comprises a detectable moiety. In some embodiments, the detectable moiety is selected from the group consisting of an affinity tag, a colored bead, a photoreactive group, a radionuclide, a hapten, a peptide, an enzyme, a fluorescent species, a luminescent species, a dye, biotin, a triazole, an alkyne, quantum dots, and a chelating species.

In some embodiments, the second support further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

Embodiments also comprise methods for binding a brain-specific extracellular vesicle or exosome to a support comprising: (a) contacting a first support that comprises a first binding agent, which specifically binds to an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample (e.g., blood, plasma, urine, or spinal fluid) that comprises a brain-specific extracellular vesicle or exosome; and (b) identifying the presence of the brain-specific extracellular vesicle or exosome bound to the first binding agent and/or the support. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Lateral flow devices comprising a first support that comprises a first binding agent, which specifically binds to an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules are contemplated for use in the methods described herein. Exemplary binding agents that are used in these lateral flow devices include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some methods, a patient is selected, classified, or identified for an analysis or determination of the presence or absence of an exosome having an antigen present wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. The selection, classification, or identification can be performed by clinical evaluation or additional diagnostic methods (e.g., neurological evaluation by a clinician or physician, and/or EEG).

In some methods, the first binding agent is an antibody, such as a monoclonal antibody, or a binding fragment thereof (e.g., a CDR or Fab fragment of an antibody, desirably a monoclonal antibody), a lectin, or an aptamer, such as a DNA aptamer that specifically binds to an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some methods, the first support is a plastic, such as a polystyrene or polyvinylchloride, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter.

In some methods, the first binding agent further comprises a detectable moiety. In some methods, the detectable moiety is selected from the group consisting of an affinity tag, colored bead, a photoreactive group, a radionuclide, a hapten, a peptide, an enzyme, a fluorescent species, a luminescent species, a dye, biotin, a triazole, an alkyne, quantum dots, and a chelating species.

In some embodiments, the first support further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2) or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

In some embodiments, the method further comprises contacting said extracellular vesicle or exosome with a second binding agent, which specifically binds to said extracellular vesicle or exosome at a site that is distinct from the site to which said first binding agent binds said extracellular vesicle or exosome, such as a second binding agent specific for an antigen present on MHC class I or MHC class II molecules (e.g., non-polymorphic regions thereof). Lateral flow devices comprising a support that comprises said second binding agent are contemplated for use in the methods described herein.

In some embodiments, the method further comprises contacting said extracellular vesicle or exosome with a second binding agent, which specifically binds to said extracellular vesicle or exosome at a site distinct from the site to which said first binding agent binds said extracellular vesicle or exosome, such as a second binding agent specific for an antigen present on MHC class I or MHC class II molecules (e.g., a non-polymorphic region thereof). In some embodiments, the second binding agent is an antibody, such as a monoclonal antibody, or a binding fragment thereof (e.g., a CDR or Fab fragment of an antibody, desirably a monoclonal antibody), a lectin, or an aptamer, such as a DNA aptamer, that specifically binds to the Fas ligand, MHC I, MHC II, CD44, placental alkaline phosphatase, TSG-101, MHC I-peptide complexes, MHC II-peptide complexes, or a protein found to be present on the surface of exosomes originating from the brain. Lateral flow devices comprising a support that comprises said second binding agent are contemplated for use in the methods described herein.

In some methods, the second binding agent is also joined to a second support or a detection moiety. In some methods, the second support is a plastic, such as a plastic plate or dish, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter or detection molecule, such as fluorescent dye, colored beads, quantum dots, enzyme etc. Lateral flow devices comprising said support or said detection moiety that comprises said second binding agent are contemplated for use in the methods described herein.

In some methods, the second binding agent further comprises a detectable moiety. In some methods, the detectable moiety is selected from the group consisting of an affinity tag, a colored bead, a photoreactive group, a radionuclide, a hapten, a peptide, an enzyme, a fluorescent species, a luminescent species, a dye, biotin, a triazole, an alkyne, quantum dots, and a chelating species.

In some embodiments, the second support further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

In some methods, the biological sample is obtained from a subject who has had a traumatic brain injury, stroke, cognitive disorder, genetic disorder, neurodegenerative disease or a subject that is elderly. In some methods, a patient is selected, classified, or identified for an analysis or determination of the presence or absence of an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. The selection, classification, or identification can be performed by clinical evaluation or other diagnostic methods (e.g., neurological evaluation by a clinician or physician, and/or EEG).

In some methods, the biological sample is selected from the group consisting of: whole blood, plasma, serum, urine, cerebrospinal fluid, saliva, lymph, aqueous humor, vitreous humor, cochlear fluid, and tears.

In some embodiments, optionally, the method further comprises isolating or analyzing a nucleic acid (e.g., DNA or RNA) and/or protein from said isolated extracellular vesicles or exosomes for the presence or amount of a disease marker, protein, or antigen present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules or a nucleic acid encoding these molecules or a fragment thereof.

Once the presence of an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules has been made, therapeutics or therapeutic protocols can be selectively provided to the patient. In preferred methods, once the presence of exosomes having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules has been made, extracorporeal therapy on said patient utilizing a binding agent directed to an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules is performed. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some methods, the same binding agent that was utilized to make the diagnosis is used in the extracorporeal therapy. In other methods, a different binding agent than the binding agent that was used for the diagnosis is used for the extracorporeal therapy. In still more methods, once a diagnosis of the presence of exosomes having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules has been made, a non-selective exosome binding agent (e.g., a binding agent specific for an antigen present on a non-polymorphic region of MHC-1 or MHC II molecule) can be used in the extracorporeal therapy so as to remove or reduce the amount of exosomes present in the patient. Preferred extracorporeal systems include approaches utilized in U.S. Pat. No. 8,288,172.

In some embodiments, the method further comprises repeating at least steps: (a) contacting a first support that comprises a first binding agent, which specifically binds an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample (e.g., blood, plasma, spinal fluid, or urine) that comprises brain-specific extracellular vesicles or exosomes; and (b) identifying the presence of the brain-specific extracellular vesicles or exosomes bound to the binding agent and/or the support at different time points. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain Antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the method further comprises providing a subject from which said biological sample was obtained with a therapeutic agent or the extracorporeal therapy described *supra* and repeating at least steps: (a) contacting a first support that comprises a first binding agent, which specifically binds an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample that comprises brain-specific extracellular vesicles or exosomes; and (b) identifying the presence of the brain-specific extracellular vesicles or exosomes bound to the binding agent and/or the support, at a time point after providing said therapeutic agent and/or the extracorporeal therapy. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In this manner, the diagnosis, disease risk assessment, and monitoring of therapeutic approaches to inhibit, ameliorate, or reduce the onset, persistence, or manifestation of a disease can be easily accomplished.

For example, a diagnosis, a disease risk assessment, and/or a monitoring of therapeutic approaches to inhibit the onset or progression of diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes can be performed (e.g., by detecting exosomes that bind to binding agents, such as antibodies or binding fragments thereof including, a CDR or Fab fragment of an antibody, desirably a monoclonal antibody, specific for antigenic sites present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules before and after administration of a therapeutic or application of an extracorporeal approach to remove exosomes having such antigens). Since the presence of one or more of the antigens present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules on exosomes can indicate the presence of a risk factor or predilection for or the existence or persistence of one or more of: diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes, it should be understood that a plurality of binding agents with varying specificities to antigenic sites present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules can be utilized with the methods described herein to identify the presence of a risk factor or predilection for or the existence or persistence of one or more of the aforementioned diseases. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

Accordingly, aspects of the disclosed invention comprise methods for analyzing brain-specific extracellular vesicles or exosomes or methods of identifying the presence of a risk factor or predilection for or the existence or persistence of one or more of diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

Some of these methods utilize the approaches described *supra.* Additional methods can be practiced by, for example, comprising in order: (a) contacting a first support that comprises a first binding agent, e.g., an antibody, such as a monoclonal antibody, or a binding fragment thereof (e.g., a CDR or Fab fragment of an antibody, desirably a monoclonal antibody), a lectin, or an aptamer, such as a DNA aptamer, which specifically binds an antigen present on a MHC Class I or MHC class II molecule, with a biological sample that comprises brain-specific extracellular vesicles or exosomes so as to generate a biological complex comprising the first binding agent joined to the brain-specific extracellular vesicles or exosomes; (b) contacting the biological complex that comprises the first binding agent joined to the brain-specific extracellular vesicles or exosomes, with a second binding agent, which specifically binds to said extracellular vesicle or exosome at a site distinct from the site to which said first binding agent binds said extracellular vesicles or exosomes; and (c) identifying the presence of the second binding agent joined to the brain-specific extracellular vesicles or exosomes. Preferred second binding agents can include, for example, an antibody, such as a monoclonal antibody, or a binding fragment thereof (e.g., a CDR or Fab fragment of an antibody, desirably a monoclonal antibody), a lectin, or an aptamer, such as a DNA aptamer, that specifically binds to an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules. Exemplary second binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Either the first binding agent or the second binding agent or both can be joined to a support, which can be a plastic, such as a plastic plate or dish, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter or detection molecule, such as fluorescent dye, colored beads, quantum dots, enzyme etc. The biological sample can be, for example, whole blood, plasma, serum, urine, cerebrospinal fluid, saliva, lymph, aqueous humor, vitreous humor, cochlear fluid, and tears obtained from a subject, preferably a subject that has been selected, classified, or identified as one to receive an analysis of the presence of an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. Once the presence of an exosome having an antigen present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules has been made, therapeutics or therapeutic protocols, such as extracorporeal removal of exosomes can be selectively provided to the patient.

In some embodiments, the method further comprises comparing the presence of the detected of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, with a designated standard to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is represented in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

In some embodiments, devices for detecting the presence of biomarkers associated with brain injury and/or a brain pathology and/or extracellular vesicles, such as exosomes, which carry such biomarkers (e.g., tau, β-amyloid, or glycosylated or phosphorylated forms of these molecules, and/or a fragment thereof or any combination thereof) in a patient sample are provided herein. In some alternatives, a device comprising a substrate, such as a membrane or filter having one or more immobilized lectins, is used to capture and/or enrich such biomarkers or extracellular vesicles, such as exosomes, which carry such biomarkers (e.g., tau, β-amyloid, or glycosylated or phosphorylated forms of these molecules, and/or a fragment thereof or any combination thereof) so as to diagnose or identify a class of subjects that may have a brain injury and/or a brain pathology, such as Alzheimer's disease, dementia, and/or CTE. Once bound to the membrane, the presence and/or amount of these biomarkers and/or extracellular vesicles, such as exosomes, which carry such biomarkers for a brain injury and/or for a brain pathology (e.g., tau, β-amyloid, or glycosylated or phosphorylated forms of these molecules, and/or a fragment thereof or any combination thereof) are detected by analyzing, preferably, the total amount of such biomarkers bound to the substrate.

In some alternatives, the biomarker detection or the detection of the extracellular vesicles, such as exosomes, having the biomarker is performed by a labeled antibody or binding fragment thereof (e.g., an antibody or binding fragment thereof specific for tau, β-amyloid, or glycosylated or phosphorylated forms of these molecules, and/or a fragment thereof or any combination thereof), which is mixed with the biological sample obtained from the subject (e.g., blood, plasma, saliva, sweat, urine, or cerebrospinal fluid) or a diluted portion thereof in a sample reservoir. As the biomarkers present in the sample, e.g., on the extracellular vesicle or exosome population in the biological sample, bind to the labeled antibodies or binding fragments thereof in the sample reservoir, the biomarker and/or extracellular vesicle or exosome population having the biomarker and bound to the labeled antibody or binding fragment thereof begins its migration along the length of the substrate by virtue of capillary action. In some alternatives, the labeled antibody or binding fragment thereof is an antibody or binding fragment thereof specific for tau or β-amyloid or a specific form of tau or β-amyloid, such as a glycosylated tau or glycosylated β-amyloid and/or a fragment thereof, wherein said antibody or binding fragment thereof is conjugated to a colored or dyed particle, silica, gold, silver, or a microsphere, such as a dyed silica bead, a colored latex bead, or a colored glass. Such metallic or dyed particles and microspheres that can be conjugated to antibodies or binding fragments thereof specific for tau, such as glycosylated tau, or β-amyloid, such as glycosylated β-amyloid, are commercially available through Bangs Laboratories or other suppliers. Several anti-tau and β-amyloid antibodies are also commercially available (*see* Pierce Laboratories, Life Technologies, or Abcam®). The biomarker and/or extracellular vesicle or exosome population having bound labeled antibodies or binding fragments thereof will migrate along the substrate until a zone comprising a lectin is encountered (e.g., RCA) at which point the biomarker and/or extracellular vesicle or exosome population having the bound labeled antibody or binding fragment thereof is captured at the zone comprising the lectin. Because the antibodies or binding fragments thereof used in these alternatives are preferably conjugated to a visually identifiable marker, such as a colored particle or microsphere, the capture of the biomarker and/or extracellular vesicles or an exosome population having the desired biomarker (e.g., tau, β-amyloid, or glycosylated or phosphorylated forms of these molecules, and/or a fragment thereof or any combination thereof) at the zone comprising the lectin will generate a visually detectable signal.

Additionally, an amount of the biomarker being screened for (e.g., tau, β-amyloid, or glycosylated or phosphorylated forms of these molecules, and/or a fragment thereof or any combination thereof) is also immobilized on the substrate, preferably an amount of the biomarker that is indicative of a brain injury or neurodegenerative disease, such as CTE or Alzheimer's disease or both, so as to provide a visually identifiable standard by which one can immediately discern whether the tested biological sample obtained from the subject has an amount of the biomarker (e.g., tau, β-amyloid, or glycosylated or phosphorylated forms of these molecules, and/or a fragment thereof or any combination thereof) in the sample, preferably the extracellular vesicle or exosome population in the sample, which is indicative of a brain injury or neurodegenerative disease such as CTE or Alzheimer's disease or both. Alternatively, the standard can be pre-printed on the device in intensity or gradients representative of the concentration of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the printed standard can be compared to the amount of target antigen in the sample. Further, an amount of the biomarker being screened (e.g., e.g., tau, β-amyloid, or glycosylated or phosphorylated forms of these molecules, and/or a fragment thereof or any combination thereof), which is found in healthy subjects can also be immobilized on the substrate so as to provide a second visually identifiable standard by which one can immediately discern whether the tested biological sample obtained from the subject has an amount of the biomarker in the sample, preferably the extracellular vesicle or exosome population in the sample, which is indicative of a normal or healthy state. Various amounts of the biomarker being screened for (e.g., tau, β-amyloid, or glycosylated or phosphorylated forms of these molecules, and/or a fragment thereof or any combination thereof) can be immobilized on the substrate so as to generate a range of standards, for example the amount of biomarker (e.g., tau, β-amyloid, or glycosylated or phosphorylated forms of these molecules, and/or a fragment thereof or any combination thereof) that can be found in a healthy individual, the amount of biomarker (e.g., tau, β-amyloid, or glycosylated or phosphorylated forms of these molecules, and/or a fragment thereof or any combination thereof) that can be found in an individual that is at risk for a neurodegenerative disease, such as CTE or Alzheimer's, and/or the amount of biomarker (e.g., tau, β-amyloid, or glycosylated or phosphorylated forms of these molecules, and/or a fragment thereof or any combination thereof) that can be found in an individual that has a clinical diagnosis of CTE or Alzheimer's disease. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

In some alternatives, the device is comprised of a substrate, such as a membrane, having a sample pad, wherein a biological sample obtained from a subject being tested is placed. When a test is run, a portion of the biological sample is placed on the sample pad, and the analytes in the biological sample migrate through the sample pad and along the substrate to a conjugate pad. At the conjugate pad, labeled antibodies or binding fragments thereof specific to the biomarker of interest (e.g., tau, β-amyloid, or glycosylated or phosphorylated forms of these molecules, and/or a fragment thereof or any combination thereof) are incorporated. The antibodies can be labeled with a metal, e.g., gold, or silver, a colored particle, colloidal gold or colloidal silver, a fluorescent moiety, or a colored latex particle. When the sample migrates onto the conjugate pad, the conjugate (e.g., antibody or binding portion thereof) resolubilizes and recognizes and attaches to specific antigens in the sample, when such antigens are present in the biological sample.

Analytes in the biological sample, now carrying the labeled antibody or binding portion thereof, continue to migrate along the substrate by capillary action to the test pad. The test pad has one or more lectins immobilized thereon (e.g., RCA). As the analytes in the biological sample migrate along the substrate past the immobilized lectin, the antibody or binding fragment-labeled biomarkers, such as biomarkers present in the sample such as on extracellular vesicles, for instance, exosomes, present in the biological sample are captured by the lectin at the test pad. Any sample not captured by the lectin continues to flow along the substrate beyond the test pad and into a reservoir membrane, which acts as a wick thereby generating the capillary action. The presence of the screened biomarker (e.g., tau, β-amyloid, or glycosylated or phosphorylated forms of these molecules, and/or a fragment thereof or any combination thereof) on the extracellular vesicle or exosome population present in the biological sample is then readily identified by visual or photographic inspection or detection of the labeled (e.g., colored particle) antibodies at the test pad. In some alternatives, one or more standards, such as an amount of the desired biomarker (e.g., tau, β-amyloid, or glycosylated or phosphorylated forms of these molecules, and/or a fragment thereof or any combination thereof) indicative of the amount of the desired biomarker present in a sample from a healthy subject, a subject at risk for CTE or Alzheimer's disease is also immobilized on the substrate. In these alternatives, labeled antibodies or binding fragments thereof specific for the biomarker of interest (e.g., tau, β-amyloid, or glycosylated or phosphorylated forms of these molecules, and/or a fragment thereof or any combination thereof) are liberated from the conjugate pad by either a buffer or the biological sample being tested and unbound labeled antibodies can then bind to the immobilized standards thereby providing a visually identifiable signal. A comparison of the signal obtained in the tested sample lane to the standard lane(s) will allow one to easily identify whether the subject has a normal or healthy amount of the biomarker, such as extracellular vesicles or exosomes having the biomarker (e.g., tau, β-amyloid, or glycosylated or phosphorylated forms of these molecules, and/or a fragment thereof or any combination thereof) or an amount of the biomarker or extracellular vesicles or exosomes having the biomarker (e.g., tau, β-amyloid, or glycosylated or phosphorylated forms of these molecules, and/or a fragment thereof or any combination thereof) that is greater than a standard that indicates the amount of the desired biomarker found in subjects having a neurodegenerative disease (e.g., CTE or Alzheimer's disease) or in subjects that are at risk for acquiring a neurodegenerative disease (e.g., CTE or Alzheimer's disease).

In some embodiments an approach for ameliorating a neurodegenerative disease is accomplished through the removal of the biomarker and/or extracellular vesicles, such as exosomes, having the biomarker (e.g., tau, β-amyloid, or glycosylated or phosphorylated forms of these molecules, and/or a fragment thereof or any combination thereof) from a patient in need thereof, such as from a patient having elevated levels of the biomarker. Accordingly, some alternatives relate to extracorporeal removal devices used for depletion, removal, or reduction in the amount of biomarkers and/or extracellular vesicles, such as exosomes, associated with brain injury from subjects in need thereof, preferably after a diagnosis of the presence of undesirable amounts of such a biomarker or extracellular vesicle, such as an exosome, having such biomarkers (e.g., tau, β-amyloid, or glycosylated or phosphorylated forms of these molecules, and/or a fragment thereof or any combination thereof) has been made utilizing one or more of the diagnostic devices described herein.

A patient can be identified as being in need thereof by using a diagnostic device as described herein to determine the amount of biomarker present in the subject's sample or in a representative number of extracellular vesicles, such as exosomes. The intensity of the color change can be compared to a control to determine the presence and amount of a desired biomarker in the test subject. When the subject is in need thereof, in one particular embodiment, a device for extracorporeal treatment of blood or a blood fraction such as plasma is provided. This device has a sorbent circulation circuit, which adheres to and retains microvesicles, such as exosomes, and a blood circulation circuit through which blood cells flow unimpeded. In some aspects, the device may be constructed as a closed system in a manner that no accumulating reservoir is needed and the sorbent circulation system accumulates the microvesicles, such as exosomes, while non-microvesicle matter is allowed to flow back into the blood circulation system and subsequently returned to the patient. Alternatively, the device may use an accumulator reservoir that is attached to the sorbent circulation circuit and connected in such a manner so that waste fluid is discarded, but volume replenishing fluid is inserted back into the blood circulation system so the substantially microvesicle, such as exosome, purified blood that is reintroduced to said patient resembles a hematocrit of significant homology to the blood that was extracted from said patient. A similar design system that can be fabricated in accordance with the disclosure provided herein so as to perform any one or more of the methods described herein for removing undesired biomarkers, or microvesicles, such as exosomes, having an undesired biomarker (e.g., tau, β-amyloid, or glycosylated or phosphorylated forms of these molecules, and/or a fragment thereof or any combination thereof) is described in detail in U.S. Pat. No 8,288,172.

Accordingly, some aspects of the present invention relate to the following embodiments:
1. A diagnostic device, comprising:
   (a) a substrate comprising a sample reservoir, wherein said sample reservoir is configured to receive an amount of a biological sample and said sample reservoir comprises an amount of one or more mobilizable labeled antibodies or binding fragments thereof specific for tau, glycosylated tau, phosphorylated tau, β-amyloid, glycosylated β-amyloid, phosphorylated β-amyloid, and/or a fragment thereof or any combination thereof;
   (b) an absorbent material in fluid communication, such as by capillary flow, with said substrate distal from said sample reservoir;
   (c) a lectin immobilized to said substrate at a test zone, wherein said test zone is in fluid communication, such as by capillary flow, with said sample reservoir and said absorbent material;
   (d) a first amount of tau, glycosylated tau, phosphorylated tau, β-amyloid, glycosylated β-amyloid, phosphorylated β-amyloid, and/or a fragment thereof or any combination thereof immobilized to said substrate at a first control/standard zone, wherein said first control/standard zone is in fluid communication, such as by capillary flow, with said sample reservoir and, wherein the first amount of protein in the first control/standard zone is an amount detectable by said mobilizable labeled antibodies or binding fragments thereof in a biological sample obtained from a healthy subject; and/or
   (e) a second amount of tau, glycosylated tau, phosphorylated tau, β-amyloid, glycosylated β-amyloid, phosphorylated β-amyloid, and/or a fragment thereof or any combination thereof immobilized to said substrate at a second control/standard zone, wherein the second amount of protein in the second control/standard zone is an amount detectable by said mobilizable labeled antibodies or binding fragments thereof in a biological sample obtained from a subject that has a brain pathology, such as Alzheimer's disease or chronic traumatic encephalopathy (CTE); and preferably, the first and/or second amounts of tau, glycosylated tau, phosphorylated tau, β-amyloid, glycosylated β-amyloid, phosphorylated β-amyloid, and/or a fragment thereof or any combination thereof is the detectable amount of said proteins in the same volume of the same biological sample from said healthy subject and said subject that has a brain pathology, respectively, as the biological sample from said tested subject.
2. The device of embodiment 1, wherein the substrate is a membrane selected from the group consisting of polysulfone, polyethersulfone, polyamide, polyimide, nitrocellulose, PVDF, nylon and cellulose acetate.
3. The device of embodiment 1, wherein the biological sample is selected from the group consisting of blood, plasma, urine, sweat, milk, cerebrospinal fluid, and saliva.
4. The device of embodiment 1, wherein the lectin is a Ricin lectin.
5. The device of embodiment 1, wherein the label on the antibodies is colloidal carbon, colloidal gold, a fluorescent label, a quantum dot, a phosphor, a colored particle, a bioluminescent marker, an enzyme label, a paramagnetic particle, or a colored latex particles.
6. The diagnostic device of any one or more of embodiments 1-5, wherein the tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, which are immobilized on the device, is in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts or if the control zone comprises tau, the amount of tau immobilized is at a concentration greater than or equal to 7, 8, 9, or 10 x 10⁸/ml.
7. A method for detecting the presence of tau, glycosylated tau, phosphorylated tau, β-amyloid, glycosylated β-amyloid, phosphorylated β-amyloid, and/or a fragment thereof or any combination thereof in a biological sample comprising:
   (a) applying a biological sample to the device of any one of embodiments 1-6; and
   (b) determining the presence or amount of tau, glycosylated tau, phosphorylated tau, β-amyloid, glycosylated β-amyloid, phosphorylated β-amyloid, and/or a fragment thereof or any combination thereof captured at said capture zone or test zone of said device.
8. A method for identifying a subject that is at risk for CTE comprising:
   (a) applying a biological sample to the device of any one of embodiments 1-6; and
   (b) identifying said subject as being at risk for CTE when the amount of tau, glycosylated tau, phosphorylated tau, β-amyloid, glycosylated β-amyloid, phosphorylated β-amyloid, and/or a fragment thereof or any combination thereof captured at the capture zone or test zone is greater than the amount of tau, glycosylated tau, phosphorylated tau, β-amyloid, glycosylated β-amyloid, phosphorylated β-amyloid, and/or a fragment thereof or any combination thereof detected in the first amount of tau, glycosylated tau, phosphorylated tau, β-amyloid, glycosylated β-amyloid, phosphorylated β-amyloid, and/or a fragment thereof or any combination thereof immobilized on said device.
9. A method for ameliorating a neurodegenerative disease comprising:
   (a) applying a biological sample from a subject having a neurodegenerative disease to a device of any one of embodiments 1-6;
   (b) determining the presence of a tau, glycosylated tau, phosphorylated tau, β-amyloid, glycosylated β-amyloid, phosphorylated β-amyloid, and/or a fragment thereof or any combination thereof captured at the capture zone or test zone of said device; and
   (c) when the amount of tau, glycosylated tau, phosphorylated tau, β-amyloid, glycosylated β-amyloid, phosphorylated β-amyloid, and/or a fragment thereof or any combination thereof captured at the capture zone or test zone exceeds the amount of tau, glycosylated tau, phosphorylated tau, β-amyloid, glycosylated β-amyloid, phosphorylated β-amyloid, and/or a fragment thereof or any combination thereof detected in the first amount of tau, glycosylated tau, phosphorylated tau, β-amyloid, glycosylated β-amyloid, phosphorylated β-amyloid, and/or a fragment thereof or any combination thereof immobilized on said device;
   (d) establishing an extracorporeal circulation system, which comprises contacting the whole blood or components thereof from said subject with a single or plurality of agents capable of binding extracellular vesicles or exosomes that have tau protein found within said blood or components thereof to remove said extracellular vesicles or exosomes that tau, glycosylated tau, phosphorylated tau, β-amyloid, glycosylated β-amyloid, phosphorylated β-amyloid, and/or a fragment thereof or any combination thereof from said whole blood or components thereof; and
   (e) returning said contacted whole blood or components thereof into the original blood, said contacted whole blood or components thereof containing substantially fewer extracellular vesicles or exosomes that have tau protein in comparison to the whole blood or components thereof originally residing in the subject.

### BRIEF DESCRIPTION OF THE DRAWINGS

In addition to the features described above, additional features and variations will be readily apparent from the following descriptions of the drawings and exemplary embodiments. It is to be understood that these drawings depict typical embodiments, and are not intended to be limiting in scope.
FIG. 1 is a video capture image of the total exosomes in circulation of a brain cancer patient quantitated using NanoSight NS 300 defined in light scatter mode.
FIG. 2 is a video capture image of the brain-derived exosomes identified by a quantum dot labeled to a first binding agent specific for a β-amyloid antigen (β-amyloid antibody clone 20.1), which is a mouse monoclonal IgG_{2b} raised against amino acids 1-40 of human β-amyloid) present on the exosomes in circulation of a brain cancer patient quantitated using NanoSight NS 300 determined in fluorescent mode.
FIG. 3 is an image of the total exosomes in circulation of a brain cancer patient quantitated using NanoSight NS 300 defined in light scatter mode in a 3-D coordination plot having particle size and relative intensity on the x-axis and y-axis respectively.
FIG. 4 is an image of the brain-derived exosomes identified by a quantum dot labeled first binding agent specific for a tau antigen (tau antibody (clone D-8), which is a mouse monoclonal IgG_{2b} raised against amino acids 1-150 of human tau) present on the exosomes in circulation of a brain cancer patient quantitated using NanoSight NS 300 defined in fluorescent mode in a 3-D coordination plot having particle size and relative intensity on the x-axis and y-axis respectively.
FIG. 5 is an image of the brain-derived exosomes identified by a labeled first binding agent specific for a β-amyloid antigen (β-amyloid antibody (clone 20.1), which is a mouse monoclonal IgG_{2b} raised against amino acids 1-40 of human β-amyloid) present on the exosomes in circulation of a brain cancer patient quantitated using NanoSight NS 300 defined in fluorescent mode in a 3-D coordination plot having particle size and relative intensity on the x-axis and y-axis respectively.
FIG. 6 is an image of the brain-derived exosomes identified by a labeled first binding agent specific for a glycoprotein A2B5 antigen (anti-A2B5 antibody [clone 105], which is a mouse monoclonal antibody raised against full length human A2B5) present on the exosomes in circulation of a brain cancer patient quantitated using NanoSight NS 300 defined in fluorescent mode in a 3-D coordination plot having particle size and relative intensity on the x-axis and y-axis respectively.
FIG. 7 is an image of the brain-derived exosomes identified by a labeled first binding agent specific for a S100 β antigen (S100 β chain antibody (clone 9A11B9), which is a mouse monoclonal IgG₁ raised against the full length recombinant human S100 β chain protein) present on the exosomes in circulation of a brain cancer patient quantitated using NanoSight NS 300 defined in fluorescent mode in a 3-D coordination plot having particle size and relative intensity on the x-axis and y-axis respectively.
FIG. 8 is an image of the brain-derived exosomes identified by a labeled first binding agent specific for a human IgG antigen, induced as an autoantibody humoral response, present on the exosomes in circulation of a brain cancer patient quantitated using NanoSight NS 300 defined in fluorescent mode in a 3-D coordination plot having particle size and relative intensity on the x-axis and y-axis respectively.
FIG. 9 is a plot of number of exosomes from a patient with brain cancer binding a specific first lectin that is biotinylated and a fluorescent indicator, streptavidin-labeled quantum dot. The presence of lectin binding present on the exosomes in circulation of a brain cancer patient quantitated using NanoSight NS 300 defined in fluorescent mode in a 3-D coordination plot having particle size and relative intensity on the x-axis and y-axis respectively.
FIG. 10A and 10B are bar graphs representing the results of chromatographically isolated exosomes from samples obtained from professional football players. The samples were incubated with agarose beads coupled with RCA in spin columns. FIG. 10A depicts unbound samples from the agarose bead columns, stained with tau antibody conjugated to anti-mouse quantum dots for fluorescent detection using NanoSight analysis. FIG. 10B depicts bound samples from the agarose bead columns, stained with tau antibody conjugated to anti-mouse quantum dots for fluorescent detection using NanoSight analysis.
FIG. 11A depicts the total protein bound to exosomes found in plasma from healthy patients as determined by Bradford assay.
FIG. 11B depicts the total protein bound to exosomes found in plasma from patients with Alzheimer's disease as determined by Bradford assay.
FIG. 11C depicts the total protein bound to exosomes found in plasma from professional football players as determined by Bradford assay.
FIG. 12 is a graphical representation of the total protein, as determined by Bradford assay, that is bound to exosomes from various plasma sources, including healthy individuals, individuals at high risk of Alzheimer's disease, and individuals with a diagnosis of Alzheimer's disease.
FIG. 13 is a schematic representation of an exemplary POC diagnostic device in accordance with embodiments described herein, configured as a LFA.
FIG. 14 is a schematic representation of an exemplary POC diagnostic device, configured as a LFA.
FIG. 15 is a schematic representation of a POC diagnostic device, configured as a LFA and depicting viewing windows for sample deposition and for analysis of results.
FIG. 16 is a schematic representation of an exemplary embodiment of the invention, wherein a standard color scale is printed onto the device for comparison of the test sample.
FIG. 17 is a schematic representation of exemplary embodiments of the invention, wherein a dipstick analytical device is provided.
FIG. 18 is a schematic representation of an exemplary embodiment of the invention, wherein a flow-through device (FTD) is depicted.

### DETAILED DESCRIPTION OF THE PREFERRED EMBODIMENTS

Although the invention is described in various exemplary embodiments and implementations as provided herein, it should be understood that the various features, aspects, and functionality described in one or more of the individual embodiments are not limited in their applicability to the particular embodiment with which they are described. Instead, they can be applied alone or in various combinations to one or more of the other embodiments of the invention, whether the embodiments are described or whether the features are presented as being a part of the described embodiment. The breadth and scope of the present invention should not be limited by any exemplary embodiments described or shown herein.

Aspects of the present invention relate to compositions, devices, and methods for detecting brain specific exosomes, in particular, exosomes having an antigen present, such as tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and the use of these compositions, devices, and detection methodologies to identify the presence of a risk factor or predilection for diseases including diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

### Definitions

Biological Samples: Some embodiments of the embodiments provided herein detect brain-specific exosomes that are present in a biological sample. Biological samples as used herein include, for example, cell culture media, and tissues and fluids obtained from a subject. As used herein, the term "subject" includes an animal, a mammal, and a human. A sample obtained from a subject can include any tissue or fluid from the subject that may contain exosomes. In preferred embodiments the biological sample is selected from the group consisting of: whole blood, plasma, serum, urine, cerebrospinal fluid, saliva, lymph, aqueous humor, vitreous humor, cochlear fluid, and tears. More examples of biological samples, which can be analyzed using the methods described herein include peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids. A biological sample may also include the blastocyl cavity, umbilical cord blood, or maternal circulation, which may be of fetal or maternal origin. The biological sample may also be a tissue sample or biopsy, from which exosomes may be obtained. For example, if the sample is a solid sample, cells from the sample can be cultured and exosome product induced. In some embodiments, the sample is ascites fluid from a subject, e.g., ascites fluid from a human subject with a brain-specific injury; cell culture media supernatant from a human primary melanoma cell line; cell culture media supernatant from a human primary colon cancer cell line; or murine macrophage, e.g., murine macrophage infected with tuberculosis.

Extracellular Vesicles or Exosomes: Some embodiments of the methods and compositions provided herein include a sample comprising brain specific extracellular vesicles or exosomes. Generally, exosomes are small vesicles that are released into the extracellular environment from a variety of different cells, for example, cells that originate from, or are derived from, the ectoderm, endoderm, or mesoderm including any such cells that have undergone genetic, environmental, and/or any other variations or alterations, specifically those originating from the brain. An exosome is typically created intracellularly when a segment of the cell membrane spontaneously invaginates and is ultimately exocytosed (*see e*.*g*., Keller et al. (2006), Immunol. Lett. 107: 102-8). In some embodiments, the exosomes have a diameter of greater than 10 nm, 20 nm, or 30 nm; a diameter that is, 30-1000 nm, 30-800 nm, 30-200 nm, or 30-100 nm. In some embodiments, exosomes have a diameter of less than, 10,000 nm, 1000 nm, 800 nm, 500 nm, 200 nm, 100 nm or 50 nm. Exosomes may also be referred to as microvesicles, tausomes, nanovesicles, vesicles, dexosomes, bleb, blebby, prostasomes, microparticles, intralumenal vesicles, endosomal-like vesicles or exocytosed vehicles. Exosomes can also include any shed membrane bound particle that is derived from either the plasma membrane or an internal membrane. Exosomes can also include cell-derived structures bounded by a lipid bilayer membrane arising from both herniated evagination separation and sealing of portions of the plasma membrane or from the export of any intracellular membrane-bounded vesicular structure containing various membrane-associated proteins of tumor origin, including surface-bound molecules derived from the host circulation that bind selectively to the tumor-derived proteins together with molecules contained in the exosome lumen including tumor-derived microRNAs or intracellular proteins. Exosomes can also include membrane fragments.

### Isolated Brain-Specific Extracellular Vesicle Compositions

In accordance with some preferred embodiments, there are provided compositions comprising an isolated extracellular vesicle or exosome joined to a first binding agent that binds specifically to an antigenic epitope present on brain-associated proteins tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule or a fragment thereof. In some embodiments, the first binding agent comprises an antibody, such as a monoclonal antibody, or a binding fragment thereof (e.g., a CDR or Fab fragment of an antibody, desirably a monoclonal antibody), a lectin, or an aptamer, such as a DNA aptamer that is specific for an antigen present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule or a fragment thereof. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments, the first binding agent is also joined to a first support, which can be a plastic, such as a polystyrene or polyvinylchloride, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter.

In accordance with some preferred embodiments, there are provided compositions comprising an isolated extracellular vesicle or exosome joined to a first binding agent that specifically binds to an antigen present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule or a fragment thereof. In some embodiments, the first binding agent comprises an antibody, such as a monoclonal antibody, or a binding fragment thereof (e.g., a CDR or Fab fragment of an antibody, desirably a monoclonal antibody), a lectin, or an aptamer, such as a DNA aptamer that is specific for an antigen present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain Antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

Some embodiments of the methods and compositions provided herein include a first binding agent or an exosome-binding agent, e.g., an antibody, such as a monoclonal antibody, or a binding fragment thereof (e.g., a CDR or Fab fragment of an antibody, desirably a monoclonal antibody), a lectin, or an aptamer, such as a DNA aptamer that is specific for an antigen present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule or a fragment thereof. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments, an exosome-binding agent can be associated with a substrate or support, for example, the exosome-binding agent can be secured to the substrate by a surface or in a way that immobilizes the first binding agent (e.g., drying or cross-linking). In some embodiments, the exosome-binding agent specifically binds exosomes derived from the brain but does not specifically bind exosomes from other tissues (e.g., less than or equal to or any number in between 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or 25% of the exosomes bound by the first binding agent originate from tissues other than the brain). Examples of brain specific exosome-binding agents include selective lectins, aptamers (e.g., nucleic acid aptamers), antibodies and binding fragments thereof (e.g., fab fragments and antibody fragments comprising CDR domains), which specifically bind to antigens and epitopes presented by or present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule, which are available for binding on exosomes (e.g., antigenic sequences or epitopes that are displayed on the surface of an exosome) are contemplated for use with one or more of the embodiments disclosed herein. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). These binding agents may be a first binding agent or a second binding agent or both. For example, in some embodiments, a first binding agent, which is a non-specific exosome binding agent, such as a lectin or an aptamer (e.g., a nucleic acid aptamer), an antibody or binding fragment thereof (e.g., a fab fragment or an antibody fragment comprising a CDR domain) is used to isolate a plurality of exosomes from diverse tissue sources, which are present in a biological sample (e.g., whole blood, plasma, serum, urine, cerebrospinal fluid, saliva, lymph, aqueous humor, vitreous humor, cochlear fluid, and tears), and a second binding agent, which is specific for an epitope or an antigen present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule is used to detect, identify, or quantitate the amount of brain specific exosomes having one or more of the aforementioned antigens in the biological sample, for example in a unit volume (e.g., a ml or liter) of said biological sample.

Antibodies or binding fragments thereof, which are contemplated for use herein can have specificity for proteins including a Fas ligand, MHC I, MHC II, CD44, placental alkaline phosphatase, TSG-101, MHC I-peptide complexes, MHC II-peptide complexes, and proteins found to be present on the exterior of microvesicles specific to a brain, as mentioned *supra.* Preferred non-specific exosome binding agents can include antibodies, such as polyclonal or monoclonal antibodies or binding fragments thereof (e.g., Fab fragments or fragments having a CDR domain), which bind specifically to non-polymorphic regions of MHC I and/or MHCII molecules. Since different lectins exhibit unique affinities for specific glycoconjugates present on populations of exosomes, the differential binding of lectins to tissue-specific exosomes serve to enrich these. Exemplary lectins that can be used to generate the biological compositions and, which can be utilized in the methods described herein include *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin. Aptamers, used to generate the biological compositions and, which can be utilized in the methods described herein include short pieces of nucleic acid such as synthetic DNA and its chemical derivatives, which bind to specific antigens (i.e. DNA antibodies). The process for generating aptamers is described in detail in U.S. Pat. No. 5,567,588.

Some embodiments relate to compositions comprising an isolated brain-specific extracellular vesicle or exosome joined to a first binding agent that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. In some embodiments, the first binding agent comprises an antibody, such as a monoclonal antibody, or a binding fragment thereof, a lectin, or an aptamer, such as a DNA aptamer that is specific tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments the first binding agent is also joined to a first support or matrix. In some embodiments said first support is a plastic, such as a polystyrene or polyvinylchloride, a chip, a membrane, such as a nylon or nitrocellulose membrane, a lateral flow device, a bead, such as an agarose, latex, acrylamide, magnetic, colored bead, or polymeric bead, quantum dots, or a fiber, such as a hollow fiber, or a filter, such as a hollow filter.

Some embodiments include a composition comprising an isolated extracellular vesicle or exosome joined to a first binding agent that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule or a fragment thereof. In some embodiments, the first binding agent comprises an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin,), or an aptamer, such as a DNA aptamer that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments, the first binding agent is also joined to a first support or matrix, wherein said first support or matrix is a plastic, such as a polystyrene or polyvinylchloride, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter. In further embodiments, the first binding agent further comprises a detectable moiety. The detectable moiety can be, for example, an affinity tag, a photoreactive group, a radionuclide, a hapten, a peptide, an enzyme, a fluorescent species, a luminescent species, a dye, biotin, a triazole, an alkyne, a colored bead, a detectable label, quantum dots, or a chelating species.

Some embodiments of the methods and compositions provided herein include an exosome-binding agent, a first binding agent, or a second binding agent comprising such a detectable moiety. More examples of detectable moieties include but are not limited to enzymes, such as horseradish peroxidase (HRP), alkaline phosphatase (AP), β-galactosidase and urease. A horseradish-peroxidase detection system can be used, for example, with the chromogenic substrate tetramethylbenzidine (TMB), which yields a soluble product in the presence of hydrogen peroxide that is detectable at 450 nm. Other convenient enzyme-linked systems include, for example, the alkaline phosphatase detection system, which can be used with the chromogenic substrate p-nitrophenyl phosphate to yield a soluble product readily detectable at 405 nm. Similarly, a β-galactosidase detection system can be used with the chromogenic substrate o-nitrophenyl-β-D-galactopyranoside (ONPG) to yield a soluble product detectable at 410 nm, or a urease detection system can be used with a substrate such as urea-bromocresol purple (Sigma Immunochemicals, St. Louis, Mo.). Green Fluorescent protein (GFP), luciferase, digoxigenin and/or other detectable labels can be used.

More examples of detectable moieties that can be joined to a first or second binding agents or both, as described herein, include but are not limited to chemilluminescent labels. Useful fluorochromes that can be utilized include but are not limited to, DAPI, fluorescein, Hoechst 33258, R-phycocyanin, B-phycoerythrin, R-phycoerythrin, rhodamine, Texas red and/or lissamine. Fluorescein or rhodamine labeled antibodies, or fluorescein- or rhodamine-labeled secondary antibodies can be useful with embodiments provided herein. Methods of detecting chemilluminescent labels are known in the art. Fluorescent detection also can be useful in certain methods provided herein. An example of a secondary antibody includes an anti-GNA antibody. Isotopes can also be useful in certain methods provided herein.

A signal from a detectable moiety can be analyzed, for example, using visual identification (e.g., observing the appearance of a color or shape or image on a filter or lateral flow device), a spectrophotometer to detect color from a chromogenic substrate; a NanoParticle Tracking Device with fluorescent mode; a radiation counter to detect radiation, such as a gamma counter for detection of ¹²⁵I; or a fluorometer to detect fluorescence in the presence of light of a certain wavelength. Where an enzyme-linked assay is used, quantitative analysis of the amount of a biomarker can be performed using an ELISA reader, visual detection of a color, or by using a spectrophotometer or a device utilizing the same, such as an EMAX Microplate Reader (Molecular Devices; Menlo Park, Calif.). The assays of the invention can be automated or performed robotically, if desired, and the signal from multiple samples and multiple different binding agents can be detected simultaneously.

Some embodiments of the methods, compositions, and devices, provided herein include an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In some embodiments, a printed standard is provided, wherein the printed standard is a printed gradient range that can be compared to the detectable sample signal, wherein the printed standard represents a range of protein concentrations in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

Some embodiments include compositions comprising an isolated brain-specific extracellular vesicle or exosome joined to a first binding agent that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and a second binding agent joined to said extracellular vesicle or exosome at a site that is distinct from a site to which said first binding agent binds said extracellular vesicle or exosome, such as a second binding agent that is specific for an MHC class I or MHC class II antigen (e.g., a non-polymorphic region of a MHC I or MHC II molecule). In some embodiments, the second binding agent is specific for Fas ligand, MHC I, MHC II, CD44, placental alkaline phosphatase, TSG-101, MHC I-peptide complexes, MHC II-peptide complexes, or proteins found to be present on the exterior of microvesicles specific to a brain. In some embodiments, the first binding agent comprises an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin,), or an aptamer, such as a DNA aptamer that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments the first binding agent is also joined to a first support or matrix, wherein said first support or matrix is a plastic, such as a polystyrene or polyvinylchloride, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter. In further embodiments, the first binding agent further comprises a detectable moiety. In still further embodiments, the detectable moiety is selected from the group consisting of an affinity tag, a colored bead, a photoreactive group, a radionuclide, a hapten, a peptide, an enzyme, a fluorescent species, a luminescent species, a dye, biotin, a triazole, an alkyne, quantum dots, and a chelating species.

Some embodiments of the methods and compositions provided herein include a second binding agent or exosome-binding agent. In some embodiments, an exosome-binding agent can be associated with a substrate, for example, the exosome-binding agent can be immobilized on a substrate or a surface or the substrate (e.g., well of a plate or wall of a tube). The second binding agent can be an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin,), or an aptamer, such as a DNA aptamer. The second binding agent can also specifically bind to an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule.

Some embodiments include compositions comprising an isolated brain-specific extracellular vesicle or exosome joined to a first binding agent such as, an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin,), or an aptamer, such as a DNA aptamer that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and a second binding agent joined to said extracellular vesicle or exosome at a site that is distinct from a site to which said first binding agent binds said extracellular vesicle or exosome, such as a second binding agent that is specific for an MHC class I or an MHC class II antigen (preferably the second binding agent binds to a non-polymorphic region of an MHC I or MHC II molecule). In some embodiments, the second binding agent is specific for Fas ligand, MHC I, MHC II, CD44, placental alkaline phosphatase, TSG-101, MHC I-peptide complexes, MHC II-peptide complexes, or proteins found to be present on the exterior of microvesicles specific to a brain. In some embodiments, the second binding agent is an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments, the first and/or second binding agents are joined to a support, wherein said support is a plastic, such as a polystyrene or polyvinylchloride, a chip, a membrane, such as a nylon or nitrocellulose membrane, a lateral flow device, a bead, such as an agarose, latex, acrylamide, magnetic, colored bead or polymeric bead, quantum dots, a fiber, such as a hollow fiber, or a filter, such as a hollow filter. In further embodiments, the first and/or second binding agents further comprise a detectable moiety. In still further embodiments, the detectable moiety is selected from the group consisting of an affinity tag, a colored bead, a photoreactive group, a radionuclide, a hapten, a peptide, an enzyme, a fluorescent species, a luminescent species, a dye, biotin, a triazole, an alkyne, quantum dots, and a chelating species.

In some embodiments the support includes an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In some embodiments, a printed standard is provided, wherein the printed standard is provided on the support, or in a kit associated with the support, and wherein the standard is a printed gradient range that can be compared to the detectable sample signal, wherein the printed standard represents a range of protein concentrations in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

### Methods of Creating Isolated Brain-Specific Extracellular Vesicle Compositions

Embodiments also comprise methods for binding a brain-specific extracellular vesicle or exosome to a support comprising: (a) contacting a first support that comprises a first binding agent such as, an antibody, such as a monoclonal antibody, or a binding fragment thereof (e.g., a Fab fragment or a fragment having a CDR domain), which specifically binds to an antigen present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample (e.g., blood, plasma, urine, or spinal fluid) that comprises a brain-specific extracellular vesicle or exosome; and (b) identifying the presence of the brain-specific extracellular vesicle or exosome bound to the first binding agent and/or the support. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain Antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Lateral flow devices comprising a first support that comprises a first binding agent, which specifically binds to an antigen present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules are contemplated for use in the methods described herein. Exemplary binding agents that are used in these lateral flow devices include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some methods, a patient is selected, classified, or identified for an analysis or determination of the presence or absence of an exosome having an antigen present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. The selection, classification, or identification can be performed by clinical evaluation or additional diagnostic methods (e.g., neurological evaluation by a clinician or physician, and/or EEG).

In some methods, the first binding agent is an antibody, such as a monoclonal antibody, or a binding fragment thereof (e.g., a CDR or Fab fragment of an antibody, desirably a monoclonal antibody), a lectin, or an aptamer, such as a DNA aptamer that specifically binds to an exosome having an antigen present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain Antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some methods, the first support is a plastic, such as a polystyrene or polyvinylchloride, a chip, a membrane, such as a nylon or nitrocellulose membrane, a lateral flow device, a bead, such as an agarose, latex, acrylamide, magnetic, or polymeric bead, which can be dyed or colored, a fiber, such as a hollow fiber, or a filter, such as a hollow filter.

In some methods, the first binding agent further comprises a detectable moiety. In some methods, the detectable moiety is selected from the group consisting of an affinity tag, colored bead, a photoreactive group, a radionuclide, a hapten, a peptide, an enzyme, a fluorescent species, a luminescent species, a dye, biotin, a triazole, an alkyne, quantum dots, and a chelating species.

In some embodiments, the first support further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In some embodiments, the immobilized protein standard is representative of a gradient from healthy protein levels, to diseased protein levels, including levels associated with shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

In some embodiments, the method further comprises contacting said extracellular vesicle or exosome with a second binding agent, which specifically binds to said extracellular vesicle or exosome at a site that is distinct from the site to which said first binding agent binds said extracellular vesicle or exosome, such as a second binding agent specific for an antigen present on MHC class I or MHC class II molecules (e.g., non-polymorphic regions thereof). Lateral flow devices comprising a support that comprises said second binding agent are contemplated for use in the methods described herein.

In some embodiments, the method further comprises contacting said extracellular vesicle or exosome with a second binding agent, which specifically binds to said extracellular vesicle or exosome at a site distinct from the site to which said first binding agent binds said extracellular vesicle or exosome, such as a second binding agent specific for an antigen present on MHC class I or MHC class II molecules (e.g., a non-polymorphic region thereof). In some embodiments, the second binding agent is an antibody, such as a monoclonal antibody, or a binding fragment thereof (e.g., a CDR or Fab fragment of an antibody, desirably a monoclonal antibody), a lectin, or an aptamer, such as a DNA aptamer, that specifically binds to the Fas ligand, MHC I, MHC II, CD44, placental alkaline phosphatase, TSG-101, MHC I-peptide complexes, MHC II-peptide complexes, or a protein found to be present on the surface of exosomes originating from the brain. Lateral flow devices comprising a support that comprises said second binding agent are contemplated for use in the methods described herein.

In some methods, the second binding agent is also joined to a second support or a detection moiety. In some methods, the second support is a plastic, such as a plastic plate or dish, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter or detection molecule, such as fluorescent dye, colored beads, quantum dots, enzyme etc. Lateral flow devices comprising a support or detection moiety that comprises said second binding agent are contemplated for use in the methods described herein.

In some methods, the second binding agent further comprises a detectable moiety. In some methods, the detectable moiety is selected from the group consisting of an affinity tag, a photoreactive group, a radionuclide, a hapten, a peptide, an enzyme, a fluorescent species, a luminescent species, a dye, biotin, a triazole, an alkyne, quantum dots, and a chelating species.

In some embodiments, the second support further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In some embodiments, the immobilized protein standard is representative of a gradient from healthy protein levels, to diseased protein levels, including levels associated with shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

In some methods, the biological sample is obtained from a subject who has had a traumatic brain injury, stroke, cognitive disorder, genetic disorder, neurodegenerative disease or a subject that is elderly. In some methods, a patient is selected, classified, or identified for an analysis or determination of the presence or absence of an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. The selection, classification, or identification can be performed by clinical evaluation or other diagnostic methods (e.g., neurological evaluation by a clinician or physician, and/or EEG).

In some methods, the biological sample is selected from the group consisting of: whole blood, plasma, serum, urine, cerebrospinal fluid, saliva, lymph, aqueous humor, vitreous humor, cochlear fluid, and tears.

In some embodiments, optionally, the method further comprises isolating or analyzing a nucleic acid (e.g., DNA or RNA or both) and/or protein from said isolated extracellular vesicles or exosomes for the presence or amount of a disease marker, protein, or antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules or a nucleic acid encoding these molecules or a fragment thereof.

In some embodiments, the method further comprises repeating at least steps: (a) contacting a first support that comprises a first binding agent such as, an antibody, such as a monoclonal antibody, or a binding fragment thereof (e.g., a Fab fragment or a fragment having a CDR domain), which specifically binds an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample (e.g., blood, plasma, spinal fluid, or urine) that comprises brain-specific extracellular vesicles or exosomes; and (b) identifying the presence of the brain-specific extracellular vesicles or exosomes bound to the binding agent and/or the support at different time points. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the method further comprises repeating at least steps: (a) contacting a first support that comprises a first binding agent such as, an antibody, such as a monoclonal antibody, or a binding fragment thereof (e.g., a Fab fragment or a fragment having a CDR domain), which specifically binds an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample (e.g., blood, plasma, spinal fluid, or urine) that comprises brain-specific extracellular vesicles or exosomes; and (b) identifying the presence of the brain-specific extracellular vesicles or exosomes bound to the binding agent and/or the support at different time points. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In some embodiments, the method further comprises providing a subject from which said biological sample was obtained with a therapeutic agent or the extracorporeal therapy described *supra* and repeating at least steps: (a) contacting a first support that comprises a first binding agent, which specifically binds an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules, with a biological sample that comprises brain-specific extracellular vesicles or exosomes; and (b) identifying the presence of the brain-specific extracellular vesicles or exosomes bound to the binding agent and/or the support, at a time point after providing said therapeutic agent and/or the extracorporeal therapy. In this manner, the diagnosis, disease risk assessment, and monitoring of therapeutic approaches to inhibit, ameliorate, or reduce the onset, persistence, or manifestation of a disease can be easily accomplished. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain Antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

Some of these methods utilize the approaches described *supra.* Additional methods can be practiced by, for example, comprising in order: (a) contacting a first support that comprises a first binding agent, e.g., an antibody, such as a monoclonal antibody, or a binding fragment thereof (e.g., a CDR or Fab fragment of an antibody, desirably a monoclonal antibody), a lectin, or an aptamer, such as a DNA aptamer, which specifically binds an antigen present on a MHC Class I or MHC class II molecule, with a biological sample that comprises brain-specific extracellular vesicles or exosomes so as to generate a biological complex comprising the first binding agent joined to the brain-specific extracellular vesicles or exosomes; (b) contacting the biological complex that comprises the first binding agent joined to the brain-specific extracellular vesicles or exosomes, with a second binding agent, which specifically binds to said extracellular vesicle or exosome at a site distinct from the site to which said first binding agent binds said extracellular vesicles or exosomes; and (c) identifying the presence of the second binding agent joined to the brain-specific extracellular vesicles or exosomes. Preferred second binding agents can include, for example, an antibody, such as a monoclonal antibody, or a binding fragment thereof (e.g., a CDR or Fab fragment of an antibody, desirably a monoclonal antibody), a lectin, or an aptamer, such as a DNA aptamer, that specifically binds to an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Either the first binding agent or the second binding agent or both can be joined to a support, which can be a plastic, such as a plastic plate or dish, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter or detection molecule, such as fluorescent dye, colored beads, quantum dots, enzyme etc. In some embodiments, a standard is immobilized on the support, in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. The biological sample can be, for example, whole blood, plasma, serum, urine, cerebrospinal fluid, saliva, lymph, aqueous humor, vitreous humor, cochlear fluid, and tears obtained from a subject, preferably a subject that has been selected, classified, or identified as one to receive an analysis of the presence of an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule. Once the presence of an exosome having an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule or an antigenic fragment of these molecules has been made, therapeutics or therapeutic protocols, such as extracorporeal removal of exosomes can be selectively provided to the patient.

### Identifying Exosome Compositions

Utilizing antibodies against MHC class I, MHC class II, and tissue specific antigens, extracellular vesicles or exosomes can be identified and isolated from biologic fluids including blood, urine, and cultured medium of cell cultures. An antibody, such as a monoclonal antibody, or a binding fragment thereof (e.g., a Fab fragment or a fragment having a CDR domain), a lectin (e.g., Galanthus nivalis agglutinin (GNA), Narcissus pseudonarcissus agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules are contemplated for use herein. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Additional binding agents have specificity for antigens or epitopes present on Fas ligand, MHC I, MHC II, CD44, placental alkaline phosphatase, TSG-101, MHC I-peptide complexes, MHC II-peptide complexes, or proteins found to be present on the exterior of brain specific microvesicles. Immunoaffinity isolation and characterization of these extracellular vesicles, utilizing a combination of the binding agents described *supra* allows one to identify and classify a patient as one having a pathology of interest (traumatic brain injury, stroke, genetic disorder, neurodegenerative disease or aging) or a risk factor for a disease such as diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes. Accordingly aspects of the invention described herein concern methods of identifying the presence of an exosome having an antigen or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules, which also allows for methods of identifying the presence of a risk factor or a predilection for diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

Again, such methods are practiced by contacting a biological sample from a subject that has been identified or selected for brain-specific exosome analysis with first or second binding agent, which is an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules and detecting the presence of the first or second binding agent bound to an exosome. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., a CDR domains or Fab fragments). The first or second binding agent can also be an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigen or epitope present on Fas ligand, MHC I, MHC II, CD44, placental alkaline phosphatase, TSG-101, MHC I-peptide complexes, MHC II-peptide complexes, or proteins found to be present on the exterior of brain specific microvesicles. The proteomic and transcriptomic cargoes of the isolated extracellular vesicles can also be analyzed (e.g., by PCR mediated detection of genes, mutations, or polymorphisms). Monitoring the presence and/or appearance of these extracellular vesicles in biologic fluids including blood, urine, and cultured medium of cell cultures over time or after therapeutic intervention (e.g., administration of a therapeutic agent or a therapeutic protocol, such as extracorporeal removal of exosomes), especially after head or brain injury (e.g., diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury) or chronic brain maladies (e.g., Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes) can be useful for real-time monitoring of the pathologic status and treatment efficacy.

The first and or second binding agents described herein can be joined to a support, surface, or matrix (e.g., a lateral flow device). Surfaces contemplated for use herein are ones that can selectively restrict passage of said microvesicles, such as surfaces that have pore sizes in the range of 20-400 nanometers in size, with surfaces having pore sized in the range of 40-300 nanometers in size being preferred, or with surfaces having a pore size in the range of 50-280 nanometers in size being especially preferred. Surfaces with selective adhesion to microvesicles contemplated for use herein can also be coated with a single compound or a plurality of compounds that bind particles that are enriched in sphingomyelin and with a lower level of phosphatidylcholine as found in the cellular membranes of non-malignant cells.

Once the exosome composition is identified, it can be analyzed for diagnosis and identification of a disease risk factor, prognosis, or therapeutic prediction of a traumatic brain injury, stroke, genetic disorder, neurodegenerative disease or aging in a subject (e.g., a disease or injury such as diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes).

### Identifying Risk Factors Using Exosome Compositions

Some embodiments of the methods and compositions provided herein include methods for the diagnosis, identification of a disease risk factor, prognosis, or therapeutic prediction of a traumatic brain injury, stroke, genetic disorder, neurodegenerative disease or aging in a subject (e.g., a disease or injury such as diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes). Some embodiments include obtaining a biological sample from a subject (e.g., blood or urine), and identifying or quantifying the exosomes present in the sample or a portion thereof, wherein the presence and/or quantity of exosomes in the sample or portion thereof is indicative that the subject may have or be at risk for a traumatic brain injury, stroke, genetic disorder, neurodegenerative disease or aging such as, diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

More embodiments include methods for monitoring progression of a traumatic brain injury, stroke, genetic disorder, neurodegenerative disease or aging such as, diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes in a subject. Some such embodiments include selecting a subject for analysis of exosomes having markers for a traumatic brain injury, stroke, genetic disorder, or neurodegenerative disease, obtaining a first biological sample from said subject, preferably a subject having a traumatic brain injury, stroke, genetic disorder, or neurodegenerative disease such as, diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes at a first time point, identifying or quantifying the presence of brain specific exosomes in the first sample, obtaining a second sample from the subject having a traumatic brain injury, stroke, genetic disorder, neurodegenerative disease or aging such as, diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes at a second time point, the second time point occurring after the first time point, identifying or quantifying brain specific exosomes in the second determination, wherein a change in the presence or quantity of brain specific exosomes from the first determination to the second determination is indicative of progression of the traumatic brain injury, stroke, genetic disorder, neurodegenerative disease or aging such as, diffuse axonal injury, including that caused by shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes. In some embodiments, an increase in the quantity of exosomes is indicative of increased severity of the condition or pathology.

In some embodiments, brain-specific extracellular vesicles or exosomes are bound to a first support or matrix, wherein said first support or matrix is a plastic, such as a polystyrene or polyvinylchloride, a chip, a membrane, such as a nylon or nitrocellulose membrane, a lateral flow device, a bead, such as an agarose, latex, acrylamide, magnetic, or polymeric bead, a fiber, such as a hollow fiber, or a filter, such as a hollow filter by: (a) contacting a first support or matrix that comprises a first binding agent, which specifically binds an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule, wherein said first binding agent is an antibody, such as a monoclonal antibody, or a binding fragment thereof, a lectin, or an aptamer, such as a DNA aptamer, with a biological sample that comprises a brain-specific extracellular vesicle or exosome, wherein said first binding agent further comprises a detectable moiety; and (b) identifying the presence of the brain-specific extracellular vesicle or exosome bound to the support. In some embodiments, the detectable moiety is selected from the group consisting of an affinity tag, a colored bead, a photoreactive group, a radionuclide, a hapten, a peptide, an enzyme, a fluorescent species, a luminescent species, a dye, biotin, a triazole, an alkyne, quantum dots, and a chelating species. Binding agents, including selective lectins, aptamers (e.g., nucleic acid aptamers), antibodies and binding fragments thereof (e.g., fab fragments and antibody fragments comprising CDR domains), which specifically bind to antigenic sites and epitopes presented by tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules, which are available for binding on exosomes (e.g., antigenic sequences or epitopes that are displayed on the surface of an exosome). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). These binding agents may be a first binding agent or a second binding agent or both.

Some embodiments of the methods and compositions provided herein include a first binding agent, or a second binding agent comprising a detectable moiety. Examples of detectable moieties include but are not limited to enzymes, such as horseradish peroxidase (HRP), alkaline phosphatase (AP), β-galactosidase and urease. A horseradish-peroxidase detection system can be used, for example, with the chromogenic substrate tetramethylbenzidine (TMB), which yields a soluble product in the presence of hydrogen peroxide that is detectable at 450 nm. Other convenient enzyme-linked systems include, for example, the alkaline phosphatase detection system, which can be used with the chromogenic substrate p-nitrophenyl phosphate to yield a soluble product readily detectable at 405 nm. Similarly, a β-galactosidase detection system can be used with the chromogenic substrate o-nitrophenyl-β-D-galactopyranoside (ONPG) to yield a soluble product detectable at 410 nm, or a urease detection system can be used with a substrate such as urea-bromocresol purple (Sigma Immunochemicals, St. Louis, Mo.).

More examples of detectable moieties include but are not limited to chemilluminescent labels. Methods of detecting chemilluminescent labels are known in the art. Fluorescent detection also can be useful in certain methods provided herein. Useful fluorochromes include but are not limited to, DAPI, fluorescein, Hoechst 33258, R-phycocyanin, B-phycoerythrin, R-phycoerythrin, rhodamine, Texas red and lissamine. Fluorescein or rhodamine labeled antibodies, or fluorescein- or rhodamine-labeled secondary antibodies can be useful with embodiments provided herein. An example of a secondary antibody includes an anti-lectin antibody, such as an anti-GNA antibody. Isotopes can also be useful in certain methods provided herein. Such moieties and assays are well known in the art.

A signal from a detectable moiety can be analyzed, for example, using visual observation or a spectrophotometer to detect color from a chromogenic substrate; a radiation counter to detect radiation, such as a gamma counter for detection of ¹²⁵I; or a fluorometer to detect fluorescence in the presence of light of a certain wavelength. Where an enzyme-linked assay is used, quantitative analysis of the amount of a biomarker can be performed using a spectrophotometer such as an EMAX Microplate Reader (Molecular Devices; Menlo Park, Calif.) in accordance with the manufacturer's instructions. The assays of the invention can be automated or performed robotically, if desired, and that the signal from multiple samples can be detected simultaneously.

In some embodiments, the support matrix further comprises an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In some embodiments, the immobilized protein standard is representative of a gradient from healthy protein levels, to diseased protein levels, including levels associated with shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

Once diagnosis is made, therapeutic protocol can be administered comprising isolating the exosome compositions, quantifying the exosome compositions, and providing requisite treatment extracorporeally.

### Isolating Exosome Compositions

Some embodiments of the methods and compositions provided herein include isolating exosomes from a biological sample obtained from a subject or a patient (e.g., peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids). As used herein, the term "isolating" refers to increasing the concentration or density of exosomes in a sample and, or, removing non-exosome substances (e.g., proteins, cells) from a sample.

In addition to the isolation methodologies, which involve the use of a first and/or a second binding agents, as described herein, additional isolation approaches can be employed. Additional methods useful with some embodiments described herein include but are not limited to size exclusion chromatography, density gradient centrifugation, differential centrifugation, nanomembrane ultrafiltration, immunoabsorbent capture, affinity purification, microfluidic separation, or combinations thereof. Size exclusion chromatography, such as gel permeation columns, centrifugation or density gradient centrifugation, and filtration methods can be used. For example, exosomes can be isolated by differential centrifugation, anion exchange and/or gel permeation chromatography (*e.g*., U.S. Pat. Nos. 6,899,863 and 6,812,023), sucrose density gradients, organelle electrophoresis (*e.g.,* U.S. Pat. No. 7,198,923), magnetic activated cell sorting (MACS), or with a nanomembrane ultrafiltration concentrator. Various combinations of isolation or concentration methods can be used.

### Methods for Quantifying Exosome Composition

Some embodiments also comprise methods for quantifying exosomes in a biological sample (e.g., in a unit volume, such as a ml or liter of a biological fluid obtained from a subject). In a particular embodiment, exosomes bound to a first and/or a second binding agent, which is/are an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules or an antigen or epitope present on Fas ligand, MHC I, MHC II, CD44, placental alkaline phosphatase, TSG-101, MHC I-peptide complexes, MHC II-peptide complexes, or proteins found to be present on the exterior of brain specific microvesicles are quantified. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In an exemplary embodiment, a biological sample comprising exosomes (e.g., blood, plasma, urine, or spinal fluid, is contacted with a substrate having a surface with said first binding agent immobilized thereon. The exosomes are bound to the substrate *vis a vis* the first binding agent. A second binding agent having a detectable label is then contacted with the exosomes bound the first binding agent and the support. The second binding agent can comprise a detectable label such as colored beads, quantum dots, and/or a radionuclide. Signal from the bound detectable label brain can be measured and quantified using conventional detection methods. Methods to determine the quantity of bound exosomes using a measured signal can also include comparing the level of the signal that is detected to a reference value, such as a standard curve, or to the amount of bound exosomes present in a control biological sample (e.g., a sample obtained from a person that lacks a brain specific disease or condition). An example standard curve includes a curve prepared using a control with various quantities of a lectin-binding compound, such as mannan or mannan coated beads. In some embodiments, the method optionally includes isolating exosomes in a sample before contacting the sample with lectin immobilized to a substrate. Methods for isolating exosomes are well known in the art and examples are also provided herein.

In some embodiments of the methods provided herein, the sensitivity of the method of detecting exosomes in a sample is, or is at least, 1 x 10¹⁰ exosomes/ml, 1 x 10⁹ exosomes/ml, 1 x 10⁸ exosomes/ml, 1 x 10⁷ exosomes/ml, or 1 x 10⁶ exosomes/ml, or a range defined by any two of the preceding values. In some embodiments of the methods provided herein, the sensitivity of detecting exosomes is relative to a reference, e.g., mannan-coated beads. In such embodiments, the sensitivity of detecting exosomes in a sample is equivalent to or is equivalent to at least, 1 x 10¹⁰ beads/ml, 1 x 10⁹ beads/ml, 1 x 10⁸ beads/ml, 1 x 10⁷ beads/ml, or 1 x 10⁶ beads/ml, or a range defined by any two of the preceding values. In some embodiments, the sensitivity of detecting exosomes in the sample is or is at least, the equivalent of 1000 ng mannan/ml, 500 ng mannan/ml, 100 ng mannan/ml, 50 ng mannan/ml, 1000 pg mannan/ml, 500 pg mannan/ml, 100 pg mannan/ml, or 50 pg mannan/ml, or a range defined by any two of the preceding values.

Some methods, composition and kits utilize lectins to quantify exosomes such as Concanavalin A, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin, and *Galanthus Nivalis* lectin (GNA), which bind specifically to injured brain cells indicating that these cells contain sugar moieties uncommon to healthy cells. Brain specific exosomes secreted from these cells have the same moieties, and are therefore available to lectin capture and detection. Specifically, Enzyme Linked Lectin Specific Assays (ELLSA) are designed to bind preferentially to carbohydrate structures common to exosomes, but not to healthy human cellular components. In some such embodiments, each ELLSA plate allows for up to 96 exosome detection tests. Further analysis of the captured exosomes is possible through detection molecules such as antibodies linked to a specific biomarker on the exosome.

Additionally, some methods include assays such as the enzyme linked immunosorbent assay (ELISA), which is a powerful tool that has been used for the past 4 decades to detect proteins in homogenous and heterogeneous samples, generally by adsorbing an antigen specific antibody to a solid surface such as a 96 well plastic plate, incubating the antigen with the adsorbed antibody, and detecting the antigen with a secondary antibody labeled with various chemicals that react to give color, fluorescence or other means of detection.

In some embodiments, the protein can be quantitated by comparison to an immobilized standard, wherein the immobilized protein is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and wherein the standard is compared to a signal generated by the detectable moiety to determine the level of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, present in the sample. In some embodiments, the standard is immobilized in a range of protein concentrations, wherein the range represents tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In some embodiments, the immobilized protein standard is representative of a gradient from healthy protein levels, to diseased protein levels, including levels associated with shaking or strong rotation of the head, concussion, CTE, contusion, second impact syndrome, penetrating injury including that occurring from the impact of a sharp object to the brain, Shaken Baby Syndrome, anoxic brain injury including anoxic anoxia, anemic anoxia, and toxic anoxia, hypoxic brain injury, and chronic brain conditions including Alzheimer's disease, amyotrophic lateral sclerosis, cerebrovascular disease, Hirschsprung's disease, demyelinating diseases, Duchenne muscular dystrophy, cognitive dysfunction disease, Parkinson's disease, brain cancers, such as glioblastoma multiforme, and/or diabetes.

### Compositions and Kits for Quantifying Exosome Compositions

Embodiments provided herein also include compositions and kits for quantifying exosomes in a biological sample. In some embodiments, a composition or kit comprises a first substrate or a first support or matrix comprising a first binding agent (e.g., an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules or an antigen or epitope present on Fas ligand, MHC I, MHC II, CD44, placental alkaline phosphatase, TSG-101, MHC I-peptide complexes, MHC II-peptide complexes. Exemplary first binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In some embodiments, the detectable agent is capable of binding to exosomes derived from a plurality of cell types. Preferably, the detectable brain specific exosome-binding agent binds specifically or preferentially to brain specific exosomes. The compositions and kits can also include a second binding agent that is an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules or an antigen or epitope present on Fas ligand, MHC I, MHC II, CD44, placental alkaline phosphatase, TSG-101, MHC I-peptide complexes, MHC II-peptide complexes. Preferably, the second binding agent binds to an exosome at an epitope or antigenic site that is different than and does not interfere with the epitope or antigenic site to which the first binding agent binds. Preferably, the second binding agent comprises a detectable label such as an affinity tag, a photoreactive group, a radionuclide, a hapten, a peptide, an enzyme, a fluorescent species, a colored bead, a quantum dot, a luminescent species, a dye, biotin, a triazole, an alkyne, and a chelating species. Exemplary second binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In one embodiment, a kit for quantifying exosomes in a biological sample comprises a substrate with a first binding agent immobilized thereon, *e.g.,* a 96-well plate or filter, or test strip, and an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigen or epitope present on a tau, β-amyloid, S100 Beta, Neuron-specific enolase, Glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules or an antigen or epitope present on Fas ligand, MHC I, MHC II, CD44, placental alkaline phosphatase, TSG-101, MHC I-peptide complexes, MHC II-peptide complexes. The kit can further comprise a second binding agent such as an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules or an antigen or epitope present on Fas ligand, MHC I, MHC II, CD44, placental alkaline phosphatase, TSG-101, MHC I-peptide complexes, MHC II-peptide complexes, wherein the second binding agent comprises a detectable label. In some embodiments, the detectable label comprises horse radish peroxidase, a fluorescent label, a quantum dot, or a radionuclide. Exemplary first and/or second binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).The first and second binding agents can be prepared to be stable with an extended shelf-life, *e.g.,* freeze-dried and/or frozen. A kit can further comprise reagents such as diluents, wash solutions, and/or substrates for detecting the detectable brain specific exosome-binding agent in an assay. An example substrate includes tetramethylbenzidine, a substrate useful with a detectable label such as horse radish peroxidase. In some embodiments, the detectable brain specific exosome-binding agent is not labeled, and a secondary binding agent which is labeled and which binds the detectable brain specific exosome-binding agent is included. In some embodiments, the substrate comprises a material selected from the group consisting of sepharose, latex, glass, polystyrene, polyvinyl, nitrocellulose and silicon.

In some embodiments of the kits provided herein, the sensitivity of the kit for detecting exosomes in a sample is or is at least, 1 x 10¹⁰ exosomes/ml, 1 x 10⁹ exosomes/ml, 1 x 10⁸ exosomes/ml, 1 x 10⁷ exosomes/ml, or 1 x 10⁶ exosomes/ml, or a range defined by any two of the preceding values. In some embodiments of the methods provided herein, the kit provides a sensitivity of detecting exosomes in a sample as compared to mannan coated beads that is equivalent to or is equivalent to at least, 1 x 10¹⁰ beads/ml, 1 x 10⁹ beads/ml, 1 x 10⁸ beads/ml, 1 x 10⁷ beads/ml, or 1 x 10⁶ beads/ml, or a range defined by any two of the preceding values. In some embodiments, the sensitivity of the kit for detecting exosomes in the sample is or is at least, the equivalent of 1000 ng mannan/ml, 500 ng mannan/ml, 100 ng mannan/ml, 50 ng mannan/ml, 1000 pg mannan/ml, 500 pg mannan/ml, 100 pg mannan/ml, or 50 pg mannan/ml, or a range defined by any two of the preceding values.

In some embodiments of the methods, compositions and kits provided herein, a substrate such as a 96-well plate or a test strip (e.g., nylon or nitrocellulose) can be prepared with a first binding agent, *e.g.,* a lectin such as GNA, by contacting the substrate with a solution of the lectin. In some embodiments, the concentration of the lectin solution that is applied is or is at least, 0.5 µg/ml, 1.25 µg/ml, 2.5 µg/ml 5 µg/ml, 10 µg/ml, 20 µg/ml, 50 µg/ml, 100 µg/ml or a range defined by any two of the preceding values. In a preferred embodiment, the concentration of the lectin solution is 10 µg/ml.

### Extracorporeal Treatment

In accordance with still another aspect of the present invention, existing methods and devices of extracorporeal treatment of blood can be integrated (in whole or in part) with the above-described methods to augment ex vivo clearance of microvesicles in a physiologically applicable manner. Exemplary methods contemplated for use herein include: a) hemofiltration; b) hemodialysis; and c) hemodiafiltration, with a preferred method including apheresis followed by filtration.

In accordance with various embodiments, extracorporeal removal of microvesicles can be performed through selective adhesion of said microvesicles to matrices or substrates that are conjugated to agents possessing higher affinity to microvesicles with a high sugar content, in comparison to microvesicles of a lower sugar content. Selective adhesion of said exosomes can be accomplished using any of the first and/or second binding agents described herein.

In accordance with yet another embodiment, there are provided methods of extracorporeally removing microvesicles from a subject in need thereof, said method comprising passing said subject's whole blood, or separated blood components, through a system capable of selectively binding and retaining microvesicles based on one or more of size, charge, affinity towards lectins, or affinity towards molecules that are known to be present on said microvesicles.

In accordance with a further embodiment, there are provided methods of extracorporeally removing microvesicles from a subject in need thereof, said methods comprising passing said subject's whole blood, or separated blood components, through a system capable of non-selectively binding and retaining microvesicles based on one or more of size, charge, affinity towards lectins, or affinity towards molecules that are known to be present on said microvesicles.

In accordance with a still further embodiment, there are provided methods of extracorporeally removing microvesicles from a subject in need thereof, said methods comprising passing said subject's whole blood, or separated blood components, through a system capable of selectively binding and retaining microvesicles based on similarities between properties of microvesicles and membranes of injured brain cells.

In accordance with yet another embodiment of the present invention, there are provided methods of extracorporeally removing microvesicles from a subject in need thereof, said methods comprising passing said subject's whole blood, or separated blood components, through a system capable of non-selectively binding and retaining microvesicles based on similarities between properties of microvesicles and membranes of injured brain cells.

Reference to lectins herein includes GNA, NPA, Concanavalin A and cyanovirin, with a presently preferred lectin being Concanavalin A.

One embodiment of the present invention relates to methods that can be used for extracorporeal treatment of blood or a blood fraction for the removal of microvesicles associated with a brain injury. Such extracorporeal devices and methods of use thereof may employ a first binding agent that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Preferred first binding agents include an antibody, such as a monoclonal antibody, or a binding fragment thereof (e.g., a CDR or Fab fragment of an antibody, desirably a monoclonal antibody), a lectin, or an aptamer, such as a DNA aptamer that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Exemplary first binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Blood is run through an extracorporeal circulation circuit that uses a hollow fiber cartridge with the membranes of said hollow fibers having sufficient permeability for the microvesicles found in the blood to be removed through the membrane of the hollow fibers and into an area outside of the fibers containing a substrate that is bound to a single or plurality of first and/or second binding agents capable of adhering to said microvesicles in a manner such that said microvesicles are attached to said agent and do not substantially re-enter the hollow fibers. Within the knowledge of one skilled in the art are available numerous types of hollow fiber systems. Selection of said hollow fiber system is dependent on the desired blood volume and rate of passage of said blood volume through the hollow fiber system. Specifically, hollow fiber cartridges may be used having lengths of 250 mm and containing 535 hollow fibers supplied by Amicon, and having the fiber dimensions: I.D. 180 micron and O.D. 360 micron, and the total contact surface area in the cartridge is 750 cm.sup.2. Alternatively, the "Plasmaflux P2" hollow fiber filter cartridge (sold by Fresenius) or Plasmart PS60 cartridges (sold by Medical srl) may be used. These and other hollow fiber systems are described by Ambrus and Horvath in U.S. Pat. No. 4,714,556. Hollow fiber cartridges such as described by Tullis in U.S. Patent Application 20040175291 (incorporated by reference herein in its entirety) may also be used. Furthermore, said hollow fiber cartridges and affinity cartridges in general are thought in U.S. Pat. Nos. 4,714,556, 4,787,974 and 6,528,057.

Regardless of hollow fiber system used, the concept needed for application of these embodiments, is that said hollow fiber filters are required to allow passage of blood cells through the interior of said hollow fiber, and allow diffusion of microvesicles to the exterior. In order to allow such diffusion, the pores on the membrane of the hollow fiber need to be of a diameter sufficient to allow particles ranging from the size of 20 nanometers to 500 nanometers in diameter. More specifically, the pores on the membrane of the hollow fiber need to be of a diameter sufficient to allow particles ranging from the size of 50 nanometers to 300 nanometers in diameter. Even more specifically, the pores on the membrane of the hollow fiber need to be of a diameter sufficient to allow particles ranging from the size of 80 nanometers to 200 nanometers in diameter. During experimentation with different hollow fibers, one skilled in the art would find it useful to utilize particles of similar size ranges as the microvesicles in order to calibrate and quantitate the ability of various pore sizes of hollow filters. One method of performing this is through the utilization of commercially available MACS Beads (Milteny Biotech), which have a size of 60 nanometers. Fluorescent, spherical latex beads ranging in size from 25 to 1000 nm are also available for this purpose (e.g., from Duke Scientific (Palo Alto, Calif.)).

The substrate or matrix to be used in practicing these embodiments preferably allows sufficient permeation of flow so that non-cellular blood components that enter the space exterior to the hollow fiber are distributed throughout the substrate or matrix material, so that substantial contact is made between the microvesicles permeating the hollow fiber filter and the microvesicle-binding agent that is attached to the substrate or matrix. Suitable substrates or matrices are known to one skilled in the art. Said substrates or matrices include silica gel, dextran, agarose, nylon polymers, polymers of acrylic acid, copolymers of ethylene and maleic acid anhydride, aminopropylsilica, aminocelite, glass beads, silicate containing diatomaceous earth or other substrates or matrices known in the art. Examples of such are described in the following patents: Lentz U.S. Pat. No. 4,708,713, Motomura U.S. Pat. No. 5,667,684, Takashima et al U.S. Pat. No. 5,041,079, and Porath and Janson U.S. Pat. No. 3,925,152. The first and/or second binding agents that are attached to said substrate are chosen based on known affinity to brain specific microvesicles, which are desired for removal (e.g., a first binding agent such as an antibody, Fab fragment, or aptamer that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Said agents may also be capable of non-specifically binding to said microvesicles, in that binding occurs both from non-brain-specific associated microvesicles, and from brain-specific associated microvesicles, or conversely, said agents may display a certain degree of selectivity for exosomes derived from brain injuries. For example, binding agents that are specific for an antigen present on MHC I or MHC II molecules can be employed, preferably a binding agent such as an antibody, Fab fragment, aptamer, or lectin specific for an antigen present on MHC I or MHC II molecules.

In one embodiment, said agents non-specifically bind all microvesicles due to common expression of molecules such as MHC I on microvesicles that are associated with conditions of brain injuries, and microvesicles that are not. Specifically, an agent that would bind both types of microvesicles would be an antibody specific to the non-polymorphic regions of MHC I. Therefore, in the embodiment of the invention in which non-selective removal of microvesicles is sought, anti-MHC I antibodies would be bound to said substrate chosen, and the combination would be placed to reside outside of the hollow fiber filters in order to allow binding of said microvesicles to the substrate, however blood cells and other components of the blood would not be removed during the passage of blood through the encased system containing said hollow fiber filters, exterior substrate, and microvesicle binding agent.

In order to achieve non-specific removal of microvesicles, another embodiment involves the use of hollow fiber filters of sufficient size of the pores on the side of the hollow fiber filter for microvesicles to exit, while not allowing blood cells to exit, and passing a continuous solution over said hollow fiber filters in order to clear said microvesicles leaking through the sides of the hollow fiber filters. In such a situation it would be critical to re-introduce the other blood components that escaped the hollow fiber filter, such as albumin, back into the microvesicle purified blood, before returning of the blood to the subject.

Alternatively, the hollow-fiber cartridge may be sealed as described in Ambrus (U.S. Pat. No. 4,714,556). In such a system, both diffusion and convection cause blood fluids (exclusive of blood cells) to pass through the pores in the hollow fibers and into contact with the capture molecules bound to the solid phase matrix. The fluids (e.g. plasma) pass back into the circulation at the distal end of the cartridge through a process known as Starling flow. In this system, there is no significant loss of blood fluids and therefore no need for blood component replacement.

In the situations where a substantially specific removal of microvesicles associated with brain injuries is desired, the said agent bound to said substrate outside of said hollow fiber filters possesses affinity to molecules specifically found on said microvesicles associated with brain injuries. Said agent may be an antibody, Fab fragment, or aptamer that is specific for an antigenic site present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In a situation where brain-specific microvesicles are meant to be withdrawn without a certain degree of selectivity from the systemic circulation of a subject in need thereof, said agent binding the matrix or substrate may be a lectin. Specific methodologies for use of lectins in removal of viruses are described by Tullis in U.S. Patent Application 20040175291 and these methodologies may also be used in part or in whole for practicing the present invention. In various embodiments of the invention, it is important that said systems include means for maintaining the blood at conditions similar to that found in the host, so that upon returning said blood to the host, no adverse reactions occur. In other words, it is within the scope of the invention to use technologies that are known to one skilled in the art to maintain blood at physiological ion concentrations, osmolality, pH, hematocrit, temperature, and flow in order to avoid harm being caused to the subject subsequent to reinfusion of blood treated as disclosed herein. Said technologies are well known to one skilled in the art.

In another embodiment, a system for extracorporeal clearance of microvesicles; either selectively removing brain-specific microvesicles, or non-selectively microvesicles that are found in healthy subjects as well as subjects with a brain injury. The invention comprises several interacting components whose primary purpose is the formation of a functional circuit capable of depleting microvesicles in order to de-repress, or in some cases augment the immune response of a patient with a brain injury. More specifically, a means for separating blood from a subject in need thereof (e.g., a brain injury patient) into plasma and cellular elements is used. Appropriate devices for such separation are available commercially, and well-known to the skilled artisan. They include, for example, the Exorim System, the Fresenius Hemocare Apheresis system, and the Gambo Prisma System. Plasma purified through said separation devices is then run over an array of filtration devices, said filtration possessing a higher affinity towards tumor associated microvesicles in comparison to other molecules. Said filtration includes, in some embodiments, microvesicle binding agents immobilized to a substrate.

In other embodiments, the agent capable of binding the brain-specific or non-brain-specific microvesicles is immobilized to a filter membrane or capillary dialysis tubing, where the plasma passes adjacent to, or through, the membranes to which said agent capable of binding the brain-specific or non-brain-specific associated microvesicles are bound. Suitable filters include those mentioned previously with respect to separation of blood components. These may be the same filters, having immobilized agents capable of binding microvesicles (either tumor associated or non-associated, or may be arranged in sequence, so that the first filter divides the blood components and the secondary, tertiary and additional filter removes one or more of the components of said brain-specific microvesicles.

Conjugation of the agent capable of binding the brain-specific or non-brain-specific microvesicle to said substrate may be accomplished by numerous means known in the art. Said means include avidin-streptavidin, cyanogen bromide coupling, and the use of a linker such as a polyethylene glycol linker. A means of returning the blood together with plasma substantially cleared of brain-specific microvesicles back to said subject is also provided herein. Preferred means are chosen by one of skill in the art based on the desired application, extent of microvesicle removal desired, patient condition, extracorporeal method chosen, and microvesicle-binding agent chosen.

In one embodiment, extracorporeal removal of microvesicles is performed in a patient who has traumatic brain injury, stroke, genetic disorder, neurodegenerative disease or elderly. It is known in the art that traumatic brain injuries, strokes, genetic disorders, neurodegenerative diseases and diseases of the elderly such as Alzheimer's contain antigens that are specific to the relevant brain pathology (e.g. tau and β-amyloid in Alzheimer's patients), expressed on other tissues but overexpressed on brain specific exosomes.

In one embodiment, frequency and length of extracorporeal treatment is performed based on the amount of time (or blood volume) needed for reduction of exosome concentration to a level significant to correlate with reduction in spontaneous T cell apoptosis. In one embodiment a reduction of spontaneous T cell apoptosis by approximately 20% in comparison to pre-extracorporeal treatment values is judged as sufficient. In another embodiment a reduction of spontaneous T cell apoptosis by approximately 50% in comparison to pre-extracorporeal treatment values is judged as sufficient. In another embodiment a reduction of spontaneous T cell apoptosis by approximately 90% in comparison to pre-extracorporeal treatment values is judged as sufficient.

Although assessment of spontaneous T cell apoptosis is used in some embodiments for judging the frequency, and/or time, and/or blood volume needed for extracorporeal treatment, other means of measuring immune responses may be used.

A device contemplated for use in therapeutic protocol such as extracorporeal removal is an affinity capture device as described below.

### Affinity Capture Devices

Some embodiments relate to affinity capture devices including the first and/or second binding agents described herein. Examples of binding agents include proteins such as lectins, antibodies, antigens, aptamers, and fragments thereof, as well as nucleic acids and oligosaccharides. Examples of lectins include *Galanthus nivalis* agglutinin (GNA), *Narcissus pseudonarcissus* agglutinin (NPA) and cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin. Binding agents may bind to a target present in a biological medium. Examples of targets include the biomarkers described herein. Preferred binding agents include a first binding agent such as, an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and/or a second binding agent that binds to said extracellular vesicle or exosome at a site that is distinct from a site to which said first binding agent binds said extracellular vesicle or exosome, such as a second binding agent that is specific for an MHC class I or an MHC class II antigen (preferably the second binding agent binds to a non-polymorphic region of an MHC I or MHC II molecule). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). As used herein the term "biological medium" is a broad term and can refer to fluid samples comprising biological material. Examples of biological media include materials such as blood, blood derivatives e.g., serum. More examples include urine, sputum, semen, saliva, tissue fluid, ascites fluid, amniotic fluid, and the like. More examples of biological media include tissue extracts, and cell culture medium.

Some embodiments can utilize devices described in International Publication No. WO 2009/023332. Some embodiments include the use of an affinity cartridge such as that found in the Hemopurifier® described below in greater detail. Devices of this general type are disclosed in U.S. Patent No. 4,714,556, U.S. Patent No. 4,787,974 and U.S. Patent No. 6,528,057. In such devices, a biological medium can be passed through the lumen of a hollow fiber membrane, wherein the first or second binding agent is located in the extralumenal space of the cartridge, which forms an approach to accept and immobilize biomarkers. Thus, the device retains exosomes having the biomarkers bound by the binding agent while allowing other biological media components to pass through the lumen. Preferred binding agents for use with these devices and methods include a first binding agent such as, an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and/or a second binding agent that binds to said extracellular vesicle or exosome at a site that is distinct from a site to which said first binding agent binds said extracellular vesicle or exosome, such as a second binding agent that is specific for an MHC class I or an MHC class II antigen (preferably the second binding agent binds to a non-polymorphic region of an MHC I or MHC II molecule). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

One embodiment of an affinity device, described in detail below with reference to the Hemopurifier®, includes multiple channels of hollow fiber membrane that forms a filtration chamber. An inlet port and an effluent port are in communication with the filtration chamber. The membrane is preferably an anisotropic membrane with the tight or retention side facing the source of a biological medium, in other words, facing the oncoming flow of a biological medium. The membrane is formed of any number of polymers known to the art, for example, polysulfone, polyethersulfone, polyamides, polyimides, and cellulose acetate. In other embodiments, the porous membrane is a sheet, rather than a channel. The sheet can be flat, or in some other configuration, such as accordion, concave, convex, conical, etc., depending on the device. In some embodiments, the membrane has pores with a mean diameter of, of less than, or of more than, 1950 nm, 1900 nm, 1850 nm, 1800 nm, 1750 nm, 1700 nm, 1650 nm, 1600 nm, 1550 nm, 1500 nm, 1450 nm, 1400 nm, 1350 nm, 1300 nm, 1250 nm, 1200 nm, 1150 nm, 1100 nm, 1050 nm, 1000 nm, 950 nm, 900 nm, 850 nm, 800 nm, 750 nm, 700 nm, 650 nm, 640 nm, 630 nm, 620 nm, 610 nm, 600 nm, 590 nm, 580 nm, 570 nm, 560 nm, 550 nm, 540 nm, 530 nm, 520 nm, 510 nm, 500 nm, 490 nm, 480 nm, 470 nm, 460 nm, 450 nm, 440 nm, 430 nm, 420 nm, 410 nm, 400 nm, 390 nm, 380 nm, 370 nm, 360 nm, 350 nm, 340 nm, 330 nm, 320 nm, 310 nm, 300 nm, 290 nm, 280 nm, 270 nm, 260 nm, 250 nm, 240 nm, 230 nm, 220 nm, 210 nm, 200 nm, 190 nm, 180 nm, 170 nm, 160 nm, 150 nm, 140 nm, 130 nm, 120 nm, 110 nm, 100 nm, 90 nm, or 85 nm, which will allow passage of macromolecules, exosomes, viral particles, and fragments thereof, but not most cellular components of a biological medium. In other embodiments, the membrane has pores in a range between any two pore diameters recited above.

In particular embodiments, the membrane can have pores 200-500 nm in diameter, more preferably, the pore size is 600 nm, which will allow passage of macromolecules, exosomes, viral particles, and fragments thereof, but not most cellular components of a biological medium, e.g., blood and blood cells (red blood cells 10,000 nm diameter, lymphocytes 7,000-12,000 nm diameter, macrophages 10,000-18,000 nm diameter, thrombocytes 1000 nm). Optionally, by selecting a pore size that is smaller than the diameter of the cellular components of a biological medium, the membrane excludes substantially all cells from passing through the pores and entering the extrachannel or extralumenal space of the device that contains the binding agent. In some embodiments, a pore size is selected that is smaller than only some blood cell types.

In another embodiment, the device comprises a processing chamber having one or more binding agents disposed within the processing chamber, wherein said binding agents binds desirable biomarkers indicative of brain injury. Preferred binding agents include a first binding agent such as, an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and/or a second binding agent that joins to said extracellular vesicle or exosome at a site that is distinct from a site to which said first binding agent binds said extracellular vesicle or exosome, such as a second binding agent that is specific for an MHC class I or an MHC class II antigen (preferably the second binding agent binds to a non-polymorphic region of an MHC I or MHC II molecule). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The biological medium can directly contact the affinity capture medium. In other embodiments, the device has a porous membrane which divides the chamber into one or more portions, such that the binding agent is located in only a portion of the chamber. The preferred device utilizes hollow channel fiber membranes, but one or more sheets of membranes that divide the chamber are also contemplated. Where a membrane is used, the biological medium is filtered by the membrane, such that some portion of the biological medium is excluded from the portion of the chamber containing the binding agent (e.g., blood cells or other large cells which cannot pass through the pores of the membrane).

In some embodiments, the binding agent can include proteins, for example, lectin, antibody, and antigen. The technology to immobilize proteins in dialysis-like cartridges has been developed (Ambrus et al., Science 201(4358): 837-839, 1978; Ambrus et al., Ann Intern Med 106(4): 531-537, 1987; Kalghatgi et al. Res Commun Chem Pathol Pharmacol 27(3): 551-561, 1980). An illustration of preparing proteins for immobilization to the hollow fibers for the method of the present invention is presented in U.S. Patent No. 4,714,556, U.S. Patent No. 4,787,974, and U.S. Patent No. 5,528,057.

For binding of binding agents, e.g., proteins, to the membrane, the polymers of the membrane are first activated, for example, made susceptible for combining chemically with proteins, by using processes known in the art. Any number of different polymers can be used. To obtain a reactive polyacrylic acid polymer, for example, carbodiimides can be used (Valuev et al., 1998, Biomaterials, 19:41-3). Once the polymer has been activated, the proteins can be attached directly or via a linker to form in either case an affinity matrix. Suitable linkers include, but are not limited to, avidin, streptavidin, biotin, protein A, and protein G. The proteins can also be directly bound to the polymer of the membrane using coupling agents such as bifunctional reagents, or can be indirectly bound. In one embodiment, the lectin, GNA, covalently coupled to agarose can be used to form an affinity matrix.

In some embodiments, a protein is attached to a substrate instead of, or in addition to, the membrane. Suitable substrates include, but are not limited to, silica (e.g. glass beads, sand, diatomaceous earth) polysaccharides (e.g. dextran, cellulose, and agarose), proteins (e.g. gelatin) and plastics (e.g. polystyrenes, polysulfones, polyethersulfones, polyesters, polyurethanes, polyacrylates and their activated and native amino and carboxyl derivatives). The protein can be bound to the substrates through standard chemical means, either directly, or through linkers such as C2 to C>20 linear and branched carbon chains, as well as the plastics, other proteins and polysaccharides listed above. For most synthetic purposes, C18 is the preferred upper limit but the chains can be added together for solubility reasons. Preferred linkers include: C2 to C18 dicarboxylates, diamines, dialdehydes, dihalides, and mixtures thereof (e.g. aminocarboxylates) in both native and activated form (e.g. disuccinimidyl suberimidate (DSS)). In some embodiments, one or more substrates can be used as linkers, alone or in combination with the substances listed as linkers. For example, dextran can be attached to sand, and additional linkers can then optionally be added to the dextran. The Hemopurifier® described below includes an example of an affinity capture device.

### HEMOPURIFIER®

The Hemopurifier® is a medical device that selectively targets the removal of infectious viruses and immunosuppressive proteins from the entire circulatory system (*See, e.g.,* U.S. Pat. No. 7226429). It has been discovered that devices such as the Hemopurifier® capture tumor-secreted exosomes that suppress the immune system of those afflicted with cancer (*See e.g.,* U.S. Pat. App. No. 20090304677). Prior to this discovery, a therapeutic strategy to directly inhibit or reverse the immunosuppressive destruction caused by exosomes did not exist in cancer care. By eliminating this mechanism, the Hemopurifier® can fill an unmet clinical need and provide the benefit of an immune-based therapy without adding drug toxicity or interaction risks to established and emerging treatment strategies.

Human studies have documented the ability of the Hemopurifier® to safely reduce viral load in Hepatitis-C virus (HCV), Human Immunodeficiency Virus (HIV), and cytomegalovirus (CMV) infected patients without the administration of antiviral drugs. *In vitro* studies verify that the Hemopurifier® has broad-spectrum capabilities against bioterror and emerging pandemic threats. These studies have confirmed the capture of Dengue Hemorrhagic Virus, Ebola Hemorrhagic Virus, Lassa Hemorrhagic Virus, West Nile Virus, H5N1 Avian Influenza Virus, 2009 H1N1 Influenza Virus, the reconstructed Spanish Flu of 1918 Virus, and Monkeypox Virus, which serves as a model for human Smallpox infection.

In some embodiments, the Hemopurifier® is a selective filtration device containing an affinity cartridge with binding agents that tightly bind to high-mannose structures unique to the surface of exosomes produced by cancer and glycoproteins residing on the envelope of viruses. Agents can include some biomarkers (*See, e.g.,* Int. Pat. Pub. No. WO 2010/065765). These agents are immobilized around approximately 2800 porous hollow fibers that run the interior length of the device. The resulting design enhances the ability to separate both exosome and viral targets away from blood cells so they can then be selectively and permanently removed from the circulatory system. In some applications, blood circulation is established into the Hemopurifier® via a catheter or other blood access device. Once blood flow has been established, treatment benefit is immediate as the entire circulatory system can pass through the Hemopurifier®, in some embodiments, in as little as 15 minutes. However, there remains a need to measure the concentration of brain specific exosomes in patient samples. Embodiments of the present invention satisfy this need by providing methods, compositions and kits for quantifying a broad spectrum of brain specific exosomes in samples. Modification of the Hemopurifier® can be accomplished to accommodate the binding agents described herein e.g., a first binding agent such as, an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and/or a second binding agent that joins to said extracellular vesicle or exosome at a site that is distinct from a site to which said first binding agent binds said extracellular vesicle or exosome, such as a second binding agent that is specific for an MHC class I or an MHC class II antigen (preferably the second binding agent binds to a non-polymorphic region of an MHC I or MHC II molecule). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). By these modifications, efficient removal of brain-specific exosomes having these biomarkers is accomplished.

### Illustrations

Antibodies specific to markers or antibodies or lectins that bind exosomes en masse can be affixed to a substrate, preferably immobilized using a variety of technologies. Preferred binding agents for immobilization include a first binding agent such as, an antibody, such as a monoclonal antibody, or a binding fragment thereof (e.g., a Fab fragment or a fragment having a CDR domain), a lectin (e.g., *Galanthus nivalis* agglutinin (GNA), *Narcissus pseudonarcissus* agglutinin (NPA), Concanavalin A or cyanovirin, *Sambucus nigra* lectin, ricin from *Ricinus communis* (RCA), *Dolichos biflorius* lectin, *Ulex Europaeus* lectin, *Vicia villosa* lectin, *Griffonia simplicifolia* lectin, *Solanium tuberosum* lectin, *Lycopersicon esculentum* lectin, *Datura stramonium* lectin, *Erythrina cristagalli* lectin, Soybean lectin, Peanut agglutinin, Wheat germ agglutinin, or Jacalin lectin), or an aptamer, such as a DNA aptamer that is specific for an antigenic site or epitope present on a tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule and/or a second binding agent that joins to said extracellular vesicle or exosome at a site that is distinct from a site to which said first binding agent binds said extracellular vesicle or exosome, such as a second binding agent that is specific for an MHC class I or an MHC class II antigen (preferably the second binding agent binds to a non-polymorphic region of an MHC I or MHC II molecule). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments).

In one embodiment, brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules are covalently coupled to agarose (preferably colored agarose) using cyanogen Bromide and cyanogen bromide (CNBr) activated agarose according to Cuatrecasas, et al (Cuatrecasas, Wilchek and Anfinsen. Proc Natl Acad Sci USA 61(2): 636-643, 1968). Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In brief, 1 ml of antibody at a concentration of 10 mg/ml in 0.1M NaHCO3 pH 9.5 is added to 1 ml CNBr activated agarose microspheres, which can include a dye (Sigma, St. Louis, Mo.) and allowed to react overnight in the cold. Care must be taken to maintain alkaline pH to prevent the potential release of HCN gas. When the reaction is complete, unreacted materials are aspirated and the antibody coupled agarose is washed extensively with sterile cold PBS. The antibody agarose affinity matrix is then stored cold until ready for use. The antibody agarose affinity matrix can be incorporated into a lateral flow diagnostic device.

In another embodiment, an antibody covalently coupled to glass beads, preferably colored glass beads, via Schiffs Base and reduction with cyanoborohydride is prepared. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The affinity matrix is prepared by a modification of the method of Hermanson (Hermanson. Bioconjugate Techniques: 785, 1996). Anti-tau monoclonal antibody dissolved to a final protein concentration of 10 mg/ml in 0.1M sodium borate pH 9.5 is added to aldehyde derivatized silica glass beads (BioConnexant, Austin Tex.). The reaction is most efficient at alkaline pH but will go at pH 7-9 and is normally done at a 2-4 fold excess of protein over coupling sites. To this mixture is added 10 µl 5M NaCNBH3 in 1N NaOH (Aldrich, St Louis, Mo.) per ml of coupling reaction and the mixture allowed to react for 2 hours at room temperature. At the end of the reaction, remaining unreacted aldehyde on the glass surfaces are capped with 20 µl 3M ethanolamine pH 9.5 per ml of reaction. After 15 minutes at room temperature, the reaction solution is decanted and the unbound proteins and reagents removed by washing extensively in PBS. The matrix is then stored in the refrigerator until ready for use. Antibodies, such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule can be conjugated or joined to glass beads, preferably colored glass beads using this method. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The antibody glass bead affinity matrix can be incorporated into a lateral flow diagnostic device.

In another embodiment, brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) are covalently coupled to Chromosorb (Diatomaceous Earth) using glutaraldehyde. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Preparation of aminated diatomaceous earth is accomplished using γ-aminopropyl triethoxysilane (GAPS) (Sigma Chemical, St. Louis, Mo.) and Chromosorb 60/80 mesh. Although other grades of diatomaceous earth may be used, Chromosorb of this mesh size (200-300 microns in diameter) is often used to prevent small particulates from entering the sample through the largest available pore sizes found in hollow-fiber cartridges used for plasma separation (^{∼}0.5 micron). Amino Chromosorb is prepared by suspension in an excess of 5% aqueous solution of GAPS in an overnight reaction. Aminated-Chromosorb is washed free of excess reagent with water and ethanol and dried overnight in a drying oven to yield an off white powder. One gram of the powder is then suspended in 5 ml 5% glutaraldehyde (Sigma) for 30 minutes. Excess glutaraldehyde is then removed by filtration and washing with water until no detectable aldehyde remains in the wash using Schiffs reagent (Sigma Chemical). The filter cake is then resuspended in 5 ml of Sigma borohydride coupling buffer containing 2-3 mg/ml of the antibody and the reaction allowed to proceed overnight at 4 degrees C. At the end of the reaction, excess antibody is washed off and the remaining aldehyde reacted with ethanolamine as described. After final washing in sterile PBS, the material is stored cold until ready for use. The antibody Chromosorb affinity matrix can be incorporated into a lateral flow diagnostic device.

In another embodiment brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) are covalently coupled to polyacrylate beads using Glutaraldehyde and Azide. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The antibodies are dissolved in a concentration of 50-200 mg/ml with human serum albumin in a phosphate-buffered aqueous medium of pH 7.0. Glutaraldehyde at a concentration of 0.05-10% is added to the solution which is then incubated for 1-24 hours, but preferably 12 hours, at 4 degree. C. Excess glutaraldehyde that remains in the reaction mixture is removed by addition of glycine, or other suitable compounds known in the art, to the solution at the end of incubation. This solution is then diafiltered through a membrane having a minimal retentively value of 500,000 molecular weight. The diafiltered antibody-bearing product is dissolved in saline or dialysis fluid. To obtain a reactive polymer to act as a substrate for the antibody, polyacrylic acid polymer beads (≦1 micron in diameter) are activated by the azide procedure. The ratios of antibody to reactive polyester are selected to avoid excessive reaction. If this ratio is appropriately adjusted, the spacing of the antibody along the polymer chain will allow a binding of the antibody with the antigen found on microvesicle without untoward steric hindrances and the antibody conjugate is intended to remain soluble.

Said antibody conjugates are subsequently loaded into a hollow fiber filter cartridge, on the exterior of said hollow fibers. The external filling ports are then sealed. This allows for passage of blood cell components through the lumen of said hollow fibers. Blood plasma containing the microvesicles convects and diffuses through pores in the hollow fibers into the extralumenal space where it contacts the antibody-polyacrylate conjugates. Treated plasma inside the cartridge diffuses back into the general circulation leaving the microvesicles attached to the insolubilized antibody. The antibody polyacrylate affinity matrix can also be incorporated into a lateral flow diagnostic device.

In another embodiment, a preparation of chemically immobilized brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) on nylon is prepared. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The method consists of serial treatments with HCl, polyethylene imine, and maleic anhydride methylvinyl ether copolymer, which results in the stable immobilization of sufficient amounts of antibodies on a nylon filter, which may be incorporated into a lateral flow device or a diagnostic test strip. In one embodiment, Polyethylenevinyl-acetate slips are immersed in 15% (wt/vol) NaOH in 80% (vol/vol) methanol at 58°C for 2 h, washed with water, heated in 2% (vol/vol) aminoacetal in 0.5 N HCl at 58°C for 5 h, washed and dried, soaked in 1% (wt/vol) maleic anhydride methylvinyl ether copolymer (MAMEC) in water-free acetone at 25°C for 2.5 h, and dried. Polyurethane slips are heated in water at 80°C for 6 h, washed with distilled water, dried, treated in 1% (wt/vol) MAMEC in water-free acetone at 25°C for 2.5 h, washed with acetone, and then dried. Polyvinyl chloride slips are immersed in 1% (wt/vol) MAMEC in water-free acetone at 25°C for 2.5 h, washed with acetone, and then dried. MAMEC is introduced onto nylon slips. After MAMEC is introduced onto various polymeric slips, the antibodies (protein concentration, 2 µg/ml) suspended in phosphate-buffered saline are immobilized on the slips at 25°C overnight.

A lateral flow diagnostic device comprising brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) can be prepared using techniques well established in the art (e.g., utilizing approaches employed by Millipore™ Rapid Lateral Flow Test Strips and Bangs Laboratories, Inc. TechNote 303. Exemplary binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). By one approach a first binding agent such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) having a detectable moiety (e.g., a colored bead) is immobilized to the nylon membrane at a discrete zone and a second binding agent, which may be an antibody, fragment thereof or lectin that is specific for an epitope or antigen on MHC I or II is immobilized to the nylon membrane at a second discrete zone. The nylon membrane having the binding agents is then incorporated into a lateral flow device. When the nylon is contacted with a biological sample, including urine, blood, plasma, or cerebral spinal fluid that comprises a brain-specific extracellular vesicle or exosome, a biological complexes comprising the first binding agent joined to the brain-specific extracellular vesicle or exosome and the second binding agent are formed.

In a further embodiments, elective removal of exosomes from blood may be accomplished after utilizing one of the diagnostic approaches described herein using plasmapheresis combined with affinity capture using any of the matrices described above. Plasmapheresis is done using either centrifugal separation or hollow-fiber plasma separation methods. The blood circuit is anticoagulated with a continuous heparin infusion in the afferent limb. The activated clotting time (ACT) is measured every hour, and the heparin infusion is adjusted to maintain the ACT between 160 and 180 seconds.

The plasma obtained from the patient may be discarded and replaced with a combination of normal saline and fresh plasma from healthy donors (i.e. plasma exchange). Alternatively, the plasma containing the microvesicles can be pumped at 60-100 ml/min over the affinity matrix which captures the exosomes. The cleaned plasma may then be reinfused into the patient.

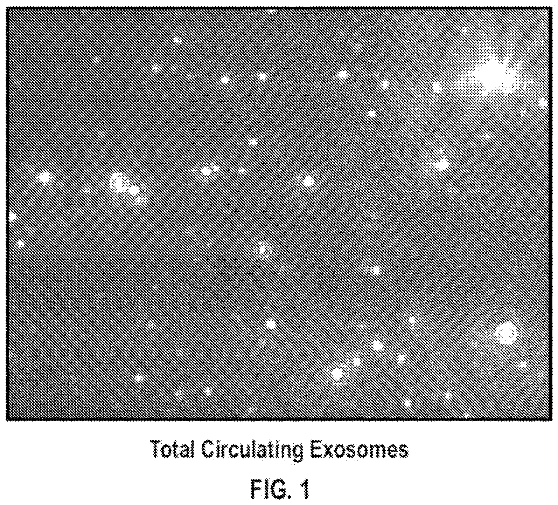

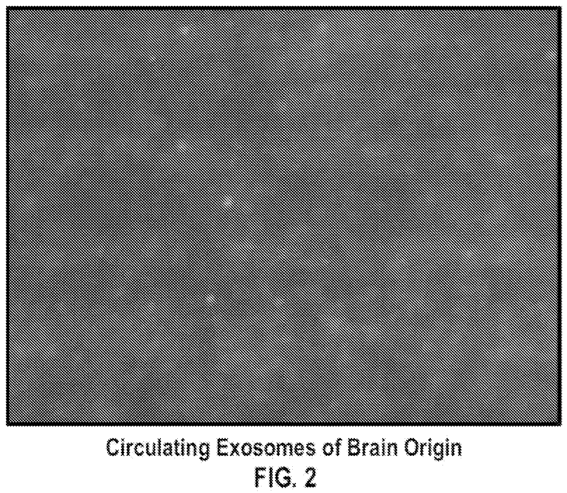

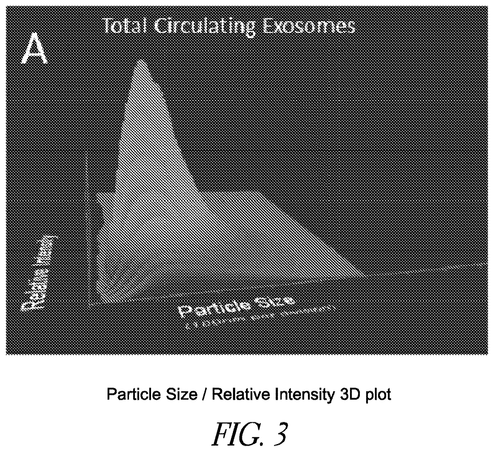

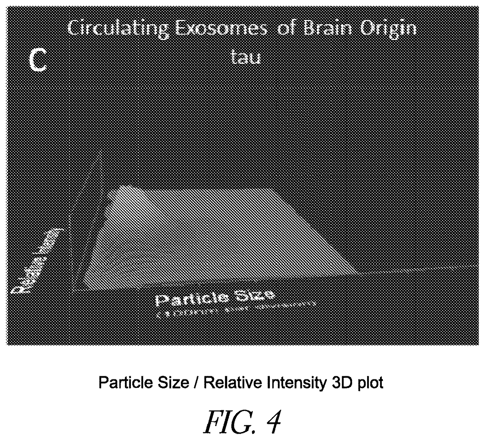

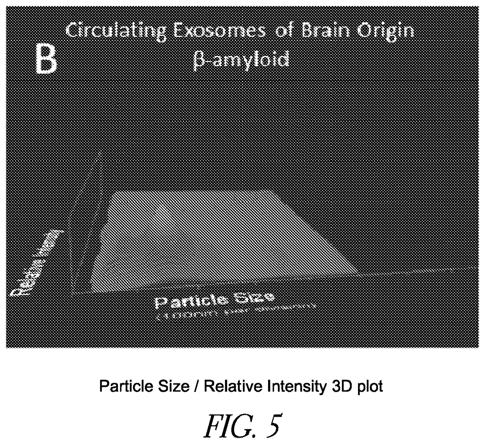

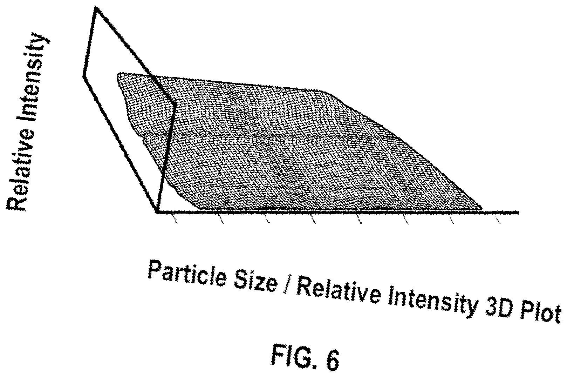

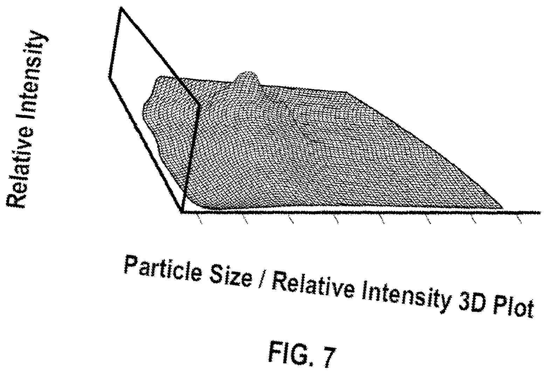

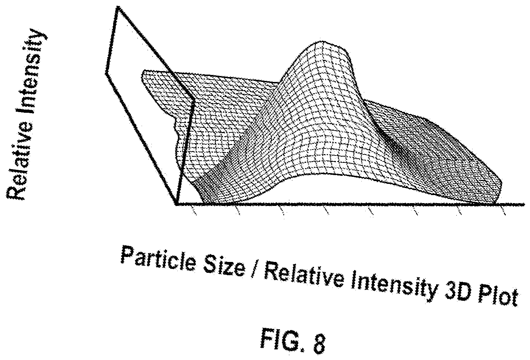

In another embodiment, identification of circulating exosomes using Streptavidin-conjugated Qdots and NS300 was performed. Exosomes in circulation of a patient with brain cancer were labeled with a first biotinylated primary antibody specific for an antigen present on β-amyloid (β-amyloid antibody (clone 20.1), which is a mouse monoclonal IgG_{2b} raised against amino acids 1-40 of human β-amyloid) and a second biotinylated primary antibody specific for an antigen present on tau (tau antibody (clone D-8), which is a mouse monoclonal IgG_{2b} raised against amino acids 1-150 of human tau). The exosomes were then visualized with streptavidin-conjugated quantum dots (Qdots). The streptavidin Qdots, which are fluorescent at 655nm, specifically bind to the biotin on the primary antibody and were detected and quantified using NanoSight NS300. FIGS. 1 and 3 depict the total exosomes in a blood sample identified by using the light scatter mode. FIGS. 2 and 5 show the identification and quantification of exosomes having an antigen present β-amyloid from a biological sample obtained from the brain cancer patient. FIG. 4 shows the identification and quantification of exosomes having an antigen present tau from a biological sample obtained from the brain cancer patient. FIG. 6 shows the identification and quantification of exosomes having an antigen present glycoprotein A2B5 from a biological sample obtained from the brain cancer patient (detected using an Anti-A2B5 antibody [clone 105], which is a mouse monoclonal antibody raised against full length human A2B5). FIG. 7 shows the identification and quantification of exosomes having an antigen present S100 β from a biological sample obtained from the brain cancer patient (detected using a S100 β chain antibody (clone 9A11B9), which is a mouse monoclonal IgG₁ raised against the full length recombinant human S100 β chain protein). FIG. 8 shows the identification and quantification of exosomes having an antigen present autoreactive IgG from a biological sample obtained from the brain cancer patient.

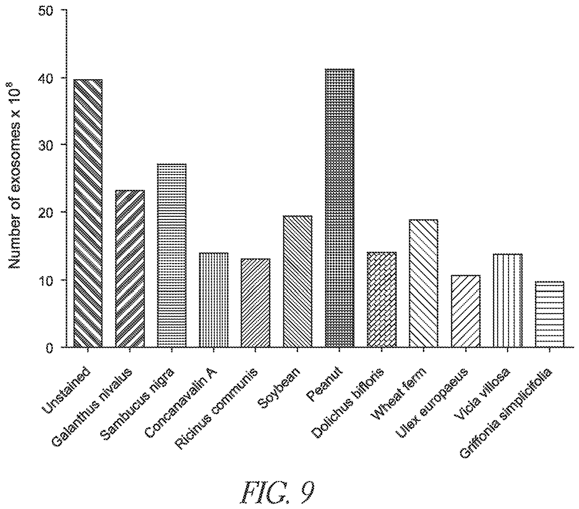

FIG. 9 is a graphical representation of the number of exosomes x 10⁸ from a patient with brain cancer. The exosome is bound by a specific lectin, as indicated on the x-axis. The lectin is biotinylated. The exosome is also bound with a fluorescent indicator, a streptavidin-labeled quantum dot. The quantity of exosome is determined using a NanoSight NS 300 defined in fluorescent mode.

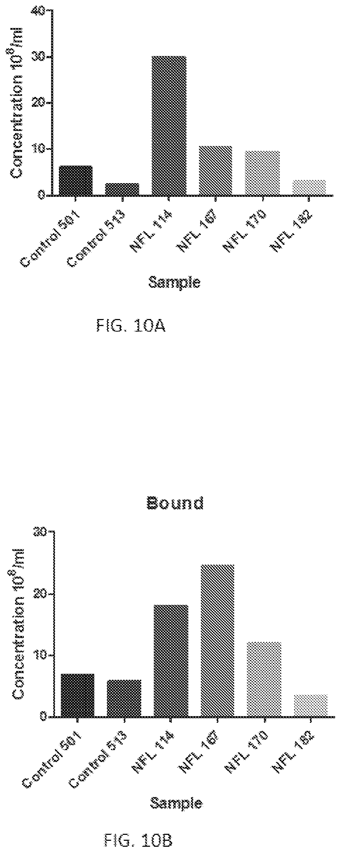

FIGS. 10A and 10B are graphical representations of the concentration of tau-associated exosomes. Samples were obtained from control patients and from professional football players. Extracellular vesicles were initially isolated based on hydrodynamic diameter, and incubated with agarose beads having RCA lectin coupled thereto, in Pierce snap cap spin columns. Samples were spun down in the columns, and the unbound samples were collected. The columns contained a resin, wherein the resin allows a range of particle sizes to pass through. The extracellular vesicles diameter can be in the range of 10 nm to 1,000 nm, or any number between. Bound samples were eluted with glycine-HCl buffer and collected. Unbound and bound samples were then stained with mouse anti-tau antibody and conjugated to anti-mouse quantum dots for fluorescent detection using NanoSight NS 300. FIG. 10A depicts the concentration of tau-associated exosomes found in the unbound eluent. FIG. 10B depicts the concentration of tau-associated exosomes found in the bound sample. As shown, particular professional football players had a significantly greater amount of extracellular vesicles comprising tau than control individuals. The data also indicate that should a subject have greater than or equal to 7, 8, 9, or 10 x 10⁸ /ml extracellular vesicles comprising tau, such subject is at risk for having traumatic brain injury (TBI) such as chronic traumatic encephalopathy (CTE) or other brain diseases, such as Alzheimer's disease. Accordingly, the amount of exosomes having tau or the amount of tau immobilized on a support in a control zone utilized in one or more of the diagnostic devices described herein is at a concentration greater than or equal to 7, 8, 9, or 10 x 10⁸ /ml as this concentration of tau indicates that the tested subject is at risk for having traumatic brain injury (TBI) such as chronic traumatic encephalopathy (CTE) or other brain diseases, such as Alzheimer's disease.

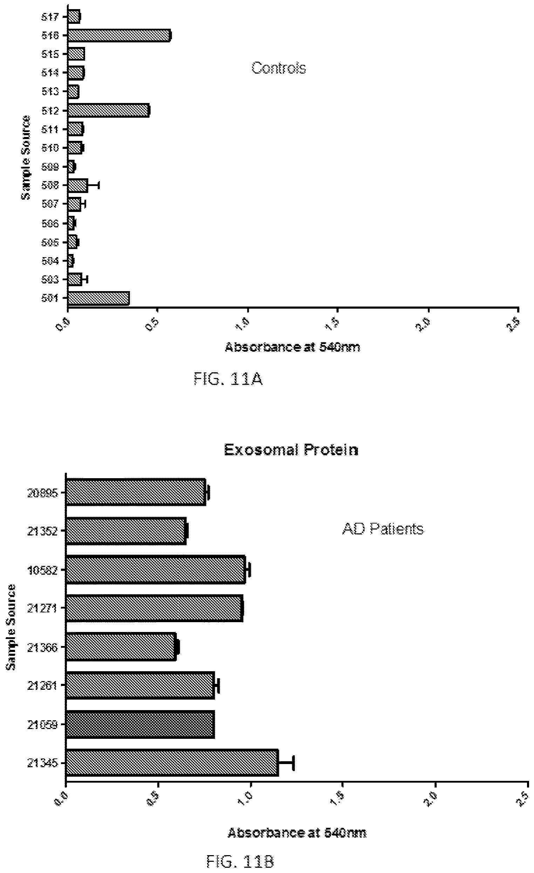

The amount of total protein bound to exosomes was also determined. FIG. 11A depicts the graphical representation of total protein bound to exosomes in control patients. Exosomes were isolated from plasma samples by chromatographic isolation based on the hydrodynamic diameter of the exosomes. Chromatographic isolation of exosomes is accomplished by contacting the sample with a resin having lectin bound thereto, as described previously. The total protein content of protein associated with the isolated exosomes was determined using the Bradford assay.

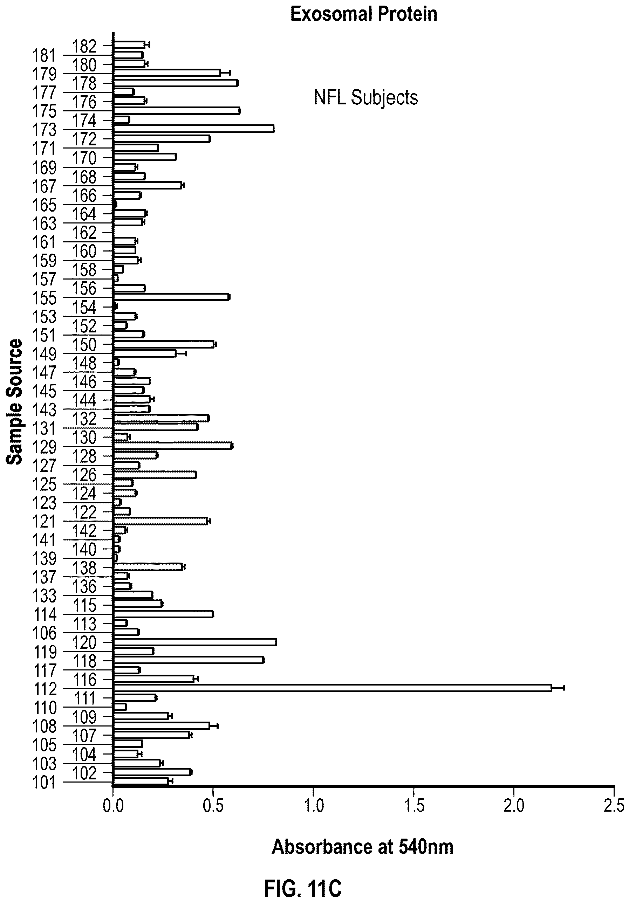

FIG. 11B was performed as described for 11A, but the plasma samples were obtained from patients diagnosed with Alzheimer's disease. FIG. 11C was performed as described, but the plasma samples were obtained from professional football players.

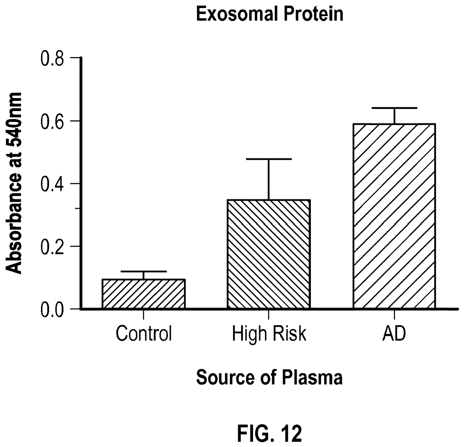

FIG. 12 represents the total protein associated with exosomes as determined by Bradford assay, and was performed as described for FIG. 11A.

In some embodiments, a diagnostic device is provided, wherein the device is useful for the detection of the presence of undesired biomarkers or extracellular vesicles, such as exosomes that contain undesired biomarkers in a biological sample from a subject, wherein said biomarkers are indicative of the presence of brain injury and/or for a brain pathology, such as Alzheimer's disease, dementia, and/or CTE. Such biomarkers can include, e.g., tau, β-amyloid, or glycosylated or phosphorylated forms of these molecules, and/or a fragment thereof or any combination thereof. The device is preferably a POC diagnostic device that allows for the rapid, convenient, inexpensive, and portable application for diagnosis of brain injury. In some embodiments, a kit is provided having a diagnostic device as described herein and one or more spin columns loaded with a size exclusion resin, which can be used to isolate an exosome fraction from a biological sample prior to said fraction being applied to one or more of the diagnostic devices described herein.

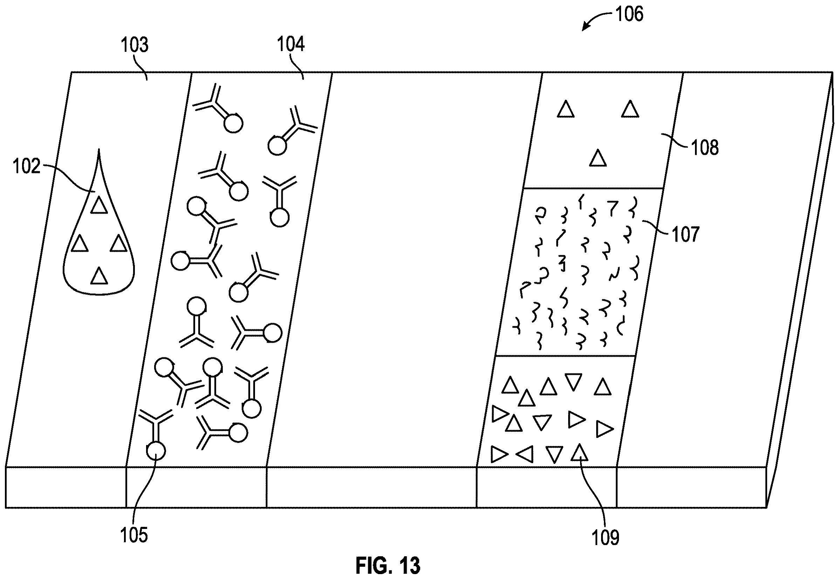

Referring now to FIG. 13, an exemplary POC diagnostic device is shown, wherein the diagnostic device is a LFA. The LFA is comprised of a solid substrate. The solid substrate may be any suitable solid material or membrane, including, for example, cellulose, nitrocellulose, cellulose acetate, polyacrylamide, polysulfone, polyethersulfone, polyamide, polyimide, polyvinylidene fluoride (PVDF), nylon, copolymer, agarose, starch, nylon polyesters, dextran, cross-linked dextran, dextran acrylamide copolymer, cross-linked hydroxymethylmethacrylate, substituted cross-linked polystyrenes, polyvinyl alcohol, wool, metal oxides, porous ceramics coated with hydrophilic organic polymers, and glass.

Sample 102 from a patient who has or is suspected of having a brain injury is deposited into the sample reservoir 103. The sample 102 may be any biological sample that may contain the analyte. Thus, the sample 102 may be selected from the following non-limiting group of body samples obtained from a subject or patient: cerebrospinal fluid, urine, saliva, serum, breast milk, sweat, saliva, ascites fluid, plasma, and whole blood. One or more of these body samples contains or is suspected of containing analyte. Here, the analyte is an undesired biomarker, which may be present in the sample accompanying an extracellular vesicle, such as an exosome, and such a biomarker can be a peptide, or protein or fragment thereof that indicates the presence of a brain injury, including the presence or proclivity for chronic traumatic encephalopathy (CTE) and Alzheimer's disease (e.g., tau, β-amyloid, or glycosylated or phosphorylated forms of these molecules, and/or a fragment thereof or any combination thereof). In some embodiments the biological sample from said patient is first subjected to column chromatography, e.g., by size-exclusion spin column, such as Chromaspin 200 or Chromaspin 400, and the eluent having the exosomes, which contain the biomarker being screened for, is applied to the diagnostic device. In some embodiments, the size exclusion resin or a small resin is used, wherein such resins have a lectin (e.g., *Galanthus nivalis* agglutinin (GNA), *Narcissus pseudonarcissus* agglutinin (NPA), Ricin (RCA), Concanavalin A or cyanovirin) coupled thereto such that a form of size exclusion and/or affinity chromatography is conducted prior to applying the eluent to a diagnostic device, as described herein. The bound exosomes can be eluted using a glycine-HCl buffer or other suitable lectin elution buffer. Preferably, such spin columns and, optionally elution buffer, are included in kits having the diagnostic devices described herein.

The analyte migrates with the liquid front through capillary action to the conjugate zone 104. The conjugate zone 104 is so termed because conjugate 105 is deposited on this region prior to sample application on the device. The conjugate 105 is a labeled anti-tau or anti-β-amyloid antibody or a binding fragment thereof. In some embodiments, the antibody or binding fragment thereof is specific for a glycosylated tau and/or a glycosylated β-amyloid but does not appreciably bind to or cross-react with an unglycosylated tau and/or unglycosylated β-amyloid or a form of tau or β-amyloid that lacks mannose sugar residues. In other embodiments, the antibody or binding fragment thereof is specific for a phosphorylated tau and/or a phosphorylated β-amyloid, but does not appreciably bind to or cross-react with unphosphorylated tau and/or unphosphorylated β-amyloid. The antibody or binding fragment thereof is labeled with any suitable label, including colloidal carbon, colloidal gold, a fluorescent label, a quantum dot, a phosphor, a colored particle, a bioluminescent marker, an enzyme label, a paramagnetic particle, or a colored latex particle. A common label for LFA devices are gold nanoparticles, because they are relatively inexpensive and provide easily observable color indications based on the surface plasmon resonance properties of gold nanoparticles.

When the sample 102 enters the conjugate zone 104, the labeled antibody or binding fragment thereof solubilizes into the solution, and the antibody or binding fragment thereof recognizes and binds to cross-reactive analyte, (e.g., tau, β-amyloid, or glycosylated or phosphorylated forms of these molecules, and/or a fragment thereof or any combination thereof) forming a conjugate complex. This complex is therefore comprised of antibody or binding fragment thereof and the biomarker, which may be associated with an extracellular vesicle, such as an exosome containing the selected biomarker for diagnosis. The complex flows by capillary action through the solid substrate to a second zone called the capture zone 106.

In this exemplary embodiment and for illustrative purposes only, the capture zone 106 is comprised of at least three specific regions: the test zone 107, and at least two control zones. However, one of skill in the art would recognize that fewer or greater numbers of zones could be used. The test zone 107 is comprised of immobilized lectin. The lectin may be selected to bind to the biomarker, e.g., tau, β-amyloid, or glycosylated or phosphorylated forms of these molecules, and/or a fragment thereof or any combination thereof, and/or to extracellular vesicles, such as exosomes, especially exosomes carrying the biomarker, when the exosomes enter the test zone. The lectin can be, for example *Galanthus nivalis* agglutinin (GNA), *Narcissus pseudonarcissus* agglutinin (NPA), cyanovirin, or *Ricinus communis* agglutinin (RCA), and in some alternatives is preferably RCA. The trapped biomarker and/or extracellular vesicles having the biomarker are readily observable, detectable, as well as, quantifiable by virtue of the color indication and the intensity or density thereof.

The control zones may have predetermined amounts of immobilized biomarker (e.g., tau, β-amyloid, or glycosylated or phosphorylated forms of these molecules, and/or a fragment thereof or any combination thereof) deposited therein. A first control zone 108 has an amount of immobilized biomarker that is indicative of the amount of biomarker found in healthy patients. A second control zone 109 has an amount of immobilized biomarker that is indicative of the amount of biomarker found in patients with brain injury. In some embodiments, the amount of immobilized biomarker is 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In view of the findings reported herein, the amount of exosomes having tau or the amount of tau immobilized on a support in a control zone utilized in one or more of the diagnostic devices described herein is at a concentration greater than or equal to 7, 8, 9, or 10 x 10⁸ /ml as this concentration of tau indicates that the tested subject is at risk for having traumatic brain injury (TBI) such as chronic traumatic encephalopathy (CTE) or other brain diseases, such as Alzheimer's disease. Excess labeled antibody that did not bind to exosomes binds to the immobilized biomarker at the control zones. Thus, in certain embodiments, at least three distinct signals are detected: the test signal, a healthy control signal, and a diseased-state control signal. The test signal is compared to the two control signals to determine the relative amount of biomarker in the test subject's sample.

In other embodiments of the invention, an external control standard is provided, wherein the standard is a printed color gradient. The printed gradient can be printed on the device itself, preferably near the viewing window, or can be printed elsewhere, for example, in separate materials that accompany a kit having the device. The printed standard may have a gradient of low intensity color to high intensity color, with a gradient of intensity between or it may have only a low intensity color and a high intensity color. The low intensity printed standard is representative of a low amount of biomarker present in the sample and/or indicates a value or amount of biomarker that is commensurate with the value or amount of biomarker found in healthy individuals (a negative control population); whereas the high intensity printed standard is representative of a high amount of biomarker present in the sample and/or indicates a value or amount of biomarker that is commensurate with the value or amount of biomarker found in individuals having a brain pathology, such as Alzheimer's disease and/or CTE (a positive control population).

The color intensity that is observed at the test zone is compared to the standard gradient to determine the relative amount of biomarker present in the test sample. Accordingly, one can immediately discern through visual observation whether the tested individual has an amount of biomarker in the biological sample that is commensurate with the amount of biomarker present in a biological sample obtained and analyzed for a healthy individual or an individual having a brain pathology, such as Alzheimer's disease and/or CTE. The relative amount of biomarker detected in the biological sample from the tested individual, as compared to the printed standard representing the amount of biomarker detectable in a healthy control sample and the control sample from a subject having a brain pathology, such as Alzheimer's disease and/or CTE provides useful clinical and/or diagnostic information even if the amount of color, representative of the amount of biomarker detected in the tested sample does not match the printed standard identically because one can immediately discern whether the tested subject has an amount of biomarker that more closely aligns with or resembles the amount of biomarker present in a healthy patient or a patient having a brain pathology, such as Alzheimer's disease and/or CTE. Should the amount of biomarker detected in the biological sample from the tested individual more closely resemble the printed standard representative of the amount of biomarker found in the healthy individual, therapeutic intervention may not be needed but should the amount of biomarker detected in the biological sample from the tested individual more closely resemble the printed standard representative of the amount of biomarker found in the individual having a brain pathology, such as Alzheimer's disease or CTE, therapeutic intervention may be considered. In view of the findings reported herein, the intensity of the printed standard in a control zone utilized in one or more of the diagnostic devices described herein can be indicative of the signal observed when a concentration of exosomes comprising tau greater than or equal to 7, 8, 9, or 10 x 10⁸ /ml is reached, as this concentration of tau indicates that the tested subject is at risk for having traumatic brain injury (TBI) such as chronic traumatic encephalopathy (CTE) or other brain diseases, such as Alzheimer's disease.

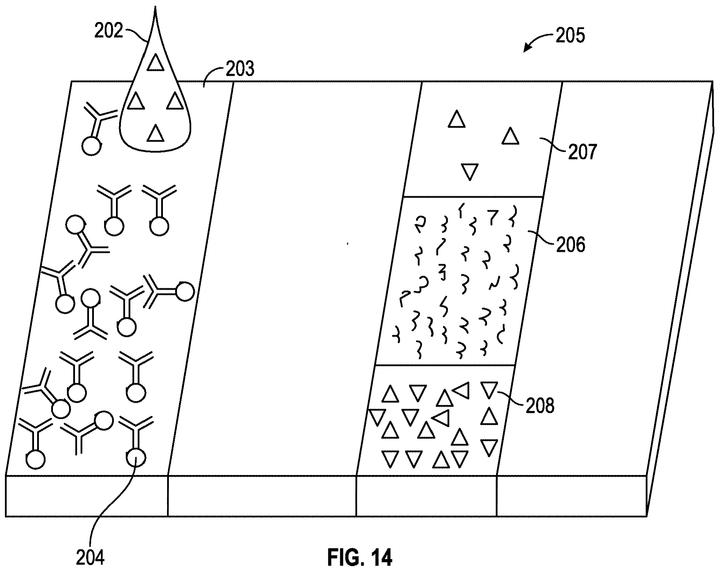

Referring to FIG. 14, an exemplary lateral flow device, or LFA diagnostic device is provided. This device combines the sample reservoir as described above, and the conjugate zone. Thus, this embodiment provides a LFA diagnostic device having a solid substrate, a sample reservoir 203, and a capture zone 205. Sample 202 is applied to the sample reservoir 203, and the placement of the sample results in the solubilization of conjugate, such as labeled antibody 204. The antibody conjugates to the analyte, and the complex flows through capillary action to the capture zone 205. In some embodiments, the capture zone 205 comprises two control zones 207, 208 and a test zone 206. The control zones are indicative of healthy and disease levels of biomarker, and the resulting observable color change at the test zone 206 is compared to the control zones, providing a rapid indication of the presence and prevalence of biomarker in the subject sample. In some embodiments, the amount of immobilized biomarker is 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In view of the findings reported herein, the amount of exosomes having tau or the amount of tau immobilized on a support in a control zone utilized in one or more of the diagnostic devices described herein is at a concentration greater than or equal to 7, 8, 9, or 10 x 10⁸ /ml as this concentration of tau indicates that the tested subject is at risk for having traumatic brain injury (TBI) such as chronic traumatic encephalopathy (CTE) or other brain diseases, such as Alzheimer's disease. In some embodiments the biological sample from said patient is first subjected to column chromatography, e.g., by size-exclusion spin column, such as Chromaspin 200 or Chromaspin 400, and the eluent having the exosomes, which contain the biomarker being screened for, is applied to the diagnostic device. In some embodiments, the size exclusion resin has a lectin (e.g., *Galanthus nivalis* agglutinin (GNA), *Narcissus pseudonarcissus* agglutinin (NPA), Ricin (RCA), Concanavalin A or cyanovirin) coupled thereto such that a form of size exclusion and affinity chromatography is conducted prior to applying the eluent to a diagnostic device, as described herein. The bound exosomes can be eluted using a glycine-HCl buffer. Preferably, such spin columns, and optionally elution buffers, are included in kits having the diagnostic devices described herein.

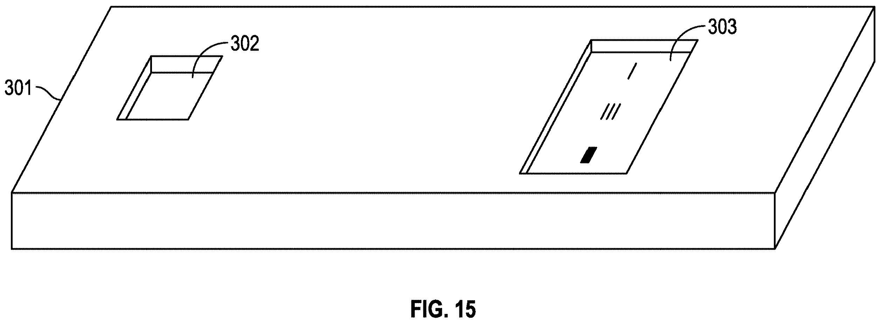

FIG. 15 provides an exemplary lateral flow device or LFA diagnostic device 301, wherein the diagnostic device comprises a processing chamber 302 configured to receive a biological sample from a subject. The device further comprises a solid substrate located within the processing chamber 302. The solid substrate is any suitable material capable of transporting a sample through capillary action, such as a porous membrane. A first porous membrane is located within the processing chamber. A second porous membrane is in fluid communication with the first porous membrane. The second porous membrane comprises mobilizable labeled antibody or a binding fragment thereof that is cross-reactive to the target analyte. Upon application to the processing chamber, the sample contacts the first porous membrane and flows through capillary action to the second porous membrane, whereupon the labeled antibody or binding fragment thereof solubilizes in the sample and forms a conjugate complex with the analyte in the sample.

A third porous membrane is in liquid communication with the second porous membrane, wherein the third porous membrane comprises immobilized lectin thereon. A fourth and fifth porous membrane are also present and in liquid communication with the second porous membrane. The fourth porous membrane has a control amount of biomarker (e.g., tau, β-amyloid, or glycosylated or phosphorylated forms of these molecules, and/or a fragment thereof or any combination thereof) immobilized thereon, wherein the amount of biomarker immobilized thereon is indicative of the amount of biomarker present in the same volume and/or dilution of the same biological sample used in the assay obtained from a healthy individual. The fifth porous membrane also has a control amount of biomarker immobilized thereon, wherein the amount of biomarker immobilized thereon is indicative of the amount of biomarker present in the same volume and/or dilution of the same biological sample used in the assay obtained from a person having a brain pathology, such as CTE and/or Alzheimer's disease.

The sample containing the analyte conjugate complex flows through capillary action from the second porous membrane to the third, fourth, and fifth porous membranes. Conjugated analyte in the sample, e.g., tau, β-amyloid, or glycosylated or phosphorylated forms of these molecules, and/or a fragment thereof or any combination thereof bound to an antibody or binding fragment thereof, which may be in the form of freely available biomarker bound to the antibody or binding fragment thereof or in the form of an extracellular vesicle or exosome having said biomarker, is captured on the third porous membrane, the accumulation of which causes a discernible color change because of the accumulation of gold nanoparticles on the third porous membrane. Excess labeled antibody or binding fragment thereof flows through the fourth and fifth porous membranes, and is captured by the immobilized biomarker on the respective membranes, the accumulation of which provides a discernible color change. The color change on the fourth porous membrane is slight, whereas the color change on the fifth porous membrane is great because lesser amounts of biomarker is immobilized on the fourth porous membrane than on the fifth porous membrane. Finally, the signal detected at the third porous membrane can be compared to the signal or label detected at the fourth and fifth porous membranes. These signals or labels are discernible through a viewing window 303, which permits the visual inspection of the color change at these locations of accumulated labeled antibody or binding fragment thereof, as well as, allowing for quantification of signal or label based on density or intensity of the signal or label. In some embodiments, the amount of immobilized biomarker is 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In view of the findings reported herein, the amount of exosomes having tau or the amount of tau immobilized on a support in a control zone utilized in one or more of the diagnostic devices described herein is at a concentration greater than or equal to 7, 8, 9, or 10 x 10⁸ /ml as this concentration of tau indicates that the tested subject is at risk for having traumatic brain injury (TBI) such as chronic traumatic encephalopathy (CTE) or other brain diseases, such as Alzheimer's disease. In some embodiments the biological sample from said patient is first subjected to column chromatography, e.g., by size-exclusion spin column, such as Chromaspin 200 or Chromaspin 400, and the eluent having the exosomes, which contain the biomarker being screened for, is applied to the diagnostic device. In some embodiments, the size exclusion resin has a lectin (e.g., *Galanthus nivalis* agglutinin (GNA), *Narcissus pseudonarcissus* agglutinin (NPA), Ricin (RCA), Concanavalin A or cyanovirin) coupled thereto such that a form of size exclusion and affinity chromatography is conducted prior to applying the eluent to a diagnostic device, as described herein. The bound exosomes can be eluted using a glycine-HCl buffer. Preferably, such spin columns, and optionally elution buffers, are included in kits having the diagnostic devices described herein.

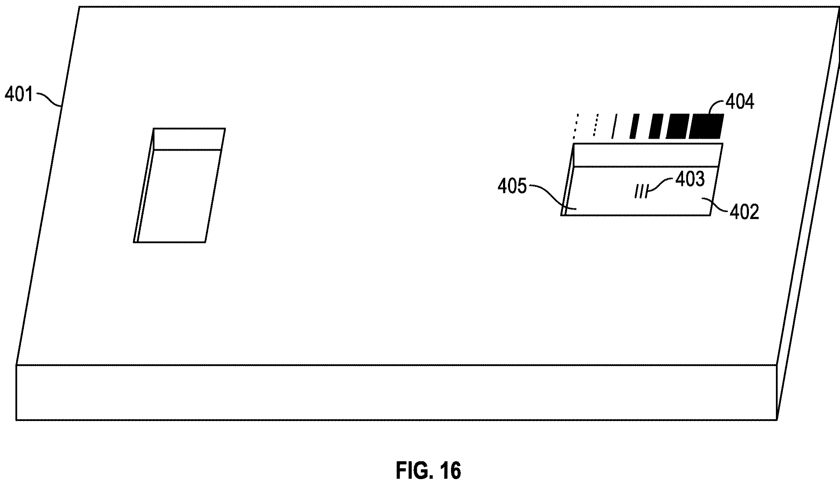

FIG. 16 provides a schematic illustration of an exemplary embodiment of the invention, wherein a lateral flow device, or LFA device 401 is depicted, having a standard color gradient 404 printed thereon. In this embodiment, the LFA does not have one or more control zones for providing standard control levels of biomarker. Instead, the signal 403 detected at the test zone 402 is compared to the printed standard color gradient 404. The printed color gradient 404 comprises a color intensity gradient, and can be printed directly onto the device 401, preferably near a viewing window 405, or it can be printed elsewhere, for example, in separate materials. The printed color gradient 404 has low intensity to high intensity, wherein the low intensity represents low levels of biomarker and high intensity represents high level of biomarker, with a gradient of ranges between low and high. The user performs a visual comparison of the signal 403 detected at the test zone 402 with the printed standard color gradient 404 to determine the relative amount of biomarker that is observed at the test zone 402.

As provided herein, the term intensity refers to the amount of color deposited at a certain location. Intensity can therefore refer to the level of color at a certain point. Intensity therefore refers to the density of color at a certain point. By way of example, when a high concentration of labeled antibody is captured at the test line, then the high density of colored particles, for example, creates a high intensity readout. Thus, in some contexts, intensity and density can be interchanged to similarly describe the concentration of labeled antibody at a specific location.

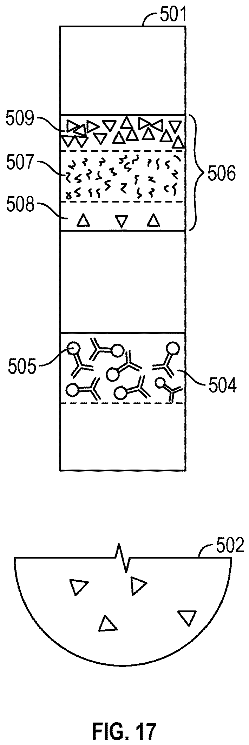

FIG. 17 depicts an exemplary POC diagnostic device, wherein the device is a dipstick diagnostic device 501. The dipstick diagnostic device is a device that provides a simple determination of analyte in a sample of interest. The dipstick comprises a solid substrate, wherein the substrate is any suitable material capable of allowing capillary action through the substrate. An exemplary dipstick device 501 is shown, wherein the dipstick comprises a conjugate zone 504 and a capture zone 506. The conjugate zone 504 comprises conjugate 505, such as labeled antibody or a binding fragment thereof. The capture zone 506 comprises a test zone 507 and two control zones 508, 509, wherein the test zone 507 comprises immobilized lectin and the control zones 508, 509 comprise immobilized biomarker (e.g., tau, β-amyloid, or glycosylated or phosphorylated forms of these molecules, and/or a fragment thereof or any combination thereof). In some embodiments, the amount of immobilized biomarker is 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In view of the findings reported herein, the amount of exosomes having tau or the amount of tau immobilized on a support in a control zone utilized in one or more of the diagnostic devices described herein is at a concentration greater than or equal to 7, 8, 9, or 10 x 10⁸ /ml as this concentration of tau indicates that the tested subject is at risk for having traumatic brain injury (TBI) such as chronic traumatic encephalopathy (CTE) or other brain diseases, such as Alzheimer's disease. In some embodiments the biological sample from said patient is first subjected to column chromatography, e.g., by size-exclusion spin column, such as Chromaspin 200 or Chromaspin 400 and the eluent having the exosomes, which contain the biomarker being screened for, is applied to the diagnostic device. In some embodiments, the size exclusion resin has a lectin (e.g., *Galanthus nivalis* agglutinin (GNA), *Narcissus pseudonarcissus* agglutinin (NPA), Ricin (RCA), Concanavalin A or cyanovirin) coupled thereto such that a form of size exclusion and affinity chromatography is conducted prior to applying the eluent to a diagnostic device, as described herein. The bound exosomes can be eluted using a glycine-HCl buffer. Preferably, such spin columns, and optionally elution buffers, are included in kits having the diagnostic devices described herein.

The dipstick device 501 is dipped into a biological sample 502 obtained from a patient that has or that is suspected of having or is at risk of having a brain injury and/or a brain pathology, such as Alzheimer's disease, dementia, and/or CTE. A biological sample, containing the desired analyte for analysis (e.g., tau, β-amyloid, or glycosylated or phosphorylated forms of these molecules, and/or a fragment thereof or any combination thereof), flows into the substrate to the conjugate zone 504, whereupon the conjugate 505, such as labeled antibody or binding fragment thereof solubilizes into the sample, and cross-reacts with freely available biomarker in the biological sample, as well as, biomarker that is bound to extracellular vesicles, such as exosomes thereby, forming a complex. These complexes flow with the liquid front by capillary action to the capture zone 506, whereupon the complex is bound by one or more immobilized lectins at the test zone 507. Excess labeled antibody or binding fragment thereof is captured in the two control zones 508, 509. The accumulation of labeled antibody or binding fragment thereof provides a visual color change at the capture zone 506, wherein the intensity or density of the color change at the test zone 507 can be compared to the intensity or density of color change at the two control zones 508, 509 so as to determine the relative amount of the analyte in the sample.

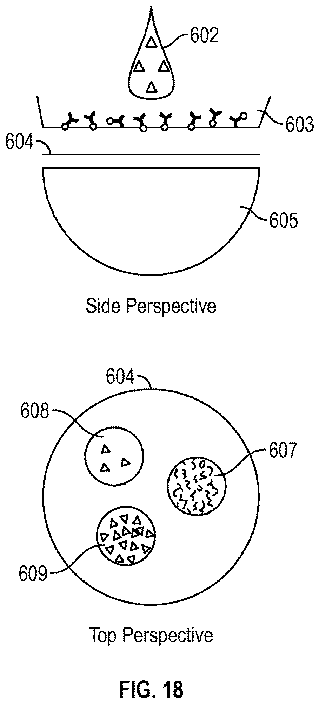

FIG. 18 depicts a POC diagnostic device, wherein the device is a flow-through device (FTD). The FTD is a point of care device wherein sample is applied to a surface, wherein analyte remains on the substrate, but excess sample flows through to an overflow reservoir. FIG. 18 illustrates an exemplary FTD comprising a first substrate 603, a second substrate 604, and an absorbent material 605. The first substrate 603 comprises mobilizable conjugate, such as labeled antibody or a binding fragment thereof against the biomarker. For illustrative purposes only, the second substrate 604 comprises a test zone 607 comprising immobilized lectin, and two control zones 608, 609 comprising immobilized biomarker (e.g., tau, β-amyloid, or glycosylated or phosphorylated forms of these molecules, and/or a fragment thereof or any combination thereof). Sample 602 obtained from a patient having or suspected of having brain injury is placed on the first substrate 603. Upon application, labeled antibody or binding fragment thereof solubilizes into the sample solution, and binds to analyte in the solution, forming a complex. This complex flows with the liquid front through the first substrate 603 to the second substrate 604. The complex binds to immobilized lectin at the test zone 607. In some embodiments, the amount of immobilized biomarker is 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts. In view of the findings reported herein, the amount of exosomes having tau or the amount of tau immobilized on a support in a control zone utilized in one or more of the diagnostic devices described herein is at a concentration greater than or equal to 7, 8, 9, or 10 x 10⁸ /ml as this concentration of tau indicates that the tested subject is at risk for having traumatic brain injury (TBI) such as chronic traumatic encephalopathy (CTE) or other brain diseases, such as Alzheimer's disease. In some embodiments the biological sample from said patient is first subjected to column chromatography, e.g., by size-exclusion spin column, such as Chromaspin 200 or Chromaspin 400, and the eluent having the exosomes, which contain the biomarker being screened for, is applied to the diagnostic device. In some embodiments, the size exclusion resin has a lectin (e.g., *Galanthus nivalis* agglutinin (GNA), *Narcissus pseudonarcissus* agglutinin (NPA), Ricin (RCA), Concanavalin A or cyanovirin) coupled thereto such that a form of size exclusion and affinity chromatography is conducted prior to applying the eluent to a diagnostic device, as described herein. The bound exosomes can be eluted using a glycine-HCl buffer. Preferably, such spin columns, and optionally elution buffers, are included in kits having the diagnostic devices described herein.

Excess labeled antibody or binding fragment thereof binds to immobilized biomarker at the first 608 and second control zones 609. The second substrate 604 may be removed by pulling it out from the FTD for visual inspection. The first control zone 608 comprises an amount of biomarker indicative of healthy subjects, and the second control zone 609 comprises an amount of biomarker indicative of disease subjects. The color indication, intensity, or density on the test zone 607 can be compared to the color indications, intensities, or densities on the two control zones 608, 609 through visual inspection or quantification of the second substrate 604.

Another aspect of the invention is the method of using the device for the detection of analyte (e.g., tau, β-amyloid, or glycosylated or phosphorylated forms of these molecules, and/or a fragment thereof or any combination thereof) in a patient suspected of having a brain injury. Some methods comprise providing any one or more of the devices described above, applying a biological sample from a patient having or suspected of having a brain injury to the device, observing or quantifying a color change at both the test zones and at the control zones, and comparing the intensity of the color change at the test zone with the intensity at the control zones, thereby determining the relative amount of biomarker in the biological sample from the test subject.

Another aspect of the invention is the method of ameliorating a neurodegenerative disease in a test subject who has been determined to have elevated levels of biomarker (e.g., tau, β-amyloid, or glycosylated or phosphorylated forms of these molecules, and/or a fragment thereof or any combination thereof). This method comprises detecting the presence and prevalence of biomarker in a test subject as described above, providing a extracorporeal circulation system, which comprises contacting the whole blood or components thereof with a single or plurality of agents capable of binding microvesicles found within the blood, and returning the blood or components thereof into the subject, whereby the blood contains substantially less neurodegenerative molecules (e.g., tau, β-amyloid, or glycosylated or phosphorylated forms of these molecules, and/or a fragment thereof or any combination thereof) in comparison to the blood or blood components originally residing in the test subject.

Exosomes in circulation of a patient with brain cancer can also be isolated and characterized as follows. A biological sample, preferably blood, urine, or cerebrospinal fluid, is obtained from a patient with brain cancer and the sample is then contacted with a support, preferably a filter, membrane, polymer, or bead, that comprises a lectin joined thereto (e.g., Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin). The non-binding materials are washed from the support and the exosomes that remain bound to the lectin and support are identified. By one approach, biotinylated antibodies, which bind to an antigenic epitope present on an MHC I molecule, are contacted with the bound exosomes, as these antibodies are contemplated to bind to all exosomes that would be present on the lectin bound support. Detection, identification, and quantification of the total number of exosomes bound to the lectin and support can be accomplished with streptavidin having a detectable moiety such as nanogold or Qdots. A second binding reaction is then conducted, wherein exosomes that are bound to the lectin and support are contacted with a second biotinylated antibody specific for an antigenic epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. Exemplary second binding agents that are used in these embodiments include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The binding of the second antibody to the exosomes that remain on the support can then be identified and quantified using a streptavidin having a detectable moiety such as nanogold or Qdots. By comparing the amounts of exosomes detected after the first binding agent (the anti-MHC I antibody) to the amounts of exosomes detected after the second binding agent (the antibody specific for an antigenic epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules), one can determine the ability of a particular lectin (e.g., Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin) to preferentially bind to exosomes that have an antigen present on tau, β-amyloid, S100Beta, Neuron-specific enolase, Glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. It is contemplated that one or more of: Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin will preferentially bind exosomes having an antigen present, wherein the antigen is one or more of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules. For example, it is contemplated that at least, equal to, or a range generated by any number in between 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the exosomes bound by one or more of the lectins: Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin will comprise an antigen present on one or more of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules.

The invention is generally disclosed herein using affirmative language to describe the numerous embodiments. The invention also includes embodiments in which subject matter is excluded, in full or in part, such as substances or materials, method steps and conditions, protocols, or procedures. Thus, even though the invention is generally not expressed herein in terms of what the invention does not include aspects that are not expressly excluded in the invention are nevertheless disclosed herein.

A number of embodiments of the invention have been described. Nevertheless, one skilled in the art, without departing from the spirit and scope of the invention, can make various changes and modifications of the invention to adapt it to various usages and conditions. For example, salts, esters, ethers and amides of invention compounds disclosed herein are within the scope of this invention. Accordingly, the following examples are intended to illustrate but not limit the scope of invention described in the claims.

### EXAMPLES

### Example 1: Preparation of Antibodies Covalently Coupled to Agarose using Cyanogen Bromide

Brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules are covalently coupled to agarose (preferably colored agarose) using cyanogen Bromide and cyanogen bromide (CNBr) activated agarose according to Cuatrecasas, et al. (Cuatrecasas, Wilchek and Anfinsen. Proc Natl Acad Sci USA 61(2): 636-643, 1968). Exemplary binding agents that are used in this example include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). In brief, 1 ml of antibody at a concentration of 10 mg/ml in 0.1M NaHCO3 pH 9.5 is added to 1 ml CNBr activated agarose microspheres, which can include a dye (Sigma, St. Louis, Mo.) and allowed to react overnight in the cold. Care must be taken to maintain alkaline pH to prevent the potential release of HCN gas. When the reaction is complete, unreacted materials are aspirated and the antibody coupled agarose is washed extensively with sterile cold PBS. The antibody agarose affinity matrix is then stored cold until ready for use. The antibody agarose affinity matrix can be incorporated into a lateral flow diagnostic device.

### Example 2: Preparation of an Antibody Covalently Coupled to Glass Beads via Schiffs Base and Reduction with Cyanoborohydride

An antibody covalently coupled to glass beads, preferably colored glass beads, via Schiffs Base and reduction with cyanoborohydride is prepared. The affinity matrix is prepared by a modification of the method of Hermanson (Hermanson. Bioconjugate Techniques: 785, 1996). Exemplary binding agents that are used in this example include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments) Anti-tau monoclonal antibody, for example, is dissolved to a final protein concentration of 10 mg/ml in 0.1M sodium borate pH 9.5 is added to aldehyde derivatized silica glass beads (BioConnexant, Austin Tex.). The reaction is most efficient at alkaline pH but will go at pH 7-9 and is normally done at a 2-4 fold excess of protein over coupling sites. To this mixture is added 10 µl 5M NaCNBH3 in IN NaOH (Aldrich, St Louis, Mo.) per ml of coupling reaction and the mixture allowed to react for 2 hours at room temperature. At the end of the reaction, remaining unreacted aldehyde on the glass surfaces are capped with 20 µl 3M ethanolamine pH 9.5 per ml of reaction. After 15 minutes at room temperature, the reaction solution is decanted and the unbound proteins and reagents removed by washing extensively in PBS. The matrix is then stored in the refrigerator until ready for use. Antibodies, such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecule can be conjugated or joined to glass beads, preferably colored glass beads using this method. The antibody glass bead affinity matrix can be incorporated into a lateral flow diagnostic device.

### Example 3: Preparation of an Exosome Specific Antibody Covalently Coupled to Chromosorb (Diatomaceous Earth) Using Glutaraldehyde

Brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) are covalently coupled to Chromosorb (Diatomaceous Earth) using glutaraldehyde. Exemplary binding agents that are used in this example include antibodies such as: β-amyloid antibody (clone 20.1), which is a mouse monoclonal IgG_{2b} raised against amino acids 1-40 of human β-amyloid; tau polysulfone, polyethersulfone polysulfone, polyethersulfone antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Preparation of aminated diatomaceous earth is accomplished using γ-aminopropyl triethoxysilane (GAPS) (Sigma Chemical, St. Louis, Mo.) and Chromosorb 60/80 mesh. Although other grades of diatomaceous earth may be used, Chromosorb of this mesh size (200-300 microns in diameter) is often used to prevent small particulates from entering the sample through the largest available pore sizes found in hollow-fiber cartridges used for plasma separation (^{∼}0.5 micron). Amino Chromosorb is prepared by suspension in an excess of 5% aqueous solution of GAPS in an overnight reaction. Aminated-Chromosorb is washed free of excess reagent with water and ethanol and dried overnight in a drying oven to yield an off white powder. One gram of the powder is then suspended in 5 ml 5% glutaraldehyde (Sigma) for 30 minutes. Excess glutaraldehyde is then removed by filtration and washing with water until no detectable aldehyde remains in the wash using Schiffs reagent (Sigma Chemical). The filter cake is then resuspended in 5 ml of Sigma borohydride coupling buffer containing 2-3 mg/ml of the antibody and the reaction allowed to proceed overnight at 4 degrees C. At the end of the reaction, excess antibody is washed off and the remaining aldehyde reacted with ethanolamine as described. After final washing in sterile PBS, the material is stored cold until ready for use. The antibody Chromosorb affinity matrix can be incorporated into a lateral flow diagnostic device.

### Example 4: Preparation of an Antibody Covalently Coupled to Polyacrylate Beads Using Glutaraldehyde and Azide

Brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) are covalently coupled to polyacrylate beads using Glutaraldehyde and Azide. Exemplary binding agents that are used in this example include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The antibodies are dissolved in a concentration of 50-200 mg/ml with human serum albumin in a phosphate-buffered aqueous medium of pH 7.0. Glutaraldehyde at a concentration of 0.05-10% is added to the solution which is then incubated for 1-24 hours, but preferably 12 hours, at 4 degree. C. Excess glutaraldehyde that remains in the reaction mixture is removed by addition of glycine, or other suitable compounds known in the art, to the solution at the end of incubation. This solution is then diafiltered through a membrane having a minimal retentively value of 500,000 molecular weight. The diafiltered antibody-bearing product is dissolved in saline or dialysis fluid. To obtain a reactive polymer to act as a substrate for the antibody, polyacrylic acid polymer beads (≦1 micron in diameter) are activated by the azide procedure. The ratios of antibody to reactive polyester are selected to avoid excessive reaction. If this ratio is appropriately adjusted, the spacing of the antibody along the polymer chain will allow a binding of the antibody with the antigen found on microvesicle without untoward steric hindrances and the antibody conjugate is intended to remain soluble.

### Example 5: Preparation of Chemically Immobilized Antibody onto Nylon

A preparation of chemically immobilized brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules on nylon is prepared. Exemplary binding agents that are used in this example include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). The method consists of serial treatments with HCl, polyethylene imine, and maleic anhydride methylvinyl ether copolymer, which results in the stable immobilization of sufficient amounts of antibodies on a nylon filter, which may be incorporated into a point of care diagnostic test, such as a lateral flow assay device or a diagnostic test strip. In one embodiment, Polyethylenevinyl-acetate slips are immersed in 15% (wt/vol) NaOH in 80% (vol/vol) methanol at 58°C for 2 h, washed with water, heated in 2% (vol/vol) aminoacetal in 0.5 N HCl at 58°C for 5 h, washed and dried, soaked in 1% (wt/vol) maleic anhydride methylvinyl ether copolymer (MAMEC) in water-free acetone at 25°C for 2.5 h, and dried. Polyurethane slips are heated in water at 80°C for 6 h, washed with distilled water, dried, treated in 1% (wt/vol) MAMEC in water-free acetone at 25°C for 2.5 h, washed with acetone, and then dried. Polyvinyl chloride slips are immersed in 1% (wt/vol) MAMEC in water-free acetone at 25°C for 2.5 h, washed with acetone, and then dried. MAMEC is introduced onto nylon slips. After MAMEC is introduced onto various polymeric slips, the antibodies (protein concentration, 2 µg/ml) suspended in phosphate-buffered saline are immobilized on the slips at 25°C overnight.

### Example 6: Lateral Flow Assay Diagnostic Device

A lateral flow assay diagnostic device comprising brain biomarker specific antibodies such as a monoclonal antibodies, or a binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules can be prepared using techniques well established in the art (e.g., utilizing approaches employed by Millipore™ Rapid Lateral Flow Test Strips and Bangs Laboratories, Inc. TechNote 303). Exemplary binding agents that are used in this example include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). By one approach a first binding agent such as monoclonal antibodies, or binding fragments thereof (e.g., a Fab fragment or a fragment having a CDR domain), which are specific for an antigenic site or epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, molecules) having a detectable moiety (e.g., a colored bead) is immobilized to the nylon membrane at a discrete zone and a second binding agent, which may be an antibody, fragment thereof or lectin that is specific for an epitope or antigen on MHC I or II is immobilized to the nylon membrane at a second discrete zone. The nylon membrane having the binding agents is then incorporated into a lateral flow device. When the nylon is contacted with a biological sample, including urine, blood, plasma, or cerebral spinal fluid that comprises a brain-specific extracellular vesicle or exosome, a biological complexes comprising the first binding agent joined to the brain-specific extracellular vesicle or exosome and the second binding agent are formed.

### Example 7 Removal of Exosomes from Blood Using Plasmapheresis

Selective removal of exosomes from blood may be accomplished using plasmapheresis combined with affinity capture using any of the matrices described in Examples 1-6. Exemplary binding agents that are used in this example include antibodies such as: β-amyloid antibody (clone 20.1), which is a mouse monoclonal IgG_{2b} raised against amino acids 1-40 of human β-amyloid; tau antibody (clone D-8), which is a mouse monoclonal IgG_{2b} raised against amino acids 1-150 of human tau; S100 β chain antibody (clone 9A11B9), which is a mouse monoclonal IgG₁ raised against the full length recombinant human S100 β chain protein; anti-A2B5 antibody [clone 105], which is a mouse monoclonal antibody raised against full length human A2B5 and binding fragments of said antibodies (e.g., the CDR domains or Fab fragments). Plasmapheresis is done using either centrifugal separation or hollow-fiber plasma separation methods. The blood circuit is anticoagulated with a continuous heparin infusion in the afferent limb. The activated clotting time (ACT) is measured every hour, and the heparin infusion is adjusted to maintain the ACT between 160 and 180 seconds.

The plasma obtained from the patient may be discarded and replaced with a combination of normal saline and fresh plasma from healthy donors (i.e. plasma exchange). Alternatively, the plasma containing the microvesicles can be pumped at 60-100 ml/min over the affinity matrix which captures the exosomes. The cleaned plasma may then be reinfused into the patient. The clearance of the microvesicles may be monitored based on the concentration of microvesicles remaining in circulation

### Example 8: Identification of Circulating Exosomes Using Streptavidin-coniugate Qdots and NS300

Exosomes in circulation of a patient with brain cancer were labeled with biotinylated primary antibody. The exosomes were then visualized with streptavidin-conjugated quantum dots (Qdots). The streptavidin Qdots, which are fluorescent at 655nm, specifically bind to the biotin on the primary antibody. The circulating exosomes were then quantitated using NanoSight NS300. Figure 1 depicts the total exosomes defined in light scatter mode. Figure 3 depicts quantification of total circulating exosomes in a 3-dimensional Cartesian plot with "particle size" on the x-axis, "relative intensity" on the y-axis, and "total circulating exosomes" on the z-axis.

### Example 9: Identification of Circulating Brain Derived Exosomes Identified by β-Amyloid Expression Using Streptavidin-coniugate Qdots and NS300

Exosomes in circulation of a patient with brain cancer were labeled with biotinylated primary antibody specific for an antigen present on β-amyloid (β-amyloid antibody (clone 20.1), which is a mouse monoclonal IgG_{2b} raised against amino acids 1-40 of human β-amyloid). The exosomes were then visualized with streptavidin-conjugated quantum dots (Qdots). The streptavidin Qdots, which are fluorescent at 655nm, specifically bind to the biotin on the primary antibody. The circulating exosomes were then quantitated using NanoSight NS300. Figure 2 depicts brain-derived exosomes identified by β-amyloid expression, determined in fluorescent mode. Figure 5 depicts brain-derived exosomes identified by β-amyloid expression, determined in fluorescent mode in a 3-dimensional Cartesian plot with "particle size" on the x-axis, "relative intensity" on the y-axis, and "Circulating Exosomes of Brain Origin β-Amyloid" on the z-axis.

### Example 10: Identification of Circulating Brain Derived Exosomes Identified by Tau Expression, S100 Expression, and A2B5 Expression Using Streptavidin-coniugate Qdots and NS300

Exosomes in circulation of a patient with brain cancer were labeled with biotinylated primary antibody specific for an antigen present on tau (tau antibody (clone D-8), which is a mouse monoclonal IgG_{2b} raised against amino acids 1-150 of human tau), S100(S100 β chain antibody (clone 9A11B9), which is a mouse monoclonal IgG₁ raised against the full length recombinant human S100 β chain protein) and A2B5(anti-A2B5 antibody [clone 105], which is a mouse monoclonal antibody raised against full length human A2B5). The exosomes were then visualized with streptavidin-conjugated quantum dots (Qdots). The streptavidin Qdots, which are fluorescent at 655nm, specifically bind to the biotin on the primary antibody. The circulating exosomes were then quantitated using NanoSight NS300. Figure 4 depicts brain-derived exosomes identified by Tau expression, determined in fluorescent mode in a 3-dimensional Cartesian plot with "particle size" on the x-axis, "relative intensity" on the y-axis, and "Circulating Exosomes of Brain Origin Tau" on the z-axis. Figure 6 depicts brain-derived exosomes identified by A2B5 expression, determined in fluorescent mode in a 3-dimensional Cartesian plot with "particle size" on the x-axis, "relative intensity" on the y-axis, and "Circulating Exosomes of Brain Origin Tau" on the z-axis. Figure 7 depicts brain-derived exosomes identified by S100 expression, determined in fluorescent mode in a 3-dimensional Cartesian plot with "particle size" on the x-axis, "relative intensity" on the y-axis, and "Circulating Exosomes of Brain Origin Tau" on the z-axis.

### Example 11: Identification of Lectins that selectively bind to Brain Derived Exosomes

Exosomes in circulation of a patient with brain cancer can be isolated and characterized as follows. A biological sample, preferably blood, urine, or cerebrospinal fluid, is obtained from a patient with brain cancer. The biological sample is then contacted with a support, preferably a filter, membrane, polymer, or bead, that comprises a lectin joined thereto (e.g., Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin). The non-binding materials are washed from the support and the exosomes that remain bound to the lectin and support are identified. By one approach, biotinylated antibodies, which bind to an antigenic epitope present on an MHC I molecule, are contacted with the bound exosomes, as these antibodies are contemplated to bind to all exosomes that would be present on the lectin bound support. Detection, identification, and quantification of the total number of exosomes bound to the lectin and support can be accomplished with streptavidin having a detectable moiety such as nanogold or Qdots. A second binding reaction is then conducted, wherein exosomes that are bound to the lectin and support are contacted with a second biotinylated antibody specific for an antigenic epitope present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. The binding of the second antibody to the exosomes that remain on the support can then be identified and quantified using a streptavidin having a detectable moiety such as nanogold or Qdots. By comparing the amounts of exosomes detected after the first binding agent (the anti-MHC I antibody) to the amounts of exosomes detected after the second binding agent (the antibody specific for an antigen present on tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules), one can determine the ability of a particular lectin (e.g., Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin) to preferentially bind to exosomes that have an antigen present, wherein the antigen is tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, or an antigenic fragment of these molecules. It is contemplated that one or more of: Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin will preferentially bind exosomes having an antigen present, wherein the antigen is one or more of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules. For example, it is contemplated that at least, equal to, or a range generated by any number in between 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the exosomes bound by one or more of the lectins: Galanthus nivalis agglutinin (GNA), Narcissus pseudonarcissus agglutinin (NPA), Concanavalin A or cyanovirin, Sambucus nigra lectin, ricin from *Ricinus communis* (RCA), Dolichos biflorius lectin, Ulex Europaeus lectin, Vicia villosa lectin, Griffonia simplicifolia lectin, Solanium tuberosum lectin, Lycopersicon esculentum lectin, Datura stramonium lectin, Erythrina cristagalli lectin, Soybean lectin, Peanut agglutinin, Wheat germ agglutinin, or Jacalin lectin will comprise an antigen present on one or more of tau, β-amyloid, S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules.

Furthermore, although the foregoing has been described in some detail by way of illustrations and examples for purposes of clarity and understanding, it will be understood by those of skill in the art that numerous and various modifications can be made without departing from the spirit of the present disclosure. Therefore, it should be clearly understood that the forms disclosed herein are illustrative only and are not intended to limit the scope of the present disclosure, but rather to also cover all modification and alternatives coming with the true scope of the invention.

## Claims

1. A diagnostic device, comprising:
a substrate comprising a sample reservoir, wherein said sample reservoir is configured to receive an amount of a biological sample and said sample reservoir comprises an amount of one or more mobilizable labeled antibodies or binding fragments thereof specific for a disease marker;
an absorbent material in fluid communication, such as by capillary flow, with said substrate distal from said sample reservoir;
a lectin immobilized to said substrate at a test zone, wherein said test zone is in fluid communication, such as by capillary flow, with said sample reservoir and said absorbent material;
a first amount of protein immobilized to said substrate at a first control/standard zone, wherein said first control/standard zone is in fluid communication, such as by capillary flow, with said sample reservoir and, wherein the first amount of protein in the first control/standard zone is an amount detectable by said mobilizable labeled antibodies or binding fragments thereof in a biological sample obtained from a healthy subject; and/or
a second amount of protein immobilized to said substrate at a second control/standard zone, wherein the second amount of protein in the second control/standard zone is an amount detectable by said mobilizable labeled antibodies or binding fragments thereof in a biological sample obtained from a subject that has a brain pathology, such as Alzheimer's disease or chronic traumatic encephalopathy (CTE); and preferably, the first and/or second amounts of protein is the detectable amount of said proteins in the same volume of the same biological sample from said healthy subject and said subject that has a brain pathology, respectively, as the biological sample from said tested subject.

2. A diagnostic device according claim 1, wherein the substrate is a membrane selected from the group consisting of polysulfone, polyethersulfone, polyamide, polyimide, nitrocellulose, PVDF, nylon and cellulose acetate.

3. A diagnostic device according to any of the preceding claims, wherein the biological sample is selected from the group consisting of blood, plasma, urine, sweat, milk, cerebrospinal fluid, and saliva.

4. A diagnostic device according to any of the preceding claims, wherein the lectin is a Ricin lectin.

5. A diagnostic device according to any of the preceding claims, wherein the label on the antibodies is colloidal carbon, colloidal gold, a fluorescent label, a quantum dot, a phosphor, a colored particle, a bioluminescent marker, an enzyme label, a paramagnetic particle, or a colored latex particles.

6. A diagnostic device according to any of the preceding claims, wherein the protein immobilized to said substrate is in the amount of 1 pg/ml to 500 µg/ml, 1 pg/ml to 100 pg/ml, 100 pg/ml to 1,000 pg/ml, 1 ng/ml to 100 ng/ml, 100 ng/ml to 1,000 ng/ml, and 1 µg/ml to 500 µg/ml, or an amount that is within a range defined by any two amounts within one or more of the aforementioned ranges of amounts.

7. A diagnostic device according to any of the preceding claims, wherein the brain pathology is chronic traumatic encephalopathy (CTE).

8. A diagnostic device according to any of the preceding claims, wherein the brain pathology is Alzheimer's disease.

9. A diagnostic device according to any of the preceding claims, wherein the device comprises S100 β.

10. A diagnostic device according to any of the preceding claims, wherein the device comprises glycoprotein A2B5.

11. A diagnostic device according to any of claims 1-8, wherein said disease marker is S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and/or a fragment thereof or any combination thereof.

12. A method for identifying a subject that is at risk for chronic traumatic encephalopathy (CTE) comprising:
applying a biological sample to a diagnostic device of claim 1-11; and
identifying said subject as being at risk for CTE when the amount of said disease marker captured at the capture zone or test zone is greater than the amount of protein detected in the first control/standard zone.

13. A method according to claim 12, wherein the disease marker is tau, glycosylated tau, phosphorylated tau, β-amyloid, glycosylated β-amyloid, phosphorylated β-amyloid, and/or a fragment thereof or any combination thereof.

14. A method according to claim 12, wherein the disease marker is S100 β, neuron-specific enolase, glycoprotein A2B5, CD133, NQ01, synaptophysin, neuronal nuclei, MAB1569, polysialic acid-neural cell adhesion molecule (PSA-NCAM), or neurogenic differentiation 1 (NeuroD or Beta2), or glycosylated or phosphorylated forms of these molecules, and/or a fragment thereof or any combination thereof.

## Patentansprüche

1. Diagnosevorrichtung, Folgendes umfassend:
ein Substrat, das ein Probenreservoir umfasst, wobei das Probenreservoir so konfiguriert ist, dass es eine Menge an einer biologischen Probe aufnehmen kann und das Probenreservoir eine Menge von einem oder mehreren, mobilisierbar gekennzeichneten Antikörpern oder bindenden Fragmenten davon umfasst, die für einen Krankheitsmarker spezifisch sind;
ein absorbierendes Material in Fluidverbindung, wie etwa durch eine Kapillarströmung, mit dem Substrat, das distal vom Probenreservoir distal ist;
ein Lektin, das in einem Testbereich an dem Substrat immobilisiert ist, wobei der Testbereich mit dem Probenreservoir und dem absorbierenden Material in Fluidverbindung steht, wie etwa durch eine Kapillarströmung;
eine erste Menge Protein, die an das Substrat an einem ersten Kontroll-/Standardbereich immobilisiert ist, wobei der erste Kontroll-/Standardbereich mit dem Probenreservoir in Fluidverbindung steht, wie etwa durch eine Kapillarströmung, und wobei die erste Menge Protein im ersten Kontroll-/Standardbereich eine Menge ist, die von den mobilisierbaren gekennzeichneten Antikörpern oder bindenden Fragmenten davon in einer biologischen Probe, die von einem gesunden Subjekt entnommen wurde, detektierbar ist; und/oder
eine zweite Menge Protein, die an das Substrat an einem zweiten Kontroll-/Standardbereich immobilisiert ist, wobei die zweite Menge Protein im zweiten Kontroll-/Standardbereich eine Menge ist, die von den mobilisierbaren gekennzeichneten Antikörpern oder bindenden Fragmenten davon in einer biologischen Probe, die einem Subjekt entnommen wurde, welches eine Gehirnpathologie, wie etwa Alzheimer-Krankheit oder chronisch-traumatische Enzephalopathie (CTE), aufweist, detektierbar ist; und wobei vorzugsweise die erste und die zweite Menge an Protein die detektierbare Menge an Protein im selben Volumen von derselben biologischen Probe vom gesunden Subjekt bzw. vom Subjekt mit der Gehirnpathologie ist, wie die biologische Probe vom geprüften Subjekt.

2. Diagnosevorrichtung nach Anspruch 1, wobei das Substrat eine Membran ist, die aus der Gruppe bestehend aus Polysulfon, Polyethersulfon, Polyamid, Polyimid, Cellulosenitrat, PVDF, Nylon und Celluloseacetat ausgewählt ist.

3. Diagnosevorrichtung nach einem der vorhergehenden Ansprüche, wobei die biologische Probe aus der Gruppe bestehend aus Blut, Plasma, Urin, Schweiß, Milch, Rückenmarksflüssigkeit und Speichel ausgewählt ist.

4. Diagnosevorrichtung nach einem der vorhergehenden Ansprüche, wobei das Lektin ein Rizin-Lektin ist.

5. Diagnosevorrichtung nach einem der vorhergehenden Ansprüche, wobei die Markierung auf den Antikörpern kolloidaler Kohlenstoff, kolloidales Gold, eine fluoreszierende Markierung, ein Quantenpunkt, ein Phosphor, ein Farbpartikel, ein biolumineszenter Marker, eine Enzymmarkierung, ein paramagnetisches Partikel oder ein farbiges Latexpartikel ist.

6. Diagnosevorrichtung nach einem der vorhergehenden Ansprüche, wobei das Protein, das auf dem Substrat immobilisiert ist, sich auf eine Menge von 1 pg/ml bis 500 µg/ml, 1 pg/ml bis 100 pg/ml, 100 pg/ml bis 1.000 pg/ml, 1 ng/ml bis 100 ng/ml, 100 ng/ml bis 1.000 ng/ml und 1 µg/ml bis 500 µg/ml oder eine Menge, die im Bereich liegt, der von jeglichen zwei Mengen in einen oder mehreren der vorstehenden Bereiche an Mengen definiert wird, beläuft.

7. Diagnosevorrichtung nach einem der vorhergehenden Ansprüche, wobei die Gehirnpathologie eine chronisch-traumatische Enzephalopathie (CTE) ist.

8. Diagnosevorrichtung nach einem der vorhergehenden Ansprüche, wobei die Gehirnpathologie die Alzheimer-Krankheit ist.

9. Diagnosevorrichtung nach einem der vorhergehenden Ansprüche, wobei die Vorrichtung S100 β umfasst.

10. Diagnosevorrichtung nach einem der vorhergehenden Ansprüche, wobei die Vorrichtung Glykoprotein A2B5 umfasst.

11. Diagnosevorrichtung nach einem der Ansprüche 1 bis 8, wobei der Krankheitsmarker S100 β, neuronenspezifische Enolase, Glykoprotein A2B5, CD133, NQ01, Synaptophysin, neuronale Zellkerne, MAB1569, Polysialinsäure-neurale Zellenadhäsionsmoleküle (PSA-NCAM) oder neurogene Differentiation 1 (NeuroD oder Beta2) oder glykosylierte oder phosphorylierte Formen dieser Moleküle und/oder ein Fragment davon oder eine Kombination davon ist.

12. Verfahren für das Identifizieren eines Subjekts, das einem Risiko für eine chronisch-traumatische Enzephalopathie (CTE) unterliegt, Folgendes umfassend:
Anwenden einer biologischen Probe auf einer Diagnosevorrichtung nach Anspruch 1-11; und
Identifizieren des Subjekts als dem Risiko für CTE unterliegend, wenn die Menge der Krankheitsmarker, die im Erfassungsbereich oder im Testbereich erfasst wurde, größer ist als die Menge an Protein, die im ersten Kontroll-/Standardbereich erfasst wurde.

13. Verfahren nach Anspruch 12, wobei der Krankheitsmarker Tau, phosphoryliertes Tau, β-Amyloid, glykosyliertes β-Amyloid, phosphoryliertes β-Amyloid und/oder ein Fragment davon oder eine Kombination davon ist.

14. Verfahren nach Anspruch 12, wobei der Krankheitsmarker S100 β, neuronenspezifische Enolase, Glykoprotein A2B5, CD133, NQ01, Synaptophysin, neuronale Zellkerne, MAB1569, Polysialinsäure-neurale Zellenadhäsionsmoleküle (PSA-NCAM) oder neurogene Differentiation 1 (NeuroD oder Beta2) oder glykosylierte oder phosphorylierte Formen dieser Moleküle und/oder ein Fragment davon oder eine Kombination davon ist.

## Revendications

1. Dispositif de diagnostic, comprenant :
un substrat comprenant un réservoir d'échantillon, dans lequel ledit réservoir d'échantillon est configuré pour recevoir une quantité d'un échantillon biologique et ledit réservoir d'échantillon comprend une quantité d'un ou de plusieurs anticorps marqués mobilisables ou de fragments de liaison de ceux-ci spécifiques d'un marqueur de maladie ;
un matériau absorbant en communication fluidique, tel que par écoulement capillaire, avec ledit substrat distal dudit réservoir d'échantillon ;
une lectine immobilisée sur ledit substrat au niveau d'une zone d'essai, dans lequel ladite zone d'essai est en communication fluidique, telle que par écoulement capillaire, avec ledit réservoir d'échantillon et ledit matériau absorbant ;
une première quantité de protéine immobilisée sur ledit substrat au niveau d'une première zone témoin/type, dans lequel ladite première zone témoin/type est en communication fluidique, telle que par écoulement capillaire, avec ledit réservoir d'échantillon et, dans lequel la première quantité de protéine dans la première zone témoin/type est une quantité détectable par lesdits anticorps marqués mobilisables ou des fragments de liaison de ceux-ci dans un échantillon biologique obtenu auprès d'un sujet sain; et/ou
une seconde quantité de protéine immobilisée sur ledit substrat au niveau d'une seconde zone témoin/type, dans lequel la seconde quantité de protéine dans la seconde zone témoin/type est une quantité détectable par lesdits anticorps marqués mobilisables ou des fragments de liaison de ceux-ci dans un échantillon biologique obtenu auprès d'un sujet qui souffre d'une pathologie au cerveau, telle que la maladie d'Alzheimer ou l'encéphalopathie traumatique chronique (CTE) ; et de préférence, les première et/ou seconde quantités de protéine sont la quantité détectable desdites protéines dans le même volume du même échantillon biologique provenant dudit sujet sain et dudit sujet qui souffre d'une pathologie au cerveau, respectivement, en tant qu'échantillon biologique provenant dudit sujet à l'essai.

2. Dispositif de diagnostic selon la revendication 1, dans lequel le substrat est une membrane choisie dans le groupe consistant en la polysulfone, la polyéthersulfone, le polyamide, le polyimide, la nitrocellulose, le PVDF, le nylon et l'acétate de cellulose.

3. Dispositif de diagnostic selon l'une quelconque des revendications précédentes, dans lequel l'échantillon biologique est choisi dans le groupe consistant en du sang, du plasma, de l'urine, de la transpiration, du lait, du liquide céphalorachidien, et de la salive.

4. Dispositif de diagnostic selon l'une quelconque des revendications précédentes, dans lequel la lectine est une lectine de ricin.

5. Dispositif de diagnostic selon l'une quelconque des revendications précédentes, dans lequel le marqueur sur les anticorps est le carbone colloïdal, l'or colloïdal, un marqueur fluorescent, une boîte quantique, un luminophore, une particule colorée, un marqueur bioluminescent, une enzyme marqueur, une particule paramagnétique, ou des particules de latex coloré.

6. Dispositif de diagnostic selon l'une quelconque des revendications précédentes, dans lequel la protéine immobilisée sur ledit substrat se trouve dans la quantité de 1 pg/mL à 500 µg/mL, 1 pg/mL à 100 pg/mL, 100 pg/mL à 1 000 pg/mL, 1 ng/mL à 100 ng/mL, 100 ng/mL à 1 000 ng/mL, et 1 µg/mL à 500 µg/mL, ou une quantité qui se trouve dans une plage définie par l'une quelconque de deux quantités au sein d'une ou de plusieurs des plages de quantités mentionnées ci-dessus.

7. Dispositif de diagnostic selon l'une quelconque des revendications précédentes, dans lequel la pathologie du cerveau est l'encéphalopathie traumatique chronique (CTE).

8. Dispositif de diagnostic selon l'une quelconque des revendications précédentes, dans lequel la pathologie du cerveau est la maladie d'Alzheimer.

9. Dispositif de diagnostic selon l'une quelconque des revendications précédentes, dans lequel le dispositif comprend S100 β.

10. Dispositif de diagnostic selon l'une quelconque des revendications précédentes, dans lequel le dispositif comprend la glycoprotéine A2B5.

11. Dispositif de diagnostic selon l'une quelconque des revendications 1 à 8, dans lequel ledit marqueur de maladie est S100 β, une énolase spécifique des neurones, la glycoprotéine A2B5, CD133, NQ01, la synaptophysine, des noyaux neuronaux, MAB1569, une molécule d'adhérence cellulaire neuronale-polyacide sialique (PSA-NCAM), ou une différentiation neurogène 1 (NeuroD ou Bêta2), ou des formes glycosylées ou phosphorylées de ces molécules, et/ou un fragment de celles-ci ou toute combinaison de celles-ci.

12. Procédé d'identification d'un sujet qui présente un risque de développer une encéphalopathie traumatique chronique (CTE) comprenant :
l'application d'un échantillon biologique à un dispositif de diagnostic des revendications 1 à 11 ; et
l'identification dudit sujet comme présentant un risque de développer une CTE lorsque la quantité dudit marqueur de maladie capturé au niveau de la zone de capture ou zone d'essai est supérieure à la quantité de protéine détectée dans la première zone témoin/type.

13. Procédé selon la revendication 12, dans lequel le marqueur de maladie est la protéine tau, la protéine tau glycosylée, la protéine tau phosphorylée, la β-amyloïde, la β-amyloïde glycosylée, la β-amyloïde phosphorylée, et/ou un fragment de celles-ci ou toute combinaison de celles-ci.

14. Procédé selon la revendication 12, dans lequel le marqueur de maladie est S100 β, l'énolase spécifique des neurones, la glycoprotéine A2B5, CD133, NQ01, la synaptophysine, des noyaux neuronaux, MAB1569, une molécule d'adhérence cellulaire neuronale-polyacide sialique (PSA-NCAM), ou une différentiation neurogène 1 (NeuroD ou Bêta2), ou des formes glycosylées ou phosphorylées de ces molécules, et/ou un fragment de celles-ci ou toute combinaison de celles-ci.
